# EUROPÄISCHE PATENTANMELDUNG

(11) **EP 4 306 954 A1**
(43) Veröffentlichungstag der Anmeldung: **17.01.2024**
(21) Anmeldenummer: 23180598.7
(22) Anmeldetag: 21.06.2023
(51) Int. Cl.: G01N 33/50, G01N 33/68

(54) **ORTSAUFGELÖSTE MASSENSPEKTROMETRISCHE BESTIMMUNG DER ZELLTOXIZITÄT**

(30) Priorität: 22.06.2022 DE 102022115561
(71) Anmelder: Bruker Daltonics GmbH & Co. KG, 28359 Bremen (DE)
(72) Erfinder: Idelevich, Evgeny, 17489 Greifswald (DE); Becker, Karsten, 17509 Laer (DE)

(57) **Zusammenfassung**

Verfahren zur Bestimmung der zytotoxischen Wirkung einer analytischen Probe auf tierische Zellen, einschließlich humaner Zellen. Das Verfahren umfasst (a) einen Probenbereitstellungsschritt, in dem eine massenspektrometrischen Probe, umfassend tierische Zellen, Nährmedium und die analytische Probe, welche potenziell einen zytotoxischen Faktor aufweist, auf mindestens einer Probenstelle eines massenspektrometrischen Probenträger bereitgestellt wird; (b) einen Kultivierungsschritt, in dem die massenspektrometrische Probe auf der Probenstelle des massenspektrometrischen Probenträgers in einer Kultivierungsvorrichtung inkubiert wird; (c) einen Flüssigkeitsentfernungsschritt, in dem Restflüssigkeit der massenspektrometrischen Probe von der Probenstelle entfernt wird; (d) einen Messschritt, in dem ortsaufgelöste Massenspektren an einer Vielzahl von Messpositionen in mindestens einer Teilfläche der Probenstelle mittels eines ortsauflösenden Massenspektrometers aufgenommen werden, wobei bei Verwendung von einem matrix-basierten ortsauflösende Massenspektrometer vor der Aufnahme der ortsaufgelösten Massenspektren im Messschritt in einem vorgelagerten Probenpräparationsschritt eine Präparation der Probenstelle durch ortstreue Auftragung einer Matrix auf mindestens die eine Teilfläche der Probenstelle erfolgt; (e) einen ersten Auswerteschritt, in dem jedes ortsaufgelöste Massenspektrum hinsichtlich des Vorhandenseins einer zellspezifischen massenspektrometrischen Signatur für die tierischen Zellen analysiert wird und jeder Messposition ein Zellpräsenzwert zugewiesen wird; (f) einen zweiten Auswerteschritt, in dem ein Bedeckungsgrad durch tierische Zellen für mindestens die Teilfläche der Probenstelle aus den Zellpräsenzwerten der Messpositionen bestimmt wird; (g) und/oder einen dritten Auswerteschritt, in dem eine Proliferationsfähigkeit aus dem bestimmten Bedeckungsgrades abgeleitet wird; (h) einen vierten Auswerteschritt, in dem die zytotoxische Wirkung der analytischen Probe auf die tierischen Zellen mittelbar oder unmittelbar aus dem bestimmten Bedeckungsgrad des zweiten Auswerteschrittes abgeleitet wird, wobei hierzu die zytotoxische Wirkung der analytischen Probe auf die tierischen Zellen aus dem Ergebnis wenigstens einer der beiden Schritte zweiter Auswerteschritt und/oder dritter Auswerteschritt abgeleitet wird.

## Beschreibung

Die vorliegende Erfindung betrifft ein massenspektrometrisches Verfahren zur Bestimmung der zytotoxischen Wirkung von zytotoxischen Faktoren auf tierische Zellen.

Zwei Hauptaufgaben der mikrobiologischer Routine-Labore sind die Identifikation der mikrobiellen Erreger sowie die antimikrobielle Empfindlichkeitstestung (d.h. Resistenztestung) der Bakterien und Pilze.

Hierzu haben in den letzten Jahren unterschiedliche massenspektrometrische Verfahren einen breiten Eingang in die Forschungslabore und in mikrobiologische Routinelabore gefunden. Insbesondere in den mikrobiologischen Laboratorien hat sich in den letzten Jahren ein spezielles Verfahren der Massenspektrometrie (MS), die "matrix-assisted laser desorption ionization - time of flight" (MALDI-ToF) Massenspektrometrie, etabliert und die meisten mikrobiologischen Laboratorien wurden im Laufe des letzten Jahrzehnts mit entsprechenden MALDI-ToF MS Geräten ausgestattet. Dabei fokussiert sich die Routine-Anwendung dieser Methode bisher jedoch ausschließlich auf die Erreger-Identifikation von Bakterien und Pilzen. Hinzu kommen erste Anwendungen zur Resistenzbestimmung der Bakterien und Pilze. Diese befinden sich bisher überwiegend noch im Entwicklungsstadium. So beschreibt zum Beispiel WO2018/099500A1, Idelevich et al. [E.A. Idelevich, K. Sparbier, M. Kostrzewa, K. Becker, Rapid detection of antibiotic resistance by MALDI-ToF mass spectrometry using a novel direct-on-target microdroplet growth assay, Clinical Microbiology and Infection, Volume 24, Issue 7, 2018.] das Kultivieren von mikrobiellen Proben auf MS-Probenträgern und die Bestimmung der Resistenz von Mikroorganismen gegenüber mikrobiellen Substanzen.

Neben der Identifikation der Bakterien und Pilze und der Bestimmung der Resistenz von Bakterien und Pilzen gegenüber bestimmten antimikrobiell wirksamen Substanzen gibt es jedoch weitere zu untersuchende Aspekte in den Laboren, bei denen keine MS zur Anwendung kommt. Neben molekularbiologischen Nachweisverfahren, wie z.B. PCR beruhen diese auf phänotypischen Nachweisverfahren, bei denen die Wirkung von zytotoxischen Faktoren über das Wachstum tierischer Zellen, z.B. kontinuierlichen Zelllinien, i.d.R. optisch (d.h. entweder durch Feststellung einer Farbreaktion oder durch mikroskopische Bestimmung der Vitalität und Morphologie der Zellen) festgestellt wird.

Ein wichtiger zu untersuchender Aspekt ist zum Beispiel die Virulenz eines Erregers. Unter der Virulenz versteht man den Ausprägungsgrad der Pathogenität eines bestimmten Isolates einer mikrobiellen Spezies, wobei die Pathogenität die grundsätzliche Fähigkeit von Erregern bedeutet, eine Erkrankung bei einem Makroorganismus (Wirt, zum Beispiel Mensch) zu verursachen. Hierbei kann man zwischen einer gezielten (hypothesenbasierten) Detektion von bestimmten relevanten Virulenz-Phänotypen und einer universellen (nichthypothesenbasierten) phänotypischen Bestimmung der Virulenz unterscheiden.

Ein weiterer wichtiger Aspekt ist eine Bestimmung der Empfindlichkeit von Viren gegenüber antiviralen Mitteln und eine Bestimmung der Zellzytotoxizität eines antiviralen Mittels. Ähnlich wie bei der Antibiotikagabe bei bakteriellen Infektionen wird die Gabe von antiviralen Mitteln in der Regel empirisch begonnen und bei Bedarf nach den Ergebnissen der antiviralen Resistenztestung angepasst. Das bedeutet, dass im Falle einer Resistenz gegenüber einem antiviralen Mittel (zum Beispiel ein Virostatikum) auf ein anderes antivirales Mittel umgestellt wird. Nicht selten zeigt erst das Ausbleiben einer Verbesserung des Zustandes des behandelten Organismus einen Verdacht auf die Resistenz des Erregers und gibt damit einen Anlass zu einer antiviralen Empfindlichkeitstestung. Auch hier können phänotypische Tests helfen, vorausgesetzt sie stehen zur Verfügung.

Neben dem Nachweis der Virulenz von Bakterien, der Bestimmung der Empfindlichkeit von Viren gegenüber antiviralen Mitteln, die Bestimmung der Zelltoxizität von antibakteriellen, antifungalen oder antiviralen Mitteln, ist auch der Nachweis der Empfindlichkeit von TumorZellen gegenüber Chemotherapeutika ein Anwendungsbereich phänotypischer Tests.

Zum Nachweis der Zytotoxizität bzw. der Virulenz stehen diverse Methoden zur Verfügung.

In WO2018/099500 ist die Detektion von Wachstum von Mikroorganismen mittels eines einfachen MALDI-ToFs offenbart. Dies ist möglich, da die Mikroorganismen eine hohe Wachstums- und Zellteilungsrate aufweisen. Es hat sich aber gezeigt, dass das oben genannten Verfahren, welche für Mikroorganismen, entwickelt worden sind, schlecht geeignet zur Detektion von Wachstum von tierischen Zellen sind, da unter anderem das Wachstum und die Zellteilung von tierischen Zellen i.d.R. bedeutend langsamer als das Wachstum und die Zellteilung von Mikroorganismen ist und die Zellzahlen im Allgemeinen wesentlich geringer sind. Gewöhnlich basieren die Tests für tierische Zellen zur Bestimmung von Virulenz und Zytotoxizität von Substanzen auf einer der folgenden Methoden:
- molekularbiologische Methoden ,
- einer Bestimmung der Viabilität (Lebensfähigkeit als Anzahl der lebenden Zellen in einer Zellpopulation) der tierischen Zellen durch
   - Analyse der metabolischen Aktivität mittels Färbung (phänotypische Methode) oder MS ,
   - Detektion spezifischer pharmakodynamischer Biomarker mittels MS
   - Zellzählung der tierischen Zellen oder Bestimmung der Konfluenz bzw. anderer morphologischer Parameter der tierischen Zellen mittels mikroskopischen/ Bilderfassungs-Verfahren (phänotypische Methode)

Zum Beispiel erfolgt der Nachweis der mikrobiellen Virulenz in vielen Fällen immer noch phänotypisch. Ein genotypischer Nachweis durch molekularbiologische Methoden (z.B. Polymerase-Kettenreaktion, PCR) ist nur in Ausnahmen möglich, wo die Virulenzgene bekannt sind (z.B. Panton-Valentine-Leukozidin und Shiga-Toxin). Die meisten Virulenz-Charakteristika werden allerdings nicht durch ein bestimmtes Gen kodiert, sondern durch mehrere Mechanismen, die molekularbiologisch (genotypisch) zum Teil noch nicht komplett entschlüsselt sind. Hinzu kommt deren hochkomplexe Regulation im Erreger, so dass der sog. Genotyp (Gesamtheit der kodierten Erbanlagen) nicht zwingend mit dem Phänotyp (ausgeprägte Eigenschaften) übereinstimmt. Daher ist eine umfassende Virulenzbestimmung nur mittels phänotypischen Nachweismethoden möglich, die jedoch sowohl aufwendig und kostenintensiv sind als auch relativ viel Zeit bis zum Ergebnis benötigen. Somit ist die Virulenzbestimmung zurzeit fast ausschließlich Forschungslaboren vorbehalten, da diese die zur universellen Virulenz-Bestimmung notwendigen Voraussetzungen, wie z.B. kostspielige Instrumente und aufwändige Methoden, erfüllen können, jedoch Routine-Laboratorien i.d.R. nicht.

Die metabolische Aktivität kann beispielsweise mit dem MTT-Zytotoxizitätstest detektiert werden [ISO 10993-5:2009(E)]. Bei dem MTT-Test wird die gelbe Substanz MTT (3-(4,5-dimethylthiazol-2-yl)-2,5-diphenyltetrazoliumbromid) durch die lebensfähigen Zellen metabolisch zu der blau-violetten Substanz Formazan reduziert. Die Anzahl der lebensfähigen Zellen korreliert dabei mit der photometrisch gemessenen Farbintensität. Zum optischen Nachweis der Färbung werden in der Regel semi-hochdurchsatzfähige UV-Spektrophotometer eingesetzt. Der Einsatz dieser Tests ist allerdings durch mindestens zwei Probleme deutlich erschwert. Zum einen ist es die lange Dauer, die für die Durchführung eines Zytotoxizitätstests heutzutage benötigt wird. Für den oben genannten MTT-Test beträgt die Zeit bis Ergebnis ungefähr drei Tage. Zum anderen ist die Durchführung der Zytotoxizitätstests technisch hochkomplex und setzt die Verfügbarkeit spezieller apparativen Ausstattung voraus, so dass diese Untersuchungen hochspezialisierten Laboren vorbehalten bleiben müssen.

Neben phänotypischen Methoden werden indirekte MS-Verfahren eingesetzt, um die Wirkung von Wirkstoffen mittels Detektion einzelner spezifischer pharmakologischer Biomarker in eukaryotischen Zellen spezifisch nachzuweisen. Der Nachweis einzelner pharmakodynamischer Biomarker mittels MALDI-ToF wurde unter anderem von Unger M. et al. [Direct Automated MALDI Mass Spectrometry Analysis of Cellular Transporter Function: Inhibition of OATP2B1 Uptake by 294 Drugs, Anal. Chem. 2020.] beschrieben. Eukaryotische Zellen auf MALDI-Trägern wurden von Bergquist, J. [Cells on the target matrix-assisted laser-desorption/ionization time-of-flight massspectrometric analysis of mammalian cells grown on the target. Chromatographia 49, S41-S48 (1999).] beschrieben. Die Proben wurden im Hinblick auf die Entdeckung neuer Peptide und Proteine analysiert.

Zur Detektion einzelner pharmakologischer Biomarker zur Untersuchung der Wirkung von Wirkstoff werden zudem auch Verfahren mit hoch-ortsauflösende MALDI-MSI eingesetzt, um die Wirkung in den einzelnen Zellen darzustellen [Munteanu B. et al., Label-Free in Situ Monitoring of Histone Deacetylase Drug Target Engagement by Matrix-Assisted Laser Desorption lonization-Mass Spectrometry Biotyping and Imaging, Analytical Chemistry, 2014. Ramallo, C. et al.. Fast Nanoliter-Scale Cell Assays Using Droplet Microarray-Mass Spectrometry Imaging, Adv. Biology; 2021.] Hierzu muss jedoch nicht nur der spezifische pharmakologische Biomarker (z.B. Metabolit-Änderung), sondern auch der direkte Bezug zum Wirkstoff bekannt sein. Nachteilig ist zudem, dass ein pharmakologischer Biomarker nicht universell für alle Klassen von Wirkstoffen nutzbar ist. Zudem ist nachteilig, dass die Detektion von Biomarker i.d.R. keine oder keine zuverlässige Aussage über die Viabilität der Zelle oder die Zytotoxizität einer Substanz zulässt. Des Weiteren lassen sich insbesondere zytotoxische Faktoren wie Bakterien, Pilze oder Viren nur schwer mit Biomarkern von tierischen Zellen nachweisen. Zudem sind hoch-ortsauflösende MALDI-MSI-Systeme sehr teuer und in wenigen Routine-Laboren vorhanden.

Alternativ wird deshalb die Zellzählung als auch die Bestimmung der Konfluenz der Zellen (bzw. des Bedeckungsgrads) eingesetzt. Diese erfolgen mittels Live-Cell-Imaging Geräten, welche mittels Mikroskopie-Modulen, Bild- und Datenerfassungen sowie eine leistungsstarke Bild- und Datenanalysen die Wachstums- und Teilungsfähigkeit der Zellen weitgehend sicher erfassen [Carlsen J. et al., Optimized High-Contrast Brightfield Microscopy Application for Noninvasive Proliferation Assays of Human Cell Cultures, Assay and Drug Development Technologies, Jul 2020.215-225.]. Nachteilig ist hier, dass die erforderlichen Geräte sehr kostenintensiv sind, in wenigen Routine-Laboren vorliegen und das Personal zur Bedienung spezifisch geschult werden muss. Der Einsatz von einfachen Mikroskopen wäre zwar kostengünstiger bezüglich der Geräteanschaffung, jedoch ist die mikroskopische Einschätzung zeitintensiv und subjektiv.

Aufgabe der Erfindung ist es, die beschriebenen Nachteile des Standes der Technik zu beheben und insbesondere ein alternatives Verfahren zur Bestimmung der Anwesenheit von zytotoxischen Faktoren in einer analytischen Probe, von der nicht bekannt ist, ob Sie einen (oder auch mehrere) zytotoxische Faktoren enthält, bereitzustellen, welches zuverlässig, einfach, schnell und kostengünstig ist. Zudem ist es Aufgabe der Erfindung, die Wirkung von potenziell zytotoxischen Faktoren auf tierische Zellen und Ihre Toxizität zuverlässig, einfach, schnell und kostengünstig zu bestimmen. Des Weiteren ist es Aufgabe der Erfindung, ein System zur Ausführung eines solchen Verfahrens zu schaffen.

Die Aufgabe wird gelöst mit einem Verfahren mit den Merkmalen des unabhängigen
Patentanspruchs 1, einem Verfahren mit den Merkmalen des unabhängigen
Patentanspruchs 2, einem Verfahren mit den Merkmalen des unabhängigen
Patentanspruchs 3 sowie mit einem System mit den Merkmalen des unabhängigen
Patentanspruchs 20. Vorteilhafte Ausführungsformen sind den abhängigen Ansprüchen zu entnehmen.

Indem ein Verfahren zur Bestimmung der zytotoxischen Wirkung einer analytischen Probe auf tierische Zellen geschaffen wurde, dass folgende Schritte umfasst:
- einen Probenbereitstellungsschritt A, in dem eine massenspektrometrische Probe umfassend tierische Zellen, Nährmedium und die analytische Probe, welche potenziell einen zytotoxischen Faktor aufweist, auf mindestens einer Probenstelle eines massenspektrometrischen Probenträgers bereitgestellt wird;
- einen Kultivierungsschritt, in dem die massenspektrometrische Probe auf der Probenstelle des massenspektrometrischen Probenträgers in einer Kultivierungsvorrichtung inkubiert wird;
- einen Flüssigkeitsentfernungsschritt, in dem Restflüssigkeit der massenspektrometrischen Probe von der Probenstelle entfernt wird;
- einen Messschritt A, in dem ortsaufgelöste Massenspektren an einer Vielzahl von Messpositionen in mindestens einer Teilfläche der Probenstelle mittels eines ortsauflösenden Massenspektrometers aufgenommen werden, wobei bei Verwendung von einem matrix-basierten ortsauflösenden Massenspektrometer vor der Aufnahme der ortsaufgelösten Massenspektren im Messschritt A in einem vorgelagerten Probenpräparationsschritt A eine Präparation der Probenstelle durch ortstreue Auftragung einer Matrix auf mindestens die eine Teilfläche der Probenstelle erfolgt;
- einen ersten Auswerteschritt A, in dem jedes ortsaufgelöste Massenspektrum hinsichtlich des Vorhandenseins einer zellspezifischen massenspektrometrischen Signatur für die tierischen Zellen analysiert wird und jeder Messposition ein Zellpräsenzwert zugewiesen wird;
- einen zweiten Auswerteschritt A, in dem ein Bedeckungsgrad durch tierische Zellen für mindestens die Teilfläche der Probenstelle aus den Zellpräsenzwerten der Messpositionen bestimmt wird;
- und/oder einen dritten Auswerteschritt A, in dem eine Proliferationsfähigkeit aus dem bestimmten Bedeckungsgrad abgeleitet wird;
- einen vierten Auswerteschritt A, in dem die zytotoxische Wirkung der analytischen Probe auf die tierischen Zellen mittelbar oder unmittelbar aus dem bestimmten Bedeckungsgrad des zweiten Auswerteschrittes A abgeleitet wird, wobei hierzu die zytotoxische Wirkung der analytischen Probe auf die tierischen Zellen aus dem Ergebnis wenigstens einer der beiden Schritte abgeleitet wird: zweiter Auswerteschritt A, dritter Auswerteschritt A;
kann die Anwesenheit von zytotoxischen Faktoren in einer analytischen Probe zuverlässig, einfach, schnell und kostengünstig überprüft werden. Zudem kann die Wirkung von potenziell zytotoxischen Faktoren auf tierische Zellen überprüft werden und Ihre Toxizität zuverlässig, einfach, schnell und kostengünstig bestimmt werden.

Es hat sich gezeigt, dass sich ortsauflösende Massenspektrometer zur Bestimmung der zytotoxischen Wirkung von analytischen Proben nutzen lassen. Tierische Zellen wachsen und teilen sich im Vergleich zu Mikroorganismen in der Regel wesentlich langsamer, was nur zu einer langsamen Zellzahlzunahme an einer Probenstelle, wenn überhaupt, führt. Somit muss eine Kultivierung tierischer Zellen von massenspektrometrischen Proben für nicht-ortsauflösenden Massenspektrometer, welche ein Summenmassenspektrum pro Probenstelle erzeugen, entsprechend lange durchgeführt werden, um ausreichende Zellmengen über die gesamte Probenstelle zu erhalten. Dies verlängert die Analysezeit und macht ein derartiges Verfahren für die Anwendung impraktikabel. Auch ist das SummenMassenspektrum einer Probenstelle häufig nicht eindeutig, da massenspektrometrische Signale von tierischen Zellen, welche vor der Kultivierung bereitgestellt worden sind (cell seed), die Detektion einer zytotoxischen Wirkung auf tierische Zellen erschweren. Es wurde hingegen erkannt, dass die flächige Ausdehnung der tierischen Zellen auf einer Probenstelle und darüber deren Proliferationsfähigkeit und schließlich eine zytotoxische Wirkung bestimmt werden kann, um die zuvor beschriebenen Nachteile bei der Bestimmung der zytotoxischen Wirkung von analytischen Proben zu vermeiden. Bevorzugt wird die flächige Ausdehnung der tierischen Zellen auf einer Probenstelle, die Proliferationsfähigkeit, die Migrationsfähigkeit und/oder die zytotoxische Wirkung mittels eines ortsauflösenden Massenspektrometers bestimmt. Dazu wird bevorzugt der Bedeckungsgrad zu einem bestimmten Zeitpunkt einer Probenstelle und/oder die daraus abgeleitete Proliferationsfähigkeit bestimmt. Besonders bevorzugt wird die Ausdehnung der tierischen Zellen auf der Probenstelle mittels Bestimmung des Bedeckungsgrades zu einem bestimmten Zeitpunkt bestimmt. Bevorzugt ist die Gesamtfläche der Probenstelle bekannt. Dadurch können das Zellwachstum und die Zellvermehrung auch bei den tierischen Zellen schnell detektiert werden. Insbesondere lässt sich dies besonders bei adhärent proliferierenden Zellen verwenden. Bevorzugt sind die tierischen Zellen deshalb adhärent proliferierende (adhärent wachsende) tierische Zellen.

Die Detektion mittels eines ortsauflösenden Massenspektrometers ist zudem schneller und einfacher als die bislang dazu eingesetzten mikroskopischen oder bildgebenden Verfahren und in bestimmten Fällen auch günstiger. So sind häufig bereits in den Laboren vorhandene Massenspektrometer durch eine Softwaremodifizierung dazu in der Lage, das Verfahren durchzuführen. Zudem ist das Verfahren universell einsetzbar.

Um eine ungleichmäßige Probenverteilung auf der Probenstelle auszuschließen, zu statistischen Zwecken und aus Gründen der Messgenauigkeit werden zwar selbst bei nicht-ortsauflösenden MALDI-ToFs bereits in der Regel an mehreren Messpositionen einer Probenstelle Messungen durchgeführt, diese werden jedoch im Rahmen der Datenverarbeitung oder Auswertung zu einem Summenparameter/MS Spektrum verrechnet. Die räumliche Information bleibt hier somit ungenutzt. Bei vielen nicht-ortsauflösenden MALDI-ToFs liegt somit die gerätetechnische Voraussetzung in der Regel vor. Es wurde erkannt, dass häufig eine Anpassung der Software ausreichend ist, um ein nicht-ortsauflösenden MALDI-ToF-MS in ein ortsauflösendes MALDI-ToF-MS für das beschriebene Verfahren umzuwandeln.

Des Weiteren wurde erkannt, dass zur Detektion der tierischen Zellen und somit der zytotoxischen Wirkung nahezu beliebige, zellspezifische massenspektrometrische Signaturen der tierischen Zelle geeignet sind, da lediglich das Vorhandensein bzw. das Fehlen der Zelle für mehrere Messpositionen bestätigt werden muss, um auf eine zytotoxische Wirkung schließen zu können. Lediglich die Präsenz oder Abwesenheit der Zelle muss detektiert werden. Spezifische pharmakologische Biomarker, wie z.B. eine Erhöhung bestimmter Metabolite, werden nicht benötigt. Es werden zum Beispiel keine Änderungen von Konzentrationen an Biomarkern verfolgt, um Rückschlüsse auf die Wirkung auf den metabolischen Zustand der Zelle schließen zu können. Somit sind keine Kenntnisse über spezifische metabolische Aktivitäten oder spezifische Biomarker, die z.B. mit dem zytotoxischen Faktor in metabolisch Verbindung stehen, erforderlich. Es sind auch keine Informationen über den zytotoxischen Faktor selbst und dessen Wirkweise auf die tierischen Zellen, d.h. z.B. auf den Metabolismus, erforderlich. Auch sind Kenntnisse über Wechselwirkungen bei mehreren vorliegenden zytotoxischen Faktoren nicht erforderlich. Der zytotoxische Faktor muss nicht einmal bekannt sein. Dadurch ist das Verfahren universell für zytotoxische Faktoren einsetzbar.

Es werden eine oder mehrere zellspezifische massenspektrometrische Signaturen verwendet, um auf das Vorhandensein einer Zelle an einer Messposition rückzuschließen. Daraus wird wiederum der Bedeckungsgrad (Anspruch 1 und Anspruch 2) oder die Zell-Ausdehnungsweite (Anspruch 3) bestimmt, um auf die Proliferationsfähigkeit der Zelle, die Migrationsfähigkeit der Zelle und/oder die zytotoxische Wirkung einer analytischen Probe zu schließen.

In einer bevorzugten Anwendung des Verfahrens ist es zu Beginn unbekannt, ob eine analytische Probe einen zytotoxischen Faktor mit einer zytotoxischen Wirkung aufweist. Das Vorhandensein einer zytotoxischen Wirkung auf die tierischen Zellen wird mittels des Verfahrens bestimmt. Das Feststellen einer zytotoxischen Wirkung auf die tierischen Zellen mittels des Verfahrens zeigt das Vorliegen mindestens eines zytotoxischen Faktors an.

Aus der zytotoxischen Wirkung auf die tierischen Zellen bzw. dem Vorliegen eines zytotoxischen Faktors ist das Vorhandensein eines virulenten Erregers in der analytischen Probe direkt ableitbar..

In einer weiterhin bevorzugten Anwendung des Verfahrens wird festgestellt, ob ein spezifischer potenziell zytotoxisch wirkender Faktor ein zytotoxischer Faktor mit einer tatsächlichen zytotoxischen Wirkung auf die tierischen Zellen ist. Zum anderen kann auch eine Wirkung von bekannten zytotoxisch wirkenden Faktoren auf eine bestimmte Art von tierischen Zellen auf weitere Arten von tierischen Zellen mit diesem Verfahren überprüft werden. Des Weiteren können auch potenziell den zytotoxischen Faktor neutralisierende Faktoren auf Ihre Wirkung auf den zytotoxischen Faktor mit diesem Verfahren getestet werden. So kann das Verfahren nach den Ansprüchen 1,2 und/oder 3 und/oder deren Unteransprüchen zum Beispiel und/oder das System nach Anspruch 21 zur Virulenz-Phänotyp-Bestimmung verwendet werden. Ebenfalls können Medikamente oder in Ihrer Wirkung unbekannte Substanzen auf ihre Wirkung getestet werden. Besonders vorteilhaft ist das Verfahren bei der Detektion von komplexen multikausalen Virulenzen. So ist das Verfahren auch dazu geeignet, die zytotoxische Wirkung bei Anwesenheit von multiplen zytotoxischen Faktoren, die Ihre zytotoxische Wirkung lediglich im Zusammenspiel zeigen, zu detektieren.

Es wurde zudem erkannt, dass bei Nutzung eines matrix-basierten Massenspektrometers eine ortstreue Auftragung der Matrix auf der Probe erforderlich ist, damit die räumliche Auflösung des massenspektrometrischen Signals in der Fläche, das heißt die Position der tierischen Zellen auf der Probenstelle, nicht verfälscht wird und Zellpräsenzwerte für jede Messposition und/oder der Bedeckungsgrad in diesem Verfahren zuverlässig ermittelt werden können. Bei der ortstreuen Auftragung der Matrix wird die Matrix derart aufgetragen, dass eine flächige Durchmischung der Zellinhalte und Zellbestandteile der tierischen Zellen auf der Probenstelle (weitgehend) vermieden wird.

Bevorzugt umfassen die beanspruchten Verfahren die jeweiligen Verfahrensschritte. Alternativ bevorzugt bestehen die beanspruchten Verfahren aus den jeweiligen Verfahrensschritten. Bevorzugt werden die beanspruchten Verfahren in der Reihenfolge des Anspruchswortlauts ausgeführt.

Durch das ortsauflösende Massenspektrometer werden ortsaufgelöste Massenspektren erzeugt. Ortsaufgelöst bedeutet bevorzugt in der Fläche aufgelöst. Die Messpositionen werden dabei zur Messung gezielt angesteuert. Bevorzugt werden die Ortskoordinaten der Messpositionen erfasst. Bevorzugt werden die Ortskoordinaten der Messpositionen jedem ortsaufgelöste Massenspektrum zugeordnet. Das ortsauflösende Massenspektrometer vermisst bevorzugt gezielt mehrere Messpositionen einer Probenstelle und weist bevorzugt jedem aufgenommenen Massenspektrum die Ortskoordinaten der jeweils zugehörigen Messposition zu. Alternativ bevorzugt weist das ortsauflösende Massenspektrometer jeder Messposition einer Probenstelle Ortskoordinaten zu und weist bevorzugt jedem aufgenommen Massenspektrum eine Messposition zu. Somit ist jedes erzeugte ortsaufgelöste Massenspektrum einer definierten Messposition auf der Probenstelle zuordenbar. Des Weiteren werden die Positionskoordinaten der Messpositionen bevorzugt gespeichert. In einer besonderen alternativen Ausgestaltung der Erfindung nach Anspruch 1 und 2 kann es jedoch genügen, die ortsaufgelösten Massenspektren den Messpositionen einer Probenstelle zuzuordnen, ohne die genauen Ortskoordinaten der Messposition zu erfassen. Entscheidend ist dann dabei, dass die Messpositionen vom ortsauflösenden Massenspektrometer gezielt, das heißt entsprechend vorgegebenen Messpositionen, vermessen werden und die Einzelmassenspektren der Messpositionen nicht zu einem Summenmassenspektrum verrechnet werden, sondern der jeweiligen Messung zugeordnet werden.

Gemäß Anspruch 3 werden die Ortskoordinaten jeder Messposition erfasst und den ortsaufgelöste Massenspektren zugeordnet.

Das ortsauflösende Massenspektrometer kann ein matrix-basiertes ortsauflösendes Massenspektrometer oder ein nicht-matrix-basiertes ortsauflösendes Massenspektrometer sein. Die matrix-basierten ortsauflösenden Massenspektrometer sind Massenspektrometer mit matrix-basierten lonisationsverfahren. Das heißt, dass sie zur Ionisation der Moleküle der massenspektrometrischen Probe eine Matrix, i.d.R. flüssig aufgetragen, verwenden. Dazu gehören zum Beispiel MALDI-ToF Massenspektrometer. Die nicht-matrix-basierten ortsauflösende Massenspektrometern sind Massenspektrometer mit nicht-matrix-basierten lonisationsverfahren. Das heißt, dass sie zur Ionisation der Moleküle der massenspektrometrischen Probe keine Matrix verwenden. Dazu gehören zum Beispiel DESI Massenspektrometer. Bevorzugt ist das ortsauflösende Massenspektrometer ein matrix-basiertes ortsauflösendes Massenspektrometer. Besonders bevorzugt ist das matrix-basierte ortsauflösende Massenspektrometer ein MALDI-ToF MS. Hierzu ist das MALDI-ToF MS derart eingerichtet, dass es ortsaufgelöst Massenspektren aufnehmen kann. Weiterhin bevorzugt ist das ortsauflösende Massenspektrometer ein MALDI-ToF MS mit Imaging Funktion, welche die Aufnahme von ortsaufgelösten Massenspektren mit einer hohen Flächendichte und somit präzise Messungen ermöglichen. Sie sind einfach in der Handhabung und schnell.

Weiterhin können ggfs. nicht-ortsauflösende MALDI-ToF MS durch einfache softwareseitige Umrüstung zu ortsauflösenden MALDI-ToF MS umfunktioniert werden. Einfache nicht-ortsauflösende MALDI-ToF MS haben den Vorteil, dass sie verhältnismäßig günstig in der Anschaffung sind, einfach in der Bedienung und bereits in vielen Laboren z.B. zur Identifizierung von Mikroorganismen etabliert ist.

Die Adhäsion und die Proliferation der tierischen Zellen im Verfahren kann in einem Verfahrensschritt während des Kultivierungsschrittes erfolgen. Auch kann die Adhäsion und die Proliferation der tierischen Zellen zweischrittig erfolgen, indem die tierischen Zellen im Probenbereitstellungsschritt A,B bereits adhäriert bereitgestellt werden und lediglich die Proliferation der adhärierten tierischen Zellen im Kultivierungsschritt erfolgt. In einer bevorzugten Ausgestaltung liegen bei der Bereitstellung der massenspektrometrischen Probe im Probenbereitstellungsschritt A,B Bestandteile der massenspektrometrischen Probe, wie die tierischen Zellen, das Nährmedium und/oder der zytotoxische Faktor bereits auf der Probenstelle vor. Dies kann zum Bespiel in getrockneter, gekühlter und/oder gefrorener Form erfolgen. So kann ein massenspektrometrischer Probenträger bereits mit proliferationsfähigen tierischen Zellen Kunden kommerziell zur Verfügung gestellt werden. Des Weiteren werden in einer weiteren bevorzugten Ausgestaltung die Bestandteile der massenspektrometrischen Probe, wie die tierischen Zellen, das Nährmedium und/oder der zytotoxische Faktor, im Probenbereitstellungsschritt A,B auf die Probenstelle aufgetragen. Mindestens ein Bestandteil der massenspektrometrischen Probenstelle wird in dieser bevorzugten Ausgestaltung gelöst oder suspendiert in einer Flüssigkeit, bevorzugt Puffer, Nährmedium oder Wasser, bereitgestellt. In einer besonders bevorzugten Ausgestaltung liegen die tierischen Zellen und das Nährmedium bereits vor und die analytische Probe oder der zytotoxische Faktor wird aufgetragen. So können die tierischen Zellen mit dem Nährmedium zum Beispiel gefriergetrocknet vorliegen. Zusätzlich kann die Probenstelle mit einem wasserlöslichen Klebemittel versehen sein, damit die getrockneten oder gefriergetrockneten tierischen Zellen und das Nährmedium auf der Probenstelle lokalisiert bleiben. In einer weiteren bevorzugten Ausgestaltung werden alle Bestandteile der massenspektrometrischen Probe, umfassend tierischen Zellen, das Nährmedium und der zytotoxische Faktor, auf die Probenstelle aufgetragen. Bevorzugt werden die tierischen Zellen dazu suspendiert im einem flüssigen Nährmedium aufgetragen.

Die tierischen Zellen sind mindestens vor Auftragung der analytischen Probe oder des zytotoxischen Faktors lebensfähig und, proliferationsfähig und/oder migrationsfähig.

Das Nährmedium umfasst sämtliche Bestandteile, die die tierischen Zellen für Ihre Proliferation und/oder Migration benötigen. Das Nährmedium kann synthetisches oder komplexes Nährmedium sein. Des Weiteren kann das Nährmedium auch Bestandteile enthalten, die zusätzlich die Kultivierung von Bakterien oder Pilzen als zytotoxische Faktoren ermöglichen.

Die Kultivierung der massenspektrometrischen Probe auf der Probenstelle des massenspektrometrischen Probenträgers in der Kultivierungsvorrichtung im Kultivierungsschritt dient der Schaffung von optimalen Kultivierungsbedingungen, um eine Proliferation der tierischen Zellen zu ermöglichen. Bevorzugt wird die Kultivierung der massenspektrometrischen Proben inklusive der Referenzproben gleichzeitig beendet. Dadurch kann der Bedeckungsgrad und/oder die Zell-Ausdehnungsweite zu einem bestimmten Zeitpunkt auf einfache Weise bestimmt werden. Damit ist eine einfache Ermittlung der Proliferationsfähigkeit und/oder Migrationsfähigkeit möglich. Die Dauer der Kultivierung und die Kultivierungsbedingungen sind an die tierischen Zellen angepasst. Bevorzugt ist die Kultivierungsdauer derart gewählt, dass bei einer Referenzprobe ohne zytotoxischen Faktor, die Teilfläche oder die gesamte Probenstelle am Ende der Kultivierung weitgehend vollständig mit tierischen Zellen bedeckt ist. Dazu stellt die Kultivierungsvorrichtung bevorzugt optimale Wachstumsbedingungen für die tierischen Zellen zur Verfügung. Hierzu zählen unter anderem Luftfeuchtigkeit, Temperatur, Kohlenstoffdioxidgehalt und Sauerstoffgehalt. So kontrolliert die Kultivierungsvorrichtung die Umgebungsbedingungen wie Luftfeuchtigkeit, Temperatur, CO₂-Gehalt in der Kultivierungsvorrichtung, O₂-Gehalt in der Kultivierungsvorrichtung, und/oder weitere Umgebungsbedingungen. Als Kultivierungsvorrichtung können insbesondere CO₂-Brutschränke dienen. Alternativ kann die Kultivierungsvorrichtung auf Bedingungen ähnlich denen des menschlichen oder des tierischen Körpers, aus dem die Ursprungsprobe gewonnen wurde, eingestellt werden. Bevorzugt ist die Luft in der Kultivierungsvorrichtung mit Wasser derart gesättigt, sodass die Flüssigkeit der massenspektrometrischen Probe, insbesondere des Nährmediums, im Wesentlichen nicht verdunstet.

Nachdem die tierischen Zellen die Bedingungen und die Zeit hatten, wachsen, sich teilen und/oder migrieren zu können, wird die Restflüssigkeit entfernt. Die Restflüssigkeit ist eine Mischung der flüssigen Bestandteile der Bestandteile der massenspektrometrischen Probe. In ihnen waren zum Beispiel die tierischen Zellen suspendiert und/oder der zytotoxische Faktor gelöst. Die flüssigen Bestandteile der Bestandteile der massenspektrometrischen Probe können das Nährmedium, Puffer, Wasser, Blut, Urin, Sekrete und andere flüssige Bestandteile umfassen. Die Entfernung der Restflüssigkeit erfolgt bevorzugt durch Kontakt mit einem saugfähigen Material und/oder durch Trocknung. Die Trocknung kann z.B. durch einfache Lufttrocknung, spezifischer Belüftung und/oder durch thermische Trocknung erfolgen. Durch Entfernung der Restflüssigkeit, insbesondere des Nährmediums, sind die zellspezifischen massenspektrometrischen Signale leichter zu erfassen. Zudem ist eine weitgehend trockene Oberfläche vorteilhaft für eine ortstreue Auftragung einer Matrix. Entscheidend ist, dass ausreichend Restflüssigkeit entfernt wird, sodass bei nicht-matrix-basierten ortsauflösenden Massenspektrometern eine Messung nicht durch Flüssigkeitsreste negativ beeinflusst wird und bei matrix-basierten ortsauflösenden Massenspektrometern sich kein Flüssigkeitsfilm mehr auf den tierischen Zellen befindet, wodurch eine ortstreue Auftragung der Matrix nicht möglich wäre. Bevorzugt wird die Restflüssigkeit bei Nutzung von adhärent wachsenden tierischen Zellen abgesaugt. Dadurch wird weitgehend vermieden, dass sich in Suspension befindliche tierische Zellen auf der Probenstelle abscheiden und das Ergebnis verfälschen. Dadurch wird die Genauigkeit des Verfahrens gesteigert. Auch werden störende nicht-zellspezifischen massenspektrometrische Signale vom Nährmedium und den restlichen Bestandteilen der massenspektrometrischen Probe reduziert. Alternativ wird die Restflüssigkeit durch Trocknung entfernt werden. Dies kann unter anderem vorteilhaft bei Nutzung von in Suspension-wachsenden tierischen Zellen sein, sodass die suspendierten Zellen auf die Probenstelle sedimentieren und an die Oberfläche adsorbieren können. Prinzipiell ist die Trocknung zur Flüssigkeitsentfernung auch bei Auftragung von adhärent wachsenden Zellen in suspendierter Form möglich, wenn die anfängliche Zellzahl und die Kultivierungsdauer aufgrund des Verbleibens nicht-adhärierter tierischer Zellen auf der Probenstelle entsprechend angepasst sind. Insbesondere bei Verwendung von matrix-basierten ortsauflösenden Massenspektrometern erfolgt bevorzugt nach Absaugung noch eine Trocknung, um die Restflüssigkeit zuverlässig zu entfernen. Dies erhöht die Auflösung bei anschließender ortstreuer Auftragung der Matrix.

Bei Verwendung von einem matrix-basierten ortsauflösende Massenspektrometer ist dem Messschritt A ein Probenpräparationsschritt A vorgelagert. Dieser Schritt fehlt bei nicht-matrix-basierten ortsauflösende Massenspektrometern. Im Probenpräparationsschritt A erfolgt eine Präparation der Probenstelle durch ortstreue Auftragung einer Matrix auf mindestens eine Teilfläche der Probenstelle. Dabei bleibt ein Vermischen der Analyten der einer Messposition der Probenstelle mit Analyten anderen Messpositionen der Probenstelle weitgehend aus. Alle Analyten verbleiben weitgehend an Ihrer Position.

Bei der ortstreuen Auftragung wird die Matrix nicht in Form eines oder weniger großen Tropfen auf die gesamten Probestelle aufgetragen. Die Auftragung erfolgt stattdessen in zahlreichen kleineren Einheiten, bevorzugt entweder mittels Tröpfchen oder durch Sublimation, verteilt über die Probenstelle. Die ortstreue Auftragung der Matrix erfolgt bevorzugt durch Auftragung einer Vielzahl von Matrixtröpfchen pro Probenstelle. Dabei werden die Vielzahl von Matrixtröpfchen bevorzugt flächig verteilt über die Probenstelle oder die Teilfläche der Probenstelle aufgetragen. Die Auftragung der Matrixtröpfchen erfolgt je nach Auftragsmethode gezielt oder ungezielt. Dadurch ist am Ende des Auftragungsprozesses bevorzugt die gesamte Teilfläche der Probenstelle oder die gesamte Probenstelle mit Matrixtröpfchen bedeckt, sodass alle tierischen Zellen der Teilfläche oder der gesamten Probenstelle der massenspektrometrischen Bestimmung zugänglich sind. Entscheidend bei der ortstreuen Auftragung der Matrix ist, dass die tierischen Zellen nicht von einem die Probenstelle überspannenden Tropfen überdeckt sind.

Die Matrix bewirkt in der Regel unter anderem ein Aufschließen der Zellen. Die ortstreue Auftragung der Matrix bewirkt, dass der Zellinhalt und die Fragmente jeder tierischen Zelle sich nicht über weite Teile die Probenstelle ausbreiten und Messpositionen, welche keine Zellen aufweisen, überdecken. Dadurch wird eine Verfälschung der Messung vermieden. Durch eine diffusionsgetriebene breite Verteilung der Zellinhalte auf der gesamten Probenstelle im Falle der Auftragung eines großen Matrixtropfen auf die gesamte Probenstelle wäre eine genaue räumliche Auflösung der Positionen der Zellen nicht mehr möglich. Bevorzugt ist der Durchmesser der Matrixtröpfchen signifikant kleiner als der Durchmesser der tierischen Zellen. Dies unterstützt, dass der Zellinhalt weitgehend innerhalb der (ursprünglichen) Zellgrenze verbleibt. Eine Auftragung von Matrix ist für nichtmatrixbasierten MS, das heißt MS mit lonisationsverfahren wie zum Beispiel DESI oder SIMS, nicht erforderlich.

Durch die Aufnahme von ortsaufgelösten Massenspektren an einer Vielzahl von Messpositionen in mindestens einer Teilfläche der Probenstelle mittels eines ortsauflösenden Massenspektrometers im Messschritt A wird für die folgenden Auswerteschritte jeder vermessenen Messposition auf der Probenstelle Ihr eigenes Massenspektrum zugewiesen. Es können eine Teilfläche der Probenstelle oder die gesamte Probenstelle Messpositionen aufweisen. Bevorzugt wird die Probenstelle oder die Teilfläche der Probenstelle nicht vollflächig vermessen, sondern stichprobenartig in Form eines Rasters vermessen - d.h. abgerastert. Die Anzahl der Messpositionen und/oder das Raster der Messpositionen richtet sich nach dem ortsauflösenden Massenspektrometer als auch nach der gewünschten Präzision und Geschwindigkeit des Verfahrens. Bevorzugt wird eine Teilfläche der Probenstelle systematisch abgerastert. Dies beschleunigt das Verfahren. Alternativ kann die gesamte Probenstelle systematisch abgerastert. Dies erhöht die Zuverlässigkeit des Verfahrens.

Durch Analyse jedes ortsaufgelösten Massenspektrums hinsichtlich des Vorhandenseins einer zellspezifischen massenspektrometrischen Signatur für die tierischen Zellen im ersten Auswerteschritt A wird für die spätere Bestimmung des Bedeckungsgrades oder der Zell-Ausdehnungsweite die Datengrundlage geschaffen. Jedes ortsaufgelöste Massenspektrum wird dabei bevorzugt für sich allein analysiert. Entscheidend dabei ist, dass die Massenspektren ortsaufgelöst verbleiben. Mehrere Massenspektren für die gleiche Messposition können dabei verrechnet werden. Die Verrechnung kann dabei insbesondere eine Mittelung umfassen. Massenspektren unterschiedlicher Messpositionen werden jedoch nicht zusammengefasst.

Bevorzugt besteht die zellspezifische massenspektrometrische Signatur weitgehend oder vollständig aus einer Auswahl von massenspektrometrischen Signalen im aufgenommenen ortsaufgelösten Massenspektrum, welche eindeutig der tierischen Zelle zuzuordnen sind. Weiterhin bevorzugt weisen diese Signale eine signifikant höhere Intensität als das Hintergrundrauschen und/oder als eine Vielzahl der nicht-zellspezifischen massenspektrometrischen Signale auf. Die zellspezifischen massenspektrometrischen Signale der ortsaufgelösten Massenspektren der tierischen Zellen können sich teilweise in Abhängigkeit der Lage der Messposition auf der tierischen Zelle oder auf der aufgeschlossenen tierischen Zelle unterscheiden, zeigen jedoch immer die Anwesenheit der tierischen Zelle an. Bevorzugt weisen zellspezifische massenspektrometrische Signaturen eine hohe Reproduzierbarkeit bei wechselnden analytischen Proben auf. Weiterhin bevorzugt weisen zellspezifische massenspektrometrische Signaturen auch eine hohe Reproduzierbarkeit bei einer Vielzahl verschiedener tierischer Zellen auf. Dadurch wird die Zuverlässigkeit des Verfahrens erhöht. Die zellspezifischen massenspektrometrischen Signale und/oder zellspezifischen massenspektrometrischen Signaturen können zudem von der Art/Typ der verwendeten tierischen Zelle abhängen. Sie können spezifisch für bestimmte Gewebearten, Zellkulturlinien, Organismen oder weitere Klassifizierungsgruppen von Zellen sein.

Ausgewählte zellspezifischen massenspektrometrischen Signaturen des gesamten ortsaufgelösten Massenspektrums können zu einer oder mehreren zellspezifischen massenspektrometrischen Signaturen zusammengefasst werden. Die zellspezifischen massenspektrometrischen Signale und zellspezifischen massenspektrometrischen Signaturen liegen bevorzugt bereits als Referenzwerte in einer Datenbank einer Datenverarbeitungseinheit vor und können bei der Analyse herangezogen werden.

Bevorzugt wird die gleiche zellspezifische massenspektrometrische Signatur für alle Messpositionen einer Probenstelle verwendet. Die zellspezifischen massenspektrometrischen Signaturen einzelner Messpositionen oder Probenstellen können sich in einer alternativen Ausgestaltung auch voneinander unterscheiden. Es können auch mehrere zellspezifische massenspektrometrische Signaturen verwendet werden, welche eine andere Auswahl an zellspezifischen massenspektrometrischen Signalen aufweisen. Entscheidend bei der Auswahl der zellspezifischen massenspektrometrischen Signatur oder massenspektrometrischen Signaturen ist, dass für jede Messposition eindeutig bestimmt werden kann, ob eine tierische Zelle vorliegt oder nicht.

In einem Spezialfall kann die zellspezifische massenspektrometrische Signatur aus lediglich einem zellspezifischen massenspektrometrischen Signal bestehen. Dies vereinfacht die Analyse der ortsaufgelösten Massenspektren, kann jedoch die Robustheit des Verfahrens senken. Insbesondere kann die Robustheit des Verfahrens bei Nutzung von biologischen Ursprungsproben, wie zum Beispiel Stuhlproben, durch Überlagerung des zellspezifischen massenspektrometrischen Signals mit Signalen von Bestandteilen der Ursprungsprobe herabgesetzt sein. Bevorzugt umfasst die zellspezifische massenspektrometrische Signatur deshalb eine Vielzahl an zellspezifischen massenspektrometrischen Signalen und/oder es werden mehrere zellspezifische massenspektrometrische Signaturen verwendet. Insbesondere bei Proben mit hohem Anteil an nicht-zellspezifischen massenspektrometrischen Signalen mit einem ähnlichen m/Z-Verhältnis wie die zellspezifischen massenspektrometrischen Signale sind mehrere zellspezifische massenspektrometrische Signale für die zellspezifische massenspektrometrische Signatur vorteilhaft. Bevorzugt werden die markantesten massenspektrometrische Signale oder das markanteste massenspektrometrische Signal der zellspezifischen massenspektrometrischen Signale für die zellspezifische massenspektrometrischen Signatur verwendet. Bevorzugt weisen das eine oder die Vielzahl an zellspezifischen massenspektrometrischen Signalen eine hohe Intensität gegenüber dem Hintergrundrauschen auf und/oder sind ausreichend von nicht-zellspezifischen Signalen beabstandet. Eine hohe Intensität gegenüber dem Hintergrundrauschen bedeutet, dass die Intensität eines massenspektrometrischen Signals mindestens zweimal so hoch wie das durchschnittliche Hintergrundrauschen ist. Bevorzugt ist das massenspektrometrische Signal mindestens fünfmal so hoch wie das durchschnittliche Hintergrundrauschen. Weiterhin bevorzugt ist das massenspektrometrische Signal mindestens zehnmal so hoch wie das durchschnittliche Hintergrundrauschen. Besonders bevorzugt ist das massenspektrometrische Signal mindestens 50-mal so hoch wie das durchschnittliche Hintergrundrauschen. Zusätzlich sind die zellspezifischen massenspektrometrischen Signale der zellspezifischen massenspektrometrischen Signatur bevorzugt ausreichend von nicht-zellspezifischen massenspektrometrischen Signalen beabstandet. Bevorzugt besteht die zellspezifische massenspektrometrische Signatur zumindest in Teilen oder vollständig aus benachbarten zellspezifischen massenspektrometrischen Signalen. Sie kann jedoch auch nicht direkt benachbarte zellspezifische massenspektrometrische Signale umfassen.

Je nachdem, an welchem Teil der Zelle das ortsaufgelöste Massenspektrum aufgenommen worden ist, können sich die erhaltenen Massenspektren unterscheiden. In einer weiteren bevorzugten Ausgestaltungsform können deshalb verschiedene zellspezifische massenspektrometrische Signaturen zur Bestimmung einer Zellpräsenz verwendet werden.

In einem weiteren Spezialfall kann die zellspezifische massenspektrometrische Signatur aus sämtlichen zellspezifischen massenspektrometrischen Signalen im aufgenommenen ortsaufgelösten Massenspektrum bestehen. In diesem Spezialfall wird jedoch im Analyseschritt bevorzugt eine Gewichtung verwendet, da die Anwesenheit einzelner zellspezifischer massenspektrometrischer Signale von nicht-zellspezifischen massenspektrometrischen Signalen, z.B. des Nährmediums, überdeckt werden kann. Des Weiteren wird das zellspezifische Signal oder die zellspezifische Signatur bevorzugt nicht oder weitgehend nicht durch andere Bestandteile der massenspektrometrischen Probe, wie zum Beispiel das Nährmedium oder den zytotoxischen Faktor, erzeugt. Somit fehlen im Wesentlichen die zellspezifischen massenspektrometrische Signale der zellspezifischen massenspektrometrischen Signatur in den Massenspektren des Nährmediums, der analytischen Probe oder der sonstigen Bestandteile in der massenspektrometrischen Probe. Dies lässt auf einfache Weise die eindeutige Aussage zu, ob tierische Zellen an der Messposition vorhanden waren oder nicht.

Bestandteile der massenspektrometrischen Probe sind zum Beispiel das Nährmedium, die tierischen Zellen, die analytische Probe, und weitere Bestandteile, welche für die Kultivierung oder die Messung erforderlich sind. Weiterhin umfassen Bestandteile der massenspektrometrischen Probe auch Teile der Bestandteile der massenspektrometrischen Probe. So kann dies Puffer, Wasser, Feststoffe, um weitere Teile umfassen, aus welchen die Bestandteile der massenspektrometrischen Probe bestehen.

Des Weiteren weisen die tierischen Zellen Bestandteile der tierischen Zellen auf, aus welchen die tierischen Zellen aufgebaut sind oder die sie beinhalten. Die Bestandteile der tierischen Zellen können zum Beispiel Proteine, Peptide, Nukleinsäuren, Lipide, Saccharide Ribosomen, Enzyme, und/oder deren Bausteine, Fragmente und/oder deren metabolische Produkte, und/oder das Zytoplasma umfassen. Bevorzugt zählen sekretierte Stoffe der Zelle nicht zu den Bestandteilen der Zelle. Weiterhin bevorzugt wird die Menge, Anwesenheit oder Produktion des Bestandteils der tierischen Zelle, welcher das zellspezifische Signal erzeugt, in der lebenden tierischen Zelle nicht von Bestandteilen der massenspektrometrischen Probe, insbesondere den zytotoxischen Faktor, beeinflusst.

Alternativ können die Bestandteile der massenspektrometrischen Probe auch einzeln vermessen werden. Die zellspezifischen massenspektrometrischen Signale sind durch Abgleich der Massenspektren von tierischen Zellen mit den Massenspektren von Nährmedium, zytotoxischen Faktoren und sonstigen Bestandteilen der Probe ermittelbar. Dazu wird zum Beispiel die zellspezifische massenspektrometrische Signatur an zwei Referenzproben ermittelt. Dabei weist die erste Referenzprobe keine tierischen Zellen auf, umfasst jedoch das Nährmedium und die weiteren Bestandteile der massenspektrometrischen Probe. Dabei weist die zweite Referenzprobe sämtliche Bestandteile der massenspektrometrischen Probe inklusive der tierischen Zellen auf, jedoch keinen zytotoxischen Faktor oder keine analytische Probe. Der Abgleich erfolgt dabei nach den dem erfahrenen Fachmann auf dem Gebiet der massenspektrometrischen Auswerteverfahren bekannten mathematischen Verfahren.

Basierend auf der Analyse jedes ortsaufgelösten Massenspektrums hinsichtlich des Vorhandenseins einer zellspezifischen massenspektrometrischen Signatur für die tierischen Zellen im ersten Auswerteschritt A,B wird bestimmt, ob an den vermessenen Messpositionen tierische Zellen bzw. aufgeschlossene tierische Zellen beim Messschritt A,B vorhanden waren oder nicht. Das Ergebnis wird dem Zellpräsenzwert zugewiesen. Analog geschieht dies für Massenspektren im ersten Auswerteschritt C und Messchritt C für Anspruch 2. Bevorzugt weist der Zellpräsenzwert zwei Werte auf: "Zelle vorhanden" oder "Zelle nicht vorhanden". Jeder vermessenen Messposition ist bevorzugt ein Zellpräsenzwert zugeordnet. Daraus wird der Bedeckungsgrad oder die Zell-Ausdehnungsweite bestimmt. Ebenfalls ist das Auswerten von Massenspektren, zum Beispiel die Analyse des Vorhandenseins bestimmter Peaks oder Intensitäten mit anschließender Bewertung, ob hier eine Zelle vorliegt oder nicht, von dem Merkmal 'Zuweisung eines Zellpräsenzwertes' umfasst. Bevorzugt muss der Zellpräsenzwert nicht explizit aufgeführt werden. Bevorzugt genügt zur Erfüllung dieses Merkmals die Bestimmung, dass an einer Messposition eine tierische Zelle vorliegt oder nicht.

Indem der Bedeckungsgrad durch tierische Zellen für mindestens die Teilfläche der Probenstelle aus den Zellpräsenzwerten der Messpositionen im zweiten Auswerteschritt A,C bestimmt wird, kann die Ausdehnung der tierischen Zellen auf der Probenstelle leicht bestimmt werden. Bevorzugt wird der Bedeckungsgrad für die gesamte Probenstelle ermittelt. Bevorzugt ist dazu die Gesamtfläche der Probenstelle bekannt.

Der Bedeckungsgrad kann im Falle der Abrasterung der gesamten Probenstelle direkt bestimmt werden oder im Falle der Abrasterung einer Teilfläche für die Probenstelle extrapoliert werden. Im Falle einer Bestimmung des Bedeckungsgrades für eine Teilfläche der Probenstelle ist bevorzugt die Größe der Teilfläche bekannt.

Der Bedeckungsgrad gibt an, welcher Anteil der Fläche der Probenstelle oder einer Teilfläche der Probenstelle mit tierischen Zellen belegt ist bzw. war (bei aufgeschlossenen Zellen). In einer bevorzugten Ausgestaltung ist der Bedeckungsgrad ein Korifluenzgrad. Hier wird der Bedeckungsgrad durch adhärent-wachsende tierischen Zellen bestimmt. In einfachster Weise kann der Bedeckungsgrad bestimmt werden, indem die Anzahl der Messpositionen mit positivem Zellpräsenzwert ins Verhältnis zur Anzahl der Messpositionen mit negativen Zellpräsenzwert gesetzt wird. Alternativ kann der Bedeckungsgrad auch bestimmt werden, indem die Zellpräsenzwerte ins Verhältnis zur Teilfläche gesetzt werden.

In einer weiteren alternativ bevorzugten Ausgestaltung umfasst das Verfahren einen zusätzlichen dritten Auswerteschritt A, in dem eine Proliferationsfähigkeit der tierischen Zellen aus dem bestimmten Bedeckungsgrad abgeleitet wird. Die zytotoxische Wirkung des zytotoxischen Faktors im vierten (bevorzugt letzten) Auswerteschritt wird dann bevorzugt aus der bestimmten Proliferationsfähigkeit der tierischen Zellen abgeleitet. Durch Einführung dieses Auswerteschrittes kann die Proliferationsfähigkeit auf einfache Weise ermittelt werden. Bevorzugt wird die Proliferationsfähigkeit in Grade abgestuft. Hierbei erfolgt der Abgleich des Bedeckungsgrades der massenspektrometrischen Probe mit einem Bedeckungsgrad einer Referenzprobe oder einem vorliegenden Referenzwert. Die vorliegenden Referenzwerte liegen bevorzugt in einer Datenbank vor. Bevorzugt umfasst die Referenzprobe die tierischen Zellen und das Nährmedium. Der Proliferationsgrad weist mindestens 2 Werte auf. Beim ersten Wert sind die Zellen nicht proliferationsfähig; d.h. der zytotoxische Faktor hat eine zytotoxische Wirkung auf die tierischen Zellen. Beim zweiten Wert sind die Zellen voll proliferationsfähig; d.h. die Proliferationsfähigkeit der tierischen Zellen ist unbeeinflusst vom zytotoxischen Faktor. Eine oder mehrere Abstufungen zwischen beiden Werten, die eine abgestuft beschränkte Proliferationsfähigkeit anzeigen, können ebenfalls vorgesehen sein. Bevorzugt erfolgt die Wiedergabe der Proliferationsfähigkeit in Prozentwerten, wobei 100% einer ungehinderten Proliferation entspricht.

Die zytotoxische Wirkung der analytischen Probe auf die tierischen Zellen wird in einem vierten (bevorzugt letzten) Auswerteschritt mittelbar oder unmittelbar aus dem bestimmten Bedeckungsgrad des zweiten Auswerteschrittes A,C abgeleitet. Hierzu wird die zytotoxische Wirkung der analytischen Probe auf die tierischen Zellen aus dem Ergebnis wenigstens einer der Schritte abgeleitet: zweiter Auswerteschritt A,C und/oder dritter Auswerteschritt A. Das bedeutet, dass in einer bevorzugten Ausgestaltungform die zytotoxische Wirkung der analytischen Probe auf die tierischen Zellen direkt aus dem Bedeckungsgrad abgeleitet wird. Dabei wird im Verfahren der dritten Auswerteschritt A nicht durchgeführt und nach dem zweiten Auswerteschritt A,C der vierte Auswerteschritt A,C ausgeführt. Dadurch kann die Ermittlung von Zwischenwerten umgangen und die Auswertung vereinfacht werden. In einer weiteren alternativen bevorzugten Ausgestaltungsform wird die zytotoxische Wirkung der analytischen Probe auf die tierischen Zellen aus dem ermittelten Proliferationsgrad aus dem dritten Auswerteschritt A, d.h. mittelbar aus dem Bedeckungsgrad, abgeleitet. Dadurch kann vor Bestimmung der zytotoxischen Wirkung der analytischen Probe die Proliferationsfähigkeit der tierischen Zellen festgestellt werden. Dies ist insbesondere bei abgestuften Bedeckungsgraden und als Zwischeninformation von Vorteil.

In einer weiteren alternativen bevorzugten Ausgestaltungsform wird die zytotoxische Wirkung der analytischen Probe auf die tierischen Zellen aus dem ermittelten Bedeckungsgrad im zweiten Auswerteschritt A,C und aus der Proliferationsfähigkeit im dritten Auswerteschritt A bestimmt, wobei beide Ergebnisse berücksichtigt werden. Die zytotoxische Wirkung wird bevorzugt in mehreren Stufen abgebildet. Zur Ableitung der zytotoxischen Wirkung kann zudem die Kultivierungszeit einbezogen werden.

Zusätzlich ist es bevorzugt, dass die tierischen Zellen adhärent wachsende tierische Zellen sind. Das Verfahren ist insbesondere vorteilhaft einsetzbar bei adhärent wachsenden tierischen Zellen, da die Proliferation dann vornämlich adhäsiv auf der Oberfläche der Probenstelle verläuft. Dies erhöht die Zuverlässigkeit des Verfahrens, da die adhärent-wachsenden tierischen Zellen weitgehend ortstreu auf der Probenstelle lokalisiert sind. Somit verbleiben die tierischen Zellen während des Verfahrens weitgehend an ihrer Position. Zudem haften Sie bereits bevorzugt während des Kultivierungsschrittes an der Oberfläche an und proliferieren dort und nicht in Suspension. Auch bilden adhärent wachsende Zellen in Suspension keine Aggregate wie Suspensionszellen. Auch zur alleinigen Bestimmung der Migrationsfähigkeit ist eine Adhäsion der tierischen Zellen erforderlich.

Gemäß einer weiteren vorteilhaften Ausgestaltung ist vorgesehen, dass die Vielzahl von Messpositionen in der mindestens einen Teilfläche der Probenstelle derart örtlich verteilt sind, sodass der bestimmte Bedeckungsgrad repräsentativ für die Teilfläche und/oder die Probenstelle ist. Dies erhöht die Verlässlichkeit der Verfahren. Bevorzugt bilden die Vielzahl von Messpositionen dazu ein gleichförmiges Raster. Bevorzugt ist in dem gleichförmigen Raster jede nicht randständige Messposition von den direkt umliegenden Messpositionen in 4 orthogonal zueinanderstehenden Richtungen gleich beabstandet.

In einer weiteren bevorzugten Ausgestaltung umfasst die massenspektrometrische Probe im Probenbereitstellungsschritt A,B des Weiteren einen potenziell Zytotoxizitätsfaktor-neutralisierenden Faktor. Dadurch kann nicht nur die Wirkung von zytotoxischen Faktoren oder analytischen Proben auf die tierischen Zellen bestimmt werden, sondern auch eine neutralisierende Wirkung eines weiteren Faktors auf den zytotoxischen Faktor oder die analytische Probe bestimmt werden. Der Zytotoxizitätsfaktor-neutralisierende Faktor kann aus folgender, nicht abschließenden Gruppe ausgewählt sein: Antibiotika, antimikrobieller Wirkstoff, antibakterieller Wirkstoff, antifungaler Wirkstoff, antiviraler Wirkstoff, antiparasitärer, Wirkstoff, pharmazeutischer Wirkstoff, Anti-Virulenz-Wirkstoff, Antikörper. Weitere Zytotoxizitätsfaktor-neutralisierende Faktoren ergeben sich je nach Anwendung. Insbesondere können Zytotoxizitätsfaktor-neutralisierende Faktoren im Verfahren eingesetzt werden, um die Wirksamkeit des Zytotoxizitätsfaktor-neutralisierenden Faktors gegen zytotoxische Faktoren wie zum Beispiel Bakterien, Pilze, Parasiten oder Viren zu testen.

Um den Proliferationsgrad und/oder die zytotoxische Wirkung abzuleiten, ist es weiterhin vorteilhaft, dass, zusätzlich zur massenspektrometrischen Probe umfassend die analytische Probe, mindestens eine Referenzprobe das Verfahren durchläuft. Dabei wird die Referenzprobe auf einer anderen Probenstelle mit dem Verfahren vermessen und ausgewertet - analog der analytischen Probe. Die massenspektrometrische Probe umfaßt entweder eine analytische Probe oder ist eine Referenzprobe. Bevorzugt durchlaufen die Referenzprobe und die massenspektrometrische Probe umfassend die analytische Probe das Verfahren gleichzeitig auf dem gleichen massenspektrometrischen Probenträger und/oder in direkter Sequenz auf verschiedenen massenspektrometrischen Probenträgern. Bevorzugt ist die Referenzprobe frei von zytotoxischen Faktoren und weist die gleichen tierische Zellen und das gleiche Nährmedium auf, die in der massenspektrometrischen Probe mit der analytischen Probe verwendeten wurden. Bevorzugt sind Referenzproben Proben mit bekanntem Auswerteergebnis, umfassend: Bedeckungsgrad, Zell-Ausdehnungsweite, Proliferationsfähigkeit, Migrationsfähigkeit und/oder zytotoxische Wirkung. Davon zu unterscheiden sind analytische Proben, welche zu untersuchende Proben mit unbekanntem Auswertergebnis betreffen. Bevorzugt werden mehrere Referenzproben auf jedem massenspektrometrischen Probenträger mitanalysiert. Des Weiteren wird bevorzugt nach der Bestimmung des Bedeckungsgrades im zweiten Auswerteschritt A,C in einem Auswertezwischenschritt A ein Vergleich des Bedeckungsgrades der Referenzprobe mit dem Bedeckungsgrad der massenspektrometrischen Probe umfassend die analytische Probe durchgeführt. Weiterhin bevorzugt wird nach der Bestimmung der Zell-Ausdehnungsweite im zweiten Auswerteschritt B in einem Auswertezwischenschritt B ein Vergleich der Zell-Ausdehnungsweite der Referenzprobe mit der Zell-Ausdehnungsweite der massenspektrometrischen Probe umfassend die analytische Probe durchgeführt.

Dabei umfasst die Referenzprobe bevorzugt nicht den mindestens einen potenziell zytotoxischen Faktor, und/oder den mindestens einen zytotoxischen Faktor und/oder den mindestens einen potenziell Zytotoxizitätsfaktor-neutralisierenden Faktor. Bevorzugt weist die Referenzprobe die gleichen tierischen Zellen und das gleiche Nährmedium auf, die in der massenspektrometrischen Probe mit der analytischen Probe verwendeten wurden. Dieser Vergleich wird dann bevorzugt in die Ableitung der Proliferationsfähigkeit im dritten Auswerteschritt A,B und/oder in die Ableitung der zytotoxischen Wirkung im vierten (bevorzugt letzten) Auswerteschritt einbezogen. Hierdurch wird sichergestellt, dass der Bedeckungsgrad, die Zell-Ausdehnungsweite und/oder der Proliferationsgrad einer entsprechenden zytotoxischen Wirkung richtig zugewiesen wird. Auch ist so eine Vergleichbarkeit zwischen mehreren Verfahrensdurchläufen gegeben.

In einer weiteren bevorzugten Ausgestaltung wird die massenspektrometrische Probe im Probenbereitstellungsschritt A,B bereitgestellt, indem die tierischen Zellen vor Auftragung des Nährmediums und der analytischen Probe bereits auf den Probenstellen vorliegen. So können die Zellen zum Beispiel im gefrorenen oder gefriergetrockneten Zustand auf der Probenstelle vorgelegt sein. Die massenspektrometrischen Probenträger können zum Beispiel vom Hersteller bereits in dieser Form ausgeliefert werden. Dies hat den Vorteil, dass sich der Nutzer die Auftragung der tierischen Zellen spart. Besonders bevorzugt liegen die tierischen Zellen vor Auftragung des Nährmediums und der analytischen Probe bereits adhäriert auf den Probenstel,len vor. Dies verkürzt die Dauer der Kultivierung im Kultivierungsschritt. Bevorzugt ist hierbei lediglich ein Teil der Probenstelle mit bereits adhärierten tierischen Zellen im Probenbereitstellungsschritt A bedeckt, sodass eine Proliferation im Kultivierungsschritt im Anschluss noch detektiert werden kann. Das heißt, dass die bereits adhäriert vorliegenden tierischen Zellen vor Auftragung des Mediums und der analytischen Probe die Probenstelle bevorzugt unvollständig bedecken. Vorteilhaft ist ein Bedeckungsgrad im Probenbereitstellungsschritt A zwischen 10 % und 90 % der Fläche der Probenstelle. Weiterhin bevorzugt ist ein Bedeckungsgrad im Probenbereitstellungsschritt A zwischen 25 % und 75% der Fläche der Probenstelle. Besonders bevorzugt ist ein Bedeckungsgrad im Probenbereitstellungsschritt A zwischen 30 % und 50 % der Fläche der Probenstelle. Bevorzugt wird der massenspektrometrische Probenträger bereits mit adhärierten tierischen Zellen auf den Probenstellen ausgeliefert. Bevorzugt ist, dass zellfreie Positionen in ausreichender Zahl vorhanden sind, um Änderungen der Oberflächenbedeckung der Probenstelle mit tierischen Zellen feststellen zu können.

In einer alternativen bevorzugten Ausgestaltung werden die tierischen Zellen durch Auftragung der tierischen Zellen auf die Probenstelle in suspendierter Form im Probenbereitstellungsschritt A,B bereitgestellt. Dies hat den Vorteil, dass die Zellen häufig besser wachsen und der Typ der tierischen Zellen selbst gewählt werden kann. Bei bestimmten Anwendungen können so auch Gewebezellen von bestimmten Menschen oder Patienten als tierische Zellen im Probenbereitstellungsschritt A,B verwendet werden. So kann zum Beispiel die Wirkung von chemotherapeutischen Wirkstoffen auf Tumorzellen und gesunde Gewebezellen vom gleichen Menschen direkt getestet werden.

Weiterhin ist bevorzugt, dass bei der Auftragung der tierischen Zellen in suspendierter Form nach dem Flüssigkeitsentfernungsschritt ein Waschschritt durchgeführt wird, indem die tierischen Zellen auf dem Probenträger gewaschen und restliche Waschflüssigkeiten entfernt werden. Dies stellt sicher, dass lediglich adhärierte Zellen im Messschritt A,B,C detektiert werden. Alternativ kann der Flüssigkeitsentfernungsschritt durch den Waschschritt ersetzt werden, um das Verfahren zu verkürzen. Das Waschen der tierischen Zellen erfolgt üblicherweise mit Pufferlösung als Waschflüssigkeit. Dabei wird zum Beispiel die Waschflüssigkeit aufgetragen und wieder entfernt. Die Menge der Waschflüssigkeit ist bevorzugt so gewählt, sodass die Probenstelle vollständig bedeckt ist oder der gesamte Probenträger benetzt ist. Die Entfernung der Waschflüssigkeit und/oder Restflüssigkeit kann zum Beispiel durch Trocknen und/oder Absaugen/Aufsagen, zum Beispiel durch Kontakt mit einem saugfähigen Medium, erfolgen.

In einer weiteren alternativen bevorzugten Ausgestaltung wird die massenspektrometrische Probe im Probenbereitstellungsschritt A,B bereitgestellt, indem die tierischen Zellen vor Auftragung des Nährmediums und der analytischen Probe bereits auf den Probenstellen zum Beispiel nicht-adhäriert vorliegen. So können die Zellen zum Beispiel im gefrorenen oder gefriergetrockneten Zustand auf der Probenstelle vorgelegt sein. Die massenspektrometrischen Probenträger können zum Beispiel vom Hersteller bereits in dieser Form ausgeliefert werden. Dies erspart dem Endnutzer die eigene Bereitstellung oder Zugabe der tierischen Zellen. Nach Auftragung der restlichen massenspektrometrischen Probe (unter anderem das Nähmedium und die analytische Probe) wird bevorzugt wie bei der oben beschriebenen Auftragung der tierischen Zellen in suspendierter Form verfahren.

Gemäß einer vorteilhaften Ausgestaltung der Erfindung wird die analytische Probe bereitgestellt, indem eine Ursprungsprobe bereitgestellt wird. Dies erspart den Aufwand der Ursprungsprobenvorbereitung. Gemäß einer weiteren vorteilhaften Ausgestaltung der Erfindung wird die analytische Probe bereitgestellt, indem Bestandteile der Ursprungsprobe, zum Beispiel lösliche Bestandteile, bereitgestellt werden. Durch eine erste Aufreinigung oder erste Auftrennung der Ursprungsprobe können insbesondere Bestandteile der Ursprungsprobe entfernt werden, die die Kultivierung und/oder den Messschritt A,B,C negativ beeinflussen würden. Zum Beispiel wäre im Falle von Stuhlproben die Entfernung von größeren unlöslichen Fragmenten wie unverdauten Essensresten relevant. Des Weiteren wird gegenüber der vollständigen Isolierung des zytotoxischen Faktors das Risiko reduziert, dass im Rahmen der Isolierung der zytotoxische Faktor abgereichert oder verändert wird. Gemäß einer alternativen vorteilhaften Ausgestaltung der Erfindung wird die Ursprungsprobe bereitgestellt, indem der zytotoxische Faktor der Ursprungsprobe in isolierter und/oder aufgereinigter Form bereitgestellt wird. Der zytotoxische Faktor kann , dabei auch aus der Ursprungsprobe isoliert worden sein. Eine Isolierung ist zwar aufwendiger, stellt jedoch eine störungsfreie Durchführung des Verfahrens sicher. Bevorzugt ist die Ursprungsprobe eine biologische Ursprungsprobe. Des Weiteren wird die Ursprungsprobe bevorzugt aus folgender Gruppe ausgewählt: Probe eines Menschen, Probe eines Tieres, Probe einer Pflanze oder Umweltprobe. Die Probe eines Menschen oder eines Tieres kann zum Beispiel eine Stuhlprobe, Urinprobe, Blutprobe, Vollblut, Serumprobe, Plasmaprobe, Liquorprobe, bronchoalveoläre Lavage (BAL)-Probe, Wundsekretprobe, sonstige Sekretproben und Exkretproben, ein Abstrich einer Wunde oder einer Schleimhaut, eine Gewebeprobe, eine Muskelprobe, eine Gehirnprobe, eine Tumorprobe, eine Organprobe oder eine Hautprobe, eine Weichteilprobe, eine Sputumprobe, eine Ausscheidungsprobe, oder eine Punktatprobe sein.

Die Probe einer Pflanze kann zum Beispiel eine Probe einer potenziell giftigen Pflanze sein.

Die Umweltprobe kann eine zum Beispiel eine Wasser- oder Abwasserprobe, Bodenprobe, Luftprobe, Oberflächenprobe, oder eine Probe eines Lebensmittels, wie zum Beispiel Fleisch, Milch, Joghurt oder jedes andere Nahrungsmittel, sein.

In einer weiteren vorteilhaften Ausgestaltung sind die tierischen Zellen Wirbeltierzellen, Säugetierzellen und/oder humane Zellen. So kann die Art der tierischen Zellen entsprechend der Anwendung ausgewählt werden. Das erhaltene Massenspektrum und die zellspezifischen massenspektrometrischen Signaturen sind in der Regel jeweils abhängig vom Typ der verwendeten tierischen Zelle.

Humane Zellen werden insbesondere dann als tierische Zellen verwendet, wenn die Wirkung eines potentiell zytotoxischen Faktors auf Menschen überprüft werden soll. Besonders bevorzugt werden humane Zellen eines jeweiligen Menschen werden, wenn eine zytotoxische Wirkung spezifisch für diese Person untersucht werden soll. So kann beispielsweise die zytotoxische Wirkung von Chemotherapeutika auf Tumorzellen und gesunde Gewebezellen eines Menschen getestet werden, um die Wirkung des Chemotherapeutikums vor Verabreichung zu prüfen. Die tierischen Zellen können auch Nervenzellen, insbesondere humane Nervenzellen, sein. Dadurch lässt sich zum Beispiel besonders vorteilhaft die Anwesenheit von Neurotoxinen als zytotoxische Faktoren überprüfen. Die Nutzung von Wirbeltierzellen, so z.B. Amphibienzellen oder Fischzellen, ermöglicht zum Beispiel eine sensitive Überprüfung von Umweltgiften.

Weiterhin ist es vorteilhaft, wenn die tierischen Zellen kontinuierliche Zelllinien, insbesondere humane kontinuierliche Zelllinien, sind. Kontinuierliche Zelllinien sind besonders leicht kultivierbar. Die meisten zurzeit verfügbaren Zellkulturlinien sind Säugetierzelllinien. Als Beispiele sind hier MDCK, HeLa, HECK, PERC und CHO Zelllinien zu nennen. Sie eignen sich durch Ihre einfache Kultivierbarkeit beispielsweise für den Einsatz in breiten Screeningverfahren, wie zum Beispiel zur Suche nach neuen pharmakologischen Wirkstoffen. In einer weiteren bevorzugten Ausgestaltung stammen die tierischen Zellen aus entnommenen Gewebeproben eines Menschen oder Tieres. Weiterhin bevorzugt stammen die tierischen Zellen aus entnommenen Tumorproben eines Menschen oder Tieres. Dadurch kann auf einfache Weise vor Gabe von Medikamenten an diesen Menschen oder das Tier die zytotoxische Wirkung dieser Medikamente auf diesen Menschen oder dieses Tier festgestellt werden.

Gemäß einer vorteilhaften Ausgestaltung der Erfindung stammt der mindestens eine zytotoxische Faktor aus folgender Gruppe: chemisches Element einschließlich dessen Isotope und Ionen, wie zum Beispiel bestimmte Metalle, niedermolekulare chemische Verbindung einschließlich dessen Ionen, hochmolekulare chemische Verbindung, pharmazeutischer Wirkstoff, Toxin, Archaea, Bakterium, Virus, Pilz, Protozoe, Alge, Parasit, Bestandteile oder sekretierte Substanzen von Mikroorganismen und anderen übertragbaren biologischen Agentien, zytotoxische Substanzen biologischen Ursprungs, zytotoxische Substanzen mikrobiellen Ursprungs aus einer humanen Probe, Chemotherapeutikum, antitumoraler Wirkstoff, Protein, Peptid, Antikörper, antimikrobielles Mittel, antivirales Mittel, antifungales Mittel, antiparasitäres Mittel oder antibakterielles Mittel oder Biozid. Die Mikroorganismen umfassen hier insbesondere Archaea, Bakterien, Viren, einzellige Pilze, einzellige Algen, Protozoen und/oder andere einzellige Parasiten. Es sei hier unmissverständlich klargestellt, dass die oben genannten Mitglieder der Gruppe an zytotoxischen Faktoren den jeweiligen Typ des zytotoxischen Faktors repräsentieren, nicht jedoch die Menge oder Vielfältigkeit beschränken. Es handelt es sich bei den jeweiligen zytotoxischen Faktoren bevorzugt ebenfalls um eine Vielzahl Ihrer Einheit. So handelt es sich beispielsweise beim zytotoxischen Faktor Bakterium um eine Vielzahl an bakteriellen Zellen. Des Weiteren können die bakteriellen Zellen einem oder mehreren Bakterienstämme angehören. Des Weiteren sind zum Beispiel auch umfasst eine Vielzahl an Archaeazellen (Archaea), viralen Partikeln (Virus), Pilzzellen (Pilz), Protozoenzellen, Algenzellen, parasitären Organismen (Parasit), Proteinmolekülen (Protein), und so weiter. Sämtliche aufgezählten Organismen (Mikroorganismen, Parasiten) können tot oder lebendig sein. Des Weiteren sind ebenfalls Bestandteile und/oder sekretierte Substanzen von sämtlichen aufgezählten Organismen und Viren umfasst. Parasiten sind besonders bevorzugt einzellige Parasiten wie zum Beispiel Protozoen. Weiterhin bevorzugt umfassen die Parasiten ebenfalls Bestandteile und/oder sekretierte Bestandteile mehrzelliger Parasiten, wie zum Beispiel parasitärer Würmer. Der Gruppe der zytotoxischen Faktoren erstreckt sich somit bevorzugt von chemischen Verbindungen über Viren und Bestandteile von Organismen bis hin zu Mikroorganismen oder anderen übertragbaren biologischen Agentien. Die breite Anwendbarkeit ist ein besonderer Vorzug des beanspruchten Verfahrens. Dabei können auch verschiedene zytotoxische Faktoren aus dieser Gruppe in Kombination in der massenspektrometrischen Probe enthalten sein.

Besonders bevorzugt umfasst die analytische Probe als zytotoxischen Faktor bakterielle Zellen, zum Beispiel eines Bakterienstamms oder mehrerer Bakterienstämme, oder deren Bestandteile und als Zytotoxizitätsfaktor-neutralisierenden Faktor ein antibakterielles Mittel, dass gegen bakterielle Zellen oder deren Bestandteile gerichtet ist und potentiell eine antibakterielle Wirkung auf bakterielle Zellen oder deren Bestandteile aufweist. Somit kann die Wirkung eines antibakteriellen Mittels auf bakterielle Zellen oder deren Bestandteile auf einfache Weise überprüft werden. Wenn die zytotoxische Wirkung der bakteriellen Zellen oder deren Bestandteile zuvor ermittelt worden oder bekannt ist, deutet eine unbeeinflusste Proliferationsfähigkeit der tierischen Zellen bei Anwesenheit der bakteriellen Zellen oder deren Bestandteile und des antibakteriellen Mittels auf eine antibakterielle Wirkung des antibakteriellen Mittels gegen diese bakteriellen Zellen .(Bakterienstamm/ Bakterienstämme) hin. Hingegen deutet eine reduzierte Proliferationsfähigkeit der tierischen Zellen bei Anwesenheit der bakteriellen Zellen oder deren Bestandteile und des antibakteriellen Mittels auf eine (Teil-)Resistenz der bakteriellen Zellen (Bakterienstamms/ Bakterienstämme) gegen das antibakterielle Mittel hin. Dazu muss die Wirkungsweise oder Struktur des antibakteriellen Mittels nicht bekannt sein, da hier lediglich die Zytotoxizitätsfaktor-neutralisierende Wirkung nachgewiesen wird, was einen Vorteil beim Screening nach neuen antibakteriellen Mitteln darstellt. Gleichzeitig kann die zytotoxische Wirkung des antibakteriellen Mittels auf die tierischen Zellen ermittelt werden. Dies hat den Vorteil, dass mit dem gleichen Verfahren antibakterielle Mittel ausgewählt werden können, die spezifisch auf die jeweiligen Bakterien wirken. Das Risiko einer schädlichen Wirkung auf einen Menschen kann weiter reduziert werden, wenn die tierischen Zellen humane Zellen sind. Bestandteile von bakteriellen Zellen umfassen ebenfalls von den bakteriellen Zellen sekretierte Substanzen.

Des Weiteren muss nicht bekannt sein, welchen zytotoxischen Faktor eine analytische Probe enthält. So können mit diesen Verfahren verschiedene Zytotoxizitätsfaktor-neutralisierende Faktoren mit Hilfe der analytischen Probe gefahrlos getestet werden - anstelle am lebenden Menschen oder Tier.

In einer weiteren bevorzugten Ausgestaltungsform umfasst die analytische Probe als zytotoxischen Faktor Viruspartikel, zum Beispiel eines Virusstamms oder mehrerer Virusstämme, oder deren Bestandteile und als Zytotoxizitätsfaktor-neutralisierenden Faktor ein antivirales Mittel, dass gegen die Viruspartikel oder deren Bestandteile gerichtet ist. Somit kann die Wirkung eines antiviralen Mittels auf die Viruspartikel oder deren Bestandteile auf einfache Weise überprüft werden. Wenn die zytotoxische Wirkung der Viruspartikel oder deren Bestandteile zuvor ermittelt worden oder bekannt ist, deutet eine unbeeinflusste Proliferationsfähigkeit der tierischen Zellen bei Anwesenheit von Viruspartikeln oder deren Bestandteilen und des antiviralen Mittels auf eine antivirale Wirkung des antiviralen Mittels gegen das Virus (Virusstamm/ Virusstämme) hin. Hingegen deutet eine reduzierte Proliferationsfähigkeit der tierischen Zellen bei Anwesenheit der Viruspartikel oder deren Bestandteile und des antiviralen Mittels auf eine (Teil-)Resistenz des Virus (Virusstamms/ Virusstämme) gegen das antivirale Mittel hin. Dazu muss die Wirkungsweise oder Struktur des antiviralen Mittels nicht bekannt sein, da hier lediglich die Wirkung nachgewiesen wird, was einen großen Vorteil beim Screening nach neuen antiviralen Mitteln darstellt. Gleichzeitig kann die zytotoxische Wirkung des antiviralen Mittels auf die tierischen Zellen ermittelt werden. Dies hat den Vorteil, dass lediglich antivirale Mittel ausgewählt werden können, die spezifisch auf die jeweiligen Viren (Virusstamm/ Virusstämme) wirken. Das Risiko einer schädlichen Wirkung auf einen Menschen kann weiter reduziert werden, wenn die tierischen Zellen humane Zellen sind.

In einer alternativen vorteilhaften Ausgestaltung umfasst die analytische Probe als zytotoxischen Faktor fungale Zellen (umfassend Pilzstamm/ Pilzstämme) oder deren Bestandteile und als Zytotoxizitätsfaktor-neutralisierenden Faktor ein antifungales Mittel, dass gegen die fungalen Zellen oder deren Bestandteile gerichtet ist. Somit kann auch die Wirkung eines antifungalen Mittels auf die fungalen Zellen oder deren Bestandteile auf einfache Weise überprüft werden. Wenn die zytotoxische Wirkung der fungalen Zellen oder deren Bestandteile zuvor ermittelt worden oder bekannt ist, deutet eine unbeeinflusste Proliferationsfähigkeit der tierischen Zellen bei Anwesenheit der fungalen Zellen oder deren Bestandteile und des antifungalen Mittels auf eine antifungale Wirkung des antifungalen Mittels gegen die fungalen Zellen oder deren Bestandteile hin. Hingegen deutet eine reduzierte Proliferationsfähigkeit der tierischen Zellen bei Anwesenheit die fungalen Zellen oder deren Bestandteile und des antifungalen Mittels auf eine (Teil-)Resistenz des Pilzes gegen das antifungale Mittel hin. Dazu muss die Wirkungsweise oder Struktur des antifungalen Mittels nicht bekannt sein, da hier lediglich die Wirkung nachgewiesen wird, was einen großen Vorteil beim Screening nach neuen antiviralen Mitteln darstellt. Gleichzeitig kann die zytotoxische Wirkung des antifungalen Mittels auf die tierischen Zellen ermittelt werden. Dies hat den Vorteil, dass lediglich antifungale Mittel ausgewählt werden können, die spezifisch auf den jeweiligen Pilz wirken. Das Risiko einer schädlichen Wirkung auf einen Menschen kann weiter reduziert werden, wenn die tierischen Zellen humane Zellen sind. Bestandteile von fungalen Zellen umfassen ebenfalls von den fungalen Zellen sekretierte Substanzen.

In einer alternativen vorteilhaften Ausgestaltung umfasst die analytische Probe als zytotoxischen Faktor mindestens einen Parasiten oder dessen Bestandteile und als Zytotoxizitätsfaktor-neutralisierenden Faktor ein antiparasitäres Mittel, dass gegen den Parasiten oder dessen Bestandteile gerichtet ist. Somit kann auch die Wirkung eines antiparasitären Mittels auf den Parasiten oder dessen Bestandteile auf einfache Weise überprüft werden. Wenn die zytotoxische Wirkung des Parasiten oder dessen Bestandteile zuvor ermittelt worden oder bekannt ist, deutet eine unbeeinflusste Proliferationsfähigkeit der tierischen Zellen bei Anwesenheit des Parasiten oder dessen Bestandteile und des antiparasitären Mittels auf eine antiparasitäre Wirkung des antiparasitären Mittels gegen den Parasiten hin. Hingegen deutet eine reduzierte Proliferationsfähigkeit der tierischen Zellen bei Anwesenheit des Parasiten oder dessen Bestandteile und des antifungalen Mittels auf eine (Teil-)Resistenz des Parasiten gegen das antiparasitäre Mittel hin. Dazu muss die Wirkungsweise oder Struktur des antiparasitären Mittels nicht bekannt sein, da hier lediglich die Wirkung nachgewiesen wird, was einen großen Vorteil beim Screening nach neuen antiparasitären Mitteln darstellt. Gleichzeitig kann die zytotoxische Wirkung des antiparasitären Mittels auf die tierischen Zellen ermittelt werden. Dies hat den Vorteil, dass lediglich antiparasitäre Mittel ausgewählt werden können, die spezifisch auf den jeweiligen Parasiten wirken. Das Risiko einer schädlichen Wirkung auf einen Menschen kann weiter reduziert werden, wenn die tierischen Zellen humane Zellen sind. Bestandteile von Parasiten umfassen ebenfalls von den Parasiten sekretierte Substanzen.

Um eine konzentrationsabhängige zytotoxische Wirkung der analytischen Probe zu bestimmen, ist es weiterhin vorteilhaft, dass jeweils unterschiedliche Konzentrationen der analytischen Probe auf verschiedene Probenstellen bereitgestellt werden. Die konzentrationsabhängige zytotoxische Wirkung ist bevorzugt eine Funktion der bestimmten Bedeckungsgrade (Anspruch 1, 2) oder der bestimmten Zell-Ausdehnungsweiten (Anspruch 3) der verschiedenen Probenstellen und der jeweils unterschiedlichen Konzentrationen der analytischen Probe. Dadurch sind auf einfache Weise die Konzentrationen der analytischen Probe ermittelbar, bei denen eine Zelltoxizität eintritt. Dies kann zum Beispiel bei der Untersuchung von pharmazeutischen Wirkstoffen wie Antibiotika oder Chemotherapeutika zur ersten Einschätzung eines anwendbaren Konzentrationsbereichs dienen.

Zusätzlich ist bevorzugt, dass das ortsauflösende Massenspektrometer und/oder das Massenspektrometer aus folgender Gruppe ausgewählt ist: Laser-Desorptions-Ionisation - MS (LDI-MS), Matrix-unterstützte Laser-Desorption/lonisation - MS (MALDI-MS), Desorptions-Elektrospray-Ionisation - MS (DESI-MS), Matrix-unterstützte Laser-Desorptions Elektrospray Ionisation - MS (MALDESI-MS), Sekundärionen - MS (SIMS), Sekundärionen-Massenspektrometrie-Imaging - MS (SIMS-Imaging-MS), Matrix-unterstützte Laser-Desorption/lonisation - Flugzeitanalysator - MS (MALDI-ToF-MS), MALDI-Tandem-FlugzeitMassenanalysator- MS (MALDI-ToF-ToF-MS), MALDI Massenspektrometrie Imaging (MALDI-MSI) oder ortsauflösendes MS mit einer Mikro-Fluid-Probenionisierungsvorrichtung. Die ortsauflösende Massenspektrometer sind weiter unterteilbar in matrix-basierte und nicht-matrix-basierte ortsauflösende Massenspektrometer. Bei Matrix-basierten ortsauflösenden Massenspektrometern erfolgt die Zugabe einer Matrix, bevorzugt flüssig, zur massenspektrometrischen Probe. Dadurch können die Analyten der massenspektrometrischen Probe beim Laserbeschuss ionisiert werden. Die erzeugten Probenionen werden dann vermessen. Bei Nicht-matrix-basierten ortsauflösenden Massenspektrometern erfolgt hingegen keine Zugabe von Matrix. Eine Matrix ist hier nicht erforderlich, um die Analyten der massenspektrometrischen Probe zu ionisieren und Probenionen aus der massenspektrometrischen Probe zu erzeugen.

Zu den matrix-basierten ortsauflösenden Massenspektrometern zählen beispielsweise bevorzugt folgende MS: MALDI-MS, MALDESI-MS, MALDI-ToF-MS, MALDI-ToF-ToF-MS, MALDI-MSI. Zu den nicht-matrix-basierten ortsauflösenden Massenspektrometern zählen beispielsweise bevorzugt folgende ortsauflösende Massenspektrometer: LDI-MS, DESI-MS, SIMS, SIMS-Imaging und das ortsauflösendes Massenspektrometer mit einer Mikro-Fluid-Probenionisierungsvorrichtung.

Des Weiteren sind unter anderem sämtliche Kombinationen mit lonenmobilitätsanalysatoren mit in den bevorzugten Ausführungsformen umfasst.

In einer weiteren bevorzugten Ausgestaltungsform erfolgt die ortstreue Auftragung der Matrix mittels eines Sprühverfahrens, Sublimationsverfahren oder sequenzieller gerichteter Positionierung einer Vielzahl von Matrixmikrotröpfchen oder Matrixnanotröpfchen auf der Probenstelle. Während die Auftragung der Matrix mittels eines Sprühverfahrens und Sublimationsverfahren weitgehend ungezielt erfolgt, erfolgt die Auftragung mittels sequenzieller gerichteter Positionierung in der Regel gezielt. Auf Grund der Einfachheit und der Geschwindigkeit des Verfahrens erfolgt die Auftragung der Matrix besonders bevorzugt mit einem Sprühverfahren. Dabei erfolgt die Auftragung der Matrix durch Besprühen der Probenstellen mit Matrix oder des massenspektrometrischen Probenträgers mittels einer Matrix-Sprühvorrichtung. Die Matrix wird dabei bevorzugt mikrodispers aufgetragen. Die Matrix-Sprühvorrichtung erzeugt kleinste Matrixtröpfchen: Matrix-Mikrotröpfchen und/oder Matrix-Nanotröpfchen. Bevorzugt haben die aufgetragenen Matrixtröpfchen einen Durchmesser von 0.01 bis 100 µm. Weiterhin bevorzugt haben die aufgetragenen Matrixtröpfchen einen Durchmesser von 0.05 bis 40 µm. Besonders bevorzugt haben die aufgetragenen Matrixtröpfchen einen Durchmesser von 0.1 bis 10 µm. Weitere Verfahren und Details zur ortstreuen Auftragung der Matrix sind dem Fachmann im Rahmen der Probenpräparation zur Imaging Massenspektrometrie bekannt.

Die Aufgabe wird weiterhin gelöst mit einem weiteren Verfahren zur Bestimmung der zytotoxischen Wirkung einer analytischen Probe auf tierische Zellen, umfassend folgende Schritte:
- einen Probenbereitstellungsschritt A, in dem eine massenspektrometrischen Probe, umfassend tierische Zellen, Nährmedium und die analytische Probe, welche potenziell einen zytotoxischen Faktor aufweist, auf mindestens einer Probenstelle eines massenspektrometrischen Probenträgers bereitgestellt wird;
- einen Kultivierungsschritt, in dem die massenspektrometrische Probe auf der Probenstelle des massenspektrometrischen Probenträgers in einer Kultivierungsvorrichtung inkubiert wird;
- einen Flüssigkeitsentfernungsschritt, in dem Restflüssigkeit der massenspektrometrischen Probe von der Probenstelle entfernt wird;
- einen Messschritt C, in dem Massenspektren an einer Vielzahl von Messpositionen verteilt über mindestens eine Teilfläche der Probenstelle mittels eines Massenspektrometers aufgenommen werden, wobei bei Verwendung von einem matrix-basierten Massenspektrometer vor der Aufnahme der Massenspektren im Messschritt C in einem vorgelagerten Probenpräparationsschritt A eine Präparation der Probenstelle durch ortstreue Auftragung einer Matrix auf mindestens die eine Teilfläche der Probenstelle erfolgt;
- einen ersten Auswerteschritt C, in dem jedes Massenspektrum hinsichtlich des Vorhandenseins einer zellspezifischen massenspektrometrischen Signatur für die tierischen Zellen analysiert wird;
- einen zweiten Auswerteschritt C, in dem ein Bedeckungsgrad durch tierische Zellen für mindestens die Teilfläche der Probenstelle aus dem Ergebnis des ersten Auswerteschrittes C bestimmt wird;
- einen vierten Auswerteschritt C, in dem die zytotoxische Wirkung der analytischen Probe auf die tierischen Zellen aus dem bestimmten Bedeckungsgrad des zweiten Auswerteschrittes C abgeleitet wird.

Es hat sich gezeigt, dass die Bestimmung des Bedeckungsgrades und der daraus ableitbaren zytotoxischen Wirkung einer Probe auch mittels eines Verfahrens möglich ist, dass kein ortsauflösendes Massenspektrometer verwendet. Bei diesem Verfahren sind keine genauen Ortskoordinaten der Messpositionen erforderlich. Somit sind die erzeugten Massenspektren nicht ortsaufgelöst. Jedoch werden auch in diesem Verfahren die Einzelmassenspektren der Messpositionen einer Probenstelle nicht zu einem Summenmassenspektrum verrechnet. Die Messpositionen sind verteilt über mindestens eine Teilfläche der Probenstelle. Die Messpositionen sind dabei weitgehend zufällig verteilt. Bevorzugt sind sie dabei weitgehend gleichmäßig verteilt. Durch eine entsprechend hohe Anzahl an Messpositionen lässt sich dadurch der Bedeckungsgrad ermitteln. Bevorzugt ist dazu der Durchmesser des Laserstrahls/Desorptionsstrahls und/oder der Proebenstelle bekannt. Der Bedeckungsgrad stellt in diesem Verfahren einen statistischen Durchschnittswert da. Die Menge an zu vermessenden Messpositionen ist bevorzugt derart hoch, sodass ein repräsentativer Bedeckungsgrad ermittelt werden kann, welcher weitgehend reproduzierbar ist und eine ausreichende Genauigkeit aufweist, zum Beispiel in Form einer statistisch akzeptablen Standardabweichung. Dieses Verfahren hat den Vorteil, dass es kein ortsauflösendes Massenspektrometer benötigt und es besonders kostengünstig ist. Es genügt ein Massenspektrometer, dass verschiedene zahlreiche Messpositionen auf einer Probenstelle vermessen kann. Dabei ist das präzise Einstellen der Messposition, zum Beispiel mittels eines präzisen X/Y-Tisches und/oder der präzisen Einstellung von Spiegeln nicht erforderlich. Ein ungefähres Anfahren jeder Messposition ist ausreichend, solange sich ein Großteil der zahlreichen Messpositionen einer Probenstelle unterscheidet. Nachteilig gegenüber den anderen Verfahren ist jedoch eine in Abhängigkeit der Anzahl der der Messpositionen herabgesetzte Genauigkeit und/oder Reproduzierbarkeit des ermittelten Bedeckungsgrades und eine entsprechende Erhöhung der erforderlichen Zeit pro Probenstelle. Zudem ist der Durchmesser des Lasers oder Desorptionsstrahls bevorzugt geringer als bei den anderen Verfahren sein, da eine höhere Zahl an Messpositionen erforderlich ist. Bevorzugt ist der Durchmesser des Lasers oder Desorptionsstrahls bzw. der Messposition derart gewählt, sodass sich bei der erforderlichen Anzahl an Messpositionen weitgehend rechnerisch keine Überschneidungen der Messpositionen ergeben. Bevorzugt wird jeder Messposition ein Zellpräsenzwert zugewiesen. Weiterhin bevorzugt wird der Bedeckungsgrad durch tierische Zellen für mindestens die Teilfläche der Probenstelle aus den Zellpräsenzwerten der Messpositionen bestimmt. Weiterhin wird in einem dritten Auswerteschritt A eine Proliferationsfähigkeit aus dem bestimmten Bedeckungsgrades abgeleitet. Weiterhin bevorzugt wird die zytotoxische Wirkung der analytischen Probe auf die tierischen Zellen aus dem Bedeckungsgrad und/oder der Proliferationsfähigkeit abgeleitet.

Die Aufgabe wird weiterhin gelöst mit einem Verfahren zur Bestimmung der zytotoxischen Wirkung mindestens einer analytischen Probe auf tierische Zellen umfassend folgende Schritte:
- einen Probenbereitstellungsschritt B, in dem eine massenspektrometrischen Probe umfassend tierische Zellen, Nährmedium und die analytischen Probe, welche potentiell einen zytotoxischen Faktor aufweist, auf mindestens einer Probenstelle eines massenspektrometrischen Probenträgers bereitgestellt wird, wobei die Probestelle eine erste und eine zweite Teilfläche aufweist, wobei die tierischen Zellen auf der ersten Teilfläche der Probenstelle bereitgestellt werden und auf der zweiten Teilfläche der Probenstelle keine tierischen Zellen bereitgestellt werden und wobei die restliche massenspektrometrische Probe auf beiden Teilflächen bereitgestellt wird;
- einen Kultivierungsschritt, in dem die massenspektrometrische Probe auf der Probenstelle des massenspektrometrischen Probenträgers in einer Kultivierungsvorrichtung inkubiert wird;
- einen Flüssigkeitsentfernungsschritt, in dem Restflüssigkeiten der massenspektrometrischen Probe auf der Probenstelle entfernt werden;
- einem Messschritt B, in dem ortsaufgelöste Massenspektren an mindestens zwei Messpositionen der Probenstelle mittels eines ortsauflösendem Massenspektrometers aufgenommen werden, wobei die erste Messposition noch tierische Zellen aufweist und die zweite Messposition keine tierischen Zellen aufweist, wobei sich beide Messpositionen auf einer Erstreckungsrichtung ausgehend von einem Referenzpunkt befinden, wobei bei Verwendung von einem matrix-basierten ortsauflösende Massenspektrometer vor der Aufnahme der ortsaufgelösten Massenspektren im Messschritt B in einem vorgelagerten Probenpräparationsschritt B eine Präparation der Probenstelle durch ortstreue Auftragung einer Matrix auf mindestens einen Teil beider Teilflächen der Probenstelle erfolgt;
- einen ersten Auswerteschritt A, in dem jedes ortsaufgelöste Massenspektrum hinsichtlich des Vorhandenseins einer zellspezifischen massenspektrometrischen Signatur für die tierische Zellen analysiert wird und jeder Messposition ein Zellpräsenzwert zugewiesen wird;
- einen zweiten Auswerteschritt B, in dem eine Zell-Ausdehnungsweite aus mindestens einer Entfernung von einem Referenzpunkt zu einem Umschlagpunkt ermittelt wird, wobei jeder Umschlagpunkt mindestens aus der ersten und der zweiten Messposition berechnet wird;
- einem viertem Auswerteschritt B, in dem die zytotoxische Wirkung der analytischen Probe aus dem Ergebnis des vorangegangenen Auswerteschrittes abgeleitet wird. Dadurch kann die Anwesenheit von zytotoxischen Faktoren in einer analytischen Probe, von denen nicht bekannt ist, ob Sie einen oder mehrere zytotoxische Faktoren enthält, zuverlässig, einfach, schnell und kostengünstig überprüft werden. Zudem kann die Wirkung von potenziell zytotoxischen Faktoren auf tierische Zellen überprüft werden und Ihre Toxizität zuverlässig, einfach, schnell und kostengünstig bestimmt werden.

Es hat sich gezeigt, dass sich zur Bestimmung der zytotoxischen Wirkung von analytischen Proben auch die Bestimmung der Zell-Ausdehnungsweite und/oder die daraus abgeleitete Proliferationsfähigkeit und/oder Migrationsfähigkeit durch Bestimmung der Ausdehnung der tierischen Zellen auf der Probenstelle mittels ortsauflösenden Massenspektrometern verwendbar ist. Bevorzugt wird die Ausdehnung der tierischen Zellen auf der Probenstelle in diesem Verfahren mittels Bestimmung einer Zell-Ausdehnungsweite bestimmt. Nach Anspruch 3 ist das bevorzugte Ergebnis des unmittelbar vorangegangenen Auswertschrittes die Zell-Ausdehnungsweite und der bevorzugte vorangegangene Auswerteschritt der zweite Auswerteschritt B.

Alternativ ist nach Anspruch 3 das Ergebnis des vorangegangenen Auswertschrittes die Proliferationsfähigkeit und/oder die Migrationsfähigkeit und der bevorzugte vorangegangene Auswerteschritt der dritte Auswerteschritt B. Durch die zusätzlichen Verfahrensschritte werden dem unter anderem dem Nutzer weitere Daten zwecks besserer Ergebnisbeurteilung zur Verfügung gestellt.

Maßgebend für das Verfahren nach Anspruch 3 ist, dass die Zell-Ausdehnungsweite der tierischen Zellen durch Wachstum, Zellteilung und/oder Zellmigration unter proliferationsgerechten und/oder zellmigrationsgerechten Bedingungen in Form einer flächigen Ausbreitung vom äußeren Rand der ersten Teilfläche beginnt. Entscheidend dabei ist, dass die zweite Teilfläche der Probenstelle, welche an die erste Teilfläche der Probenstelle angrenzt, zu Beginn des Kultivierungsschrittes frei von tierischen Zellen ist. Die zytotoxische Wirkung wird schließlich über das Ausmaß der Ausdehnung der tierischen Zellen bestimmt. Dabei ist zum Beispiel die radiale Ausdehnung der Bedeckung der zweiten Teilfläche mit tierischen Zellen durch Proliferation und/oder Migration ein Maß für zytotoxische Wirkung einer analytischen Probe oder eines zytologischen Faktors.

Auf einfache Weise kann das Ausmaß der Ausdehnung der tierischen Zellen über eine Bestimmung der Zell-Ausdehnungsweite erreicht werden. Dabei wird die Grenze der Ausbreitungsfläche der tierischen Zellen zu einem Zeitpunkt t ermittelt. Die Bestimmung der Grenze der Ausbreitungsfläche erfolgt dabei über die Bestimmung eines Umschlagpunktes. Die Bestimmung des Umschlagpunktes erfolgt, indem die Anwesenheit oder die Abwesenheit der tierischen Zellen an der Grenze massenspektrometrisch detektiert wird. Die Zell-Ausdehnungsweite wird dann bestimmt, indem die Entfernung von einem Referenzpunkt zum Umschlagpunkt bestimmt wird.

Indem im Probenvorbereitungsschritt B auf einer ersten Teilfläche die tierischen Zellen bereitgestellt werden und auf einer zweiten Teilfläche keine tierischen Zellen bereitgestellt werden, kann die Ausdehnung der tierischen Zellen als zuverlässiges Maß für die Proliferationsfähigkeit, die Migrationsfähigkeit und/oder die zytotoxische Wirkung verwendet werden. Bevorzugt sind die tierischen Zellen adhärent wachsende Zellen. Weiterhin bevorzugt sind die tierischen Zellen vor der Zugabe der restlichen massenspektrometrischen Probe bereits auf der ersten Teilfläche adhäriert. Der Probenbereitstellungsschritt B teilt sich in einen ersten und einen zweiten Teilschritt auf. So erfolgt in einem ersten Teilschritt des Probenbereitstellungsschrittes B eine erste Kultivierung von tierischen Zellen mit Nährmedium auf der ersten Teilfläche der Probenstelle. Eine analytische Probe oder ein zytotoxischer Faktor ist hier nicht anwesend. Hierbei adhärieren die tierischen Zellen an die erste Teilfläche und proliferieren auf der ersten Teilfläche. Die zweite Teilfläche bleibt dabei frei von tierischen Zellen. Dies kann zum Beispiel durch gezielte Auftragung von Nährmedium erfolgen. Nach dem erstem Teilschritt des Probenbereitstellungsschrittes B wird die Restflüssigkeit bevorzugt von der ersten Teilfläche entfernt. Nach dem ersten Teilschritt erfolgt der zweite Teilschritt des Probenbereitstellungsschrittes B. Im zweiten Teilschritt des Probenbereitstellungsschrittes B werden Nährmedium, die analytische Probe und weitere Bestandteile der massenspektrometrischen Probe auf beiden Teilflächen oder die gesamte Probenfläche aufgetragen. Es werden hierbei keine tierischen Zellen aufgetragen.

Im Kultivierungsschritt erfolgt eine flächige Ausbreitung vom äußeren Rand der ersten Teilfläche aus. Diese erfolgt im Kultivierungsschritt bevorzugt weitgehend gleichmäßig. Dabei erfolgt die Ausdehnung bevorzugt in beiden Dimensionen der Fläche. Insbesondere eine kreisflächige Ausgestaltung der ersten Teilfläche mit umliegender zweiten Teilfläche ergibt eine zweidimensionale Ausbreitung. Alternativ können die erste Teilfläche und die zweite Teilfläche derart ausgestaltet sein, dass die Ausdehnung weitgehend eindimensional, d.h. in einer Richtung erfolgt.

Weiterhin bevorzugt wird die Probenstelle nach dem ersten und/oder zweiten Teilschritt des Probenbereitstellungsschritt B und/oder nach dem Kultivierungsschritt mit einer Waschlösung gewaschen, um nicht adhärierte tierische Zellen zu entfernen.

Die erste Teilfläche kann eine kreisförmige Fläche sein, die von der zweiten Teilfläche vollumfänglich in Form eines Ringes umgrenzt ist. Alternativ kann die erste Teilfläche nicht vollumfänglich an die zweite Teilfläche angrenzen. Entscheidend ist, dass beide Teilflächen aneinander angrenzen.

Der Kultivierungsschritt und der Flüssigkeitsentfernungsschritt erfolgen wie zuvor beschrieben.

Durch Aufnahme von ortsaufgelöste Massenspektren an mindestens zwei Messpositionen der Probenstelle im Messschritt B mittels eines ortsauflösendem Massenspektrometers wird die Ausbreitungsgrenze der tierischen Zellen bestimmt. Die Ausbreitungsgrenze wird dabei direkt mit Hilfe des Umschlagpunktes bestimmt. Die erste Messposition weist dabei noch tierische Zellen auf. Die zweite Messposition weist dabei keine tierischen Zellen auf. Somit liegen die erste Messposition und die zweite Messposition auf einer jeweils anderen Seite der Ausbreitungsgrenze der tierischen Zellen. Dabei liegen beide Messpositionen bevorzugt direkt an der Ausbreitungsgrenze. Bevorzugt sind die beiden Messpositionen benachbart. Je näher beide Punkte an der Ausbreitungsgrenze lokalisiert sind, desto präziser ist die Zell-Ausdehnungsweite bestimmbar. Durch eine bevorzugte mehrfache Messung entlang jeweils mindestens einer Ausdehnungsrichtung der tierischen Zellen sind eine erste Messposition und eine zweite Messposition auffindbar, welche die größte Nähe zur Ausbreitungsgrenze aufweisen. Bevorzugt ist die zweite Messposition auf der zweiten Teilfläche lokalisiert.

Die Präparation der Probenstelle durch ortstreue Auftragung einer Matrix auf mindestens eine Teilfläche der Probenstelle bei Verwendung von einem matrix-basierten ortsauflösende Massenspektrometer vor der Aufnahme der ortsaufgelösten Massenspektren im Messschritt B in einem vorgelagerten Probenpräparationsschritt B ist bereits zuvor im Rahmen des Probenpräparationsschrittes A in analoger Weise beschrieben worden, wobei hier im Unterschied die Auftragung der Matrix auf mindestens einen Teil beider Teilflächen der Probenstelle erfolgt. Besonders bevorzugt wird die Matrix auf die gesamte Probenstelle aufgetragen. Alternativ kann eine Auftragung der Matrix auf einen Teil beider Teilflächen der Probenstelle in einer Ausdehnungsrichtung ausgehend vom Referenzpunkt aufgetragen werden. Bei Verwendung von nicht-matrix-basierten ortsauflösenden Massenspektrometern wird im Verfahren der Probenpräparationsschritt B nicht durchgeführt.

Ebenso ist der erste Auswerteschritt A bereits zuvor beschrieben worden.

Im zweiten Auswerteschritt B wird die Zell-Ausdehnungsweite bestimmt. Diese wird mittels der Entfernung von einem Referenzpunkt zu einem Umschlagpunkt ermittelt. Dabei können auch mehrere Entfernungen einer Probenstelle zu einer Zell-Ausdehnungsweite verrechnet werden. So können zum Beispiel Entfernungen entlang verschiedener Ausdehnungsrichtungen dazu verwendet werden. Jeder Umschlagpunkt wird mindestens aus der ersten und der zweiten Messposition mit verschiedenen Zellpräsenzwerten berechnet. Dabei weist die erste Messposition bevorzugt einen Zellpräsenzwert auf, welcher das Vorhandensein einer tierischen Zelle anzeigt. Dabei zeigt die zweite Messposition bevorzugt einen Zellpräsenzwert auf, welcher die Abwesenheit einer tierischen Zelle anzeigt. Weiterhin bevorzugt umfassen eine oder mehrere weitere Messpositionen, in Erstreckungsrichtung vom Referenzpunkt ausgehend vor der ersten Messposition, im wesentlichen Zellpräsenzwerte, welche die Anwesenheit von tierischen Zellen anzeigen. Weiterhin bevorzugt umfassen eine oder mehrere weitere Messpositionen, in Erstreckungsrichtung vom Referenzpunkt ausgehend nach der zweiten Messposition, im wesentlichen Zellpräsenzwerte, welche die Abwesenheit von tierischen Zellen anzeigen. Dadurch lässt sich der Umschlagpunkt präzise ermitteln.

Beim vierten Auswerteschritt B wird bevorzugt aus der Zell-Ausdehnungsweite die zytotoxische Wirkung der analytischen Probe oder des zytotoxischen Faktors auf die tierischen Zellen abgeleitet. Bevorzugt wird dazu die Zell-Ausdehnungsweite mit gespeicherten Referenzwerten oder mit den Werten von Referenzproben abgeglichen. Bevorzugt durchläuft dazu zusätzlich zur massenspektrometrischen Probe umfassend die analytische Probe mindestens eine Referenzprobe das Verfahren. Alternativ können gespeicherte Referenzwerte von Referenzproben verwendet werden. Zu den möglichen verschiedenen Typen von Referenzprobe sei hier unter anderem auf die Erläuterungen zu Referenzproben in der Beschreibung verwiesen.

In einer weiteren bevorzugten Ausgestaltung umfasst das Verfahren nach Anspruch 3 einen dritten Auswerteschritt B, in dem eine Proliferationsfähigkeit und/oder eine Migrationsfähigkeit der tierischen Zellen aus der bestimmten Zell-Ausdehnungsweite des zweiten Auswerteschrittes B abgeleitet wird. Dabei wird die zytotoxische Wirkung der analytischen Probe oder des zytotoxischen Faktors im vierten Auswerteschritt B aus der bestimmten Proliferationsfähigkeit und/oder der Migrationsfähigkeit der tierischen Zellen abgeleitet.

Somit wird die zytotoxische Wirkung entweder unmittelbar aus der Zell-Ausdehnungsweite oder mittelbar aus der Zell-Ausdehnungsweite über die Proliferationsfähigkeit und/oder Migrationsfähigkeit abgeleitet. Durch die unmittelbare Ableitung wird das Verfahren vereinfacht. Es können bei der unmittelbaren Ableitung zum Beispiel auch mehrere Zell-Ausdehnungsweiten einer Probenstelle oder mehrerer Probenstellen miteinander verrechnet werden. Insbesondere kann eine Durchschnittswertbildung erfolgen. Durch die mittelbare Ableitung der zytotoxischen Wirkung aus der Zell-Ausdehnungsweite wird dem Anwender eine weitere nützliche Zwischeninformation zur Verfügung gestellt. Die Ermittlung der Proliferationsfähigkeit und/oder Migrationsfähigkeit ist insbesondere bei abgestuften Zell-Ausdehnungsweiten hilfreich. Des Weiteren können mehrere Zell-Ausdehnungsweiten auch miteinander zu einer Proliferationsfähigkeit und/oder Migrationsfähigkeit verrechnet werden. In einer weiteren alternativen Ausgestaltungsform kann die zytotoxische Wirkung der analytischen Probe auf die tierischen Zellen aus der ermittelten Zell-Ausdehnungsweite im zweiten Auswerteschritt B und aus der Proliferationsfähigkeit und/oder Migrationsfähigkeit im dritten Auswerteschritt B bestimmt werden, wobei beide Ergebnisse berücksichtigt werden. Die zytotoxische Wirkung wird bevorzugt in mehreren Stufen abgebildet.

In einer weiteren bevorzugten Ausgestaltung umfasst das Verfahren nach Anspruch 3 einen Auswertezwischenschritt B. Im Auswertezwischenschritt B wird ein Vergleich der Zell-Ausdehnungsweite mindestens einer Referenzprobe mit der Zell-Ausdehnungsweite der massenspektrometrischen Probe umfassend die analytische Probe durchgeführt. Dieser Vergleich kann in die Ableitung der Proliferationsfähigkeit und/oder der Migrationsfähigkeit in dritten Auswerteschritt B und/oder in die Ableitung der zytotoxischen Wirkung im vierten Auswerteschritt B einbezogen werden. Dadurch kann auf einfache Weise zuverlässig aus der Zell-Ausdehnungsweite auf die zytotoxische Wirkung der analytischen Probe geschlossen werden. In einer alternativen bevorzugten Ausgestaltung wird der Vergleich mit gespeicherten Referenzwerten durchgeführt.

Die Aufgabe wird weiterhin gelöst mit einem System zur Bestimmung der zytotoxischen Wirkung einer analytischen Probe auf tierische Zellen mittels eines ortsauflösendem Massenspektrometers umfassend mindestens ein ortsauflösendes Massenspektrometer zur Erzeugung ortsaufgelösten Massenspektren, einen massenspektrometrischen Probenträger mit Probenstellen und eine Datenverarbeitungseinheit zur Steuerung des ortsauflösenden Massenspektrometers und zur Auswertung der erzeugten ortsaufgelösten Massenspektren, dass dazu eingerichtet ist, jedes ortsaufgelösten Massenspektrums hinsichtlich des Vorhandenseins mindestens einer zellspezifischen massenspektrometrischen Signatur für die tierische Zellen zu analysieren; einen Bedeckungsgrad durch die tierischen Zellen für mindestens eine Teilfläche der Probenstellen und/oder eine Zell-Ausdehnungsweite der tierischen Zellen in mindestens einer Erstreckungsrichtung der Probenstelle zu bestimmen; und aus dem Bedeckungsgrad und/oder aus der Zell-Ausdehnungsweite die zytotoxische Wirkung der analytischen Probe abzuleiten.

Dabei kann die zytotoxische Wirkung bevorzugt unmittelbar aus dem Bedeckungsgrad und/oder der Zell-Ausdehnungsweite, oder mittelbar über eine aus dem Bedeckungsgrad und/oder der Zell-Ausdehnungsweite bestimmte Proliferationsfähigkeit und/oder Migrationsfähigkeit, abgeleitet werden. Unmittelbar schließt explizit weitere einfache mathematische Standardberechnungen, wie zum Beispiel die Mittelwertbildung oder der Abgleich mit einer Datenbank bevorzugt mit ein.

Das ortsauflösende Massenspektrometer ist bevorzugt ein Massenspektrometer aus folgender Gruppe: LDI-MS, MALDI-MS, DESI-MS, MALDESI-MS, SIMS, SIMS-Imaging-MS, MALDI-ToF-MS, MALDI-ToF-ToF-MS, MALDI-MSI, ortsauflösendes Massenspektrometer mit einer Mikro-Fluid-Probenionisierungsvorrichtung. Die Datenverarbeitungseinheit ist bevorzugt ein Computer, welcher im Massenspektrometer integriert oder als separates System eingerichtet ist. Zudem umfasst der Computer bevorzugt eine entsprechende Software mit Algorithmen zur Auswertung und zu Steuerung des Massenspektrometers. Die Auswertung umfasst bevorzugt die Auswerteschritte der oben beschriebenen Verfahren. Auch steuert die Datenverarbeitungseinheit bevorzugt das ortsauflösende Massenspektrometer zur Durchführung des Messschrittes A,B,C der zuvor genannten Verfahren. Insbesondere führt die Software zusammen mit der Datenverarbeitungseinheit bevorzugt Berechnungen für die oben beschriebenen Verfahren durch. So kann zum Beispiel auch der Wert der Referenzprobe mit dem Wert der massenspektrometrischen Probe mittels der Software verrechnet werden. Des Weiteren stellt die Software mit der Datenverarbeitungseinheit bevorzugt die Ergebnisse dar. Dabei wird bevorzugt für jede Probenstelle eine Information über die zytotoxische Wirkung in einer massenspektrometrischen Probe ausgegeben. Alternativ oder zusätzlich wird bevorzugt für jede Probenstelle die Proliferationsfähigkeit und/oder die Migrationsfähigkeit angegeben. Bevorzugt ist das System dazu eingerichtet, die Verfahren nach Anspruch 1,2 und/oder 3 und/oder deren abhängigen Ansprüchen auszuführen. Alternativ kann das System anstelle eines ortsauflösenden Massenspektrometers ein nicht-ortsauflösenden Massenspektrometer umfassen, wenn es dazu eingerichtet ist, dass Verfahren nach Anspruch 3 durchzuführen.

Da sich die offenbarungsgemäßen Verfahren als Betriebsweisen des offenbarungsgemäßen Systems eignet, sind die oben mit Bezug auf die Verfahren erläuterten Ausführungsformen auch in entsprechender Weise auf das System anwendbar.

Ebenso sind die mit Bezug auf die Verfahren nach Anspruch 1 und dessen Unteransprüchen erläuterten Ausführungsformen und Erläuterungen zu den Merkmalen, wie zum Beispiel zum Bedeckungsgrad und/oder zur Proliferationsfähigkeit, bei entsprechender dem Fachmann offensichtlichen Übertragbarkeit vollständig oder teilweise auf die Verfahren nach Anspruch 2 und/oder 3 anwendbar.

Die Anwendungsbreite der Verfahren und des Systems ist groß. Folgend wird dazu in Bezug auf die Anwendungsmöglichkeiten vereinheitlich von dem Verfahren gesprochen, wodurch sämtliche beanspruchten Verfahren und das System miterfasst sind. Das Verfahren kann unter anderem im klinischen Bereich verwendet werden. Das Ergebnis des Verfahrens kann unterstützend bei Diagnosen sein. Aus der zytotoxischen Wirkung im Verfahren kann direkt eine Virulenz abgeleitet werden. Insbesondere kann aus der zytotoxischen Wirkung die Anwesenheit auf einen virulenten Erreger abgeleitet werden. Besonders vorteilhaft ist die Kombination des erfindungsgemäßen Verfahrens mit anderen analytischen Methoden, welche die Anwesenheit eines zytotoxischen Faktors, jedoch nicht dessen zytotoxische Wirkung detektieren können.

So kann das Verfahren zum Beispiel trotz fehlender Informationen über den genauen Resistenzmechanismus von zum Beispiel Viren (zytotoxischer Faktoren) gegenüber einem antiviralen Mittel (Zytotoxizitätsfaktor-neutralisierenden Faktoren) die Resistenz eines Virus gegenüber einem antiviralen Mittel anzeigen. Dazu können als tierischen Zellen zum Beispiel kontinuierliche Zelllinien verwendet werden. Beispielhaft seien hier virale Infektionen mit oder Ausbrüche von den Viren Herpes-simplex-Virus (HSV), Varizella-Zoster-Virus (VZV) oder Cytomegalovirus (CMV) aufgeführt.

Insbesondere kann die Kombination eines Mikroorganismen-Identifikations-Software-Modul (zum Beispiel Brukers MBT Compass inklusive der Library) mit dem Verfahren nicht nur die Anwesenheit eines zytotoxischen Faktors, sondern neben der Gattung und/oder der Spezies eines Mikroorganismus mittels zum Beispiel eines MALDI-Biotyper, eine genauere Klassifizierung, insbesondere von Virulenzphänotypen, vorgenommen werden. In bestimmten Fällen können so auch die Taxonomieebene Subspezies und/oder die Taxonomieebene Pathovar mit dem Verfahren bestimmt werden. Dadurch kann zum Beispiel eine Diagnose beschleunigt und/oder präzisiert werden oder das Klinikmanagement verbessert werden. Beispielhaft seien hier folgend einige klinische Anwendungsbeispiele aufgeführt. Zum Beispiel kann die Anwesenheit hypervirulente Stämme von *Klebsiella pneumoniae* mit dem Verfahren auf einfache und schnelle Weise detektiert werden. Des Weiteren kann mit dem Verfahren zum Beispiel die Anwesenheit von relevante Virulenzphänotypen von uropathogenen *Escherichia coli* Stämmen im Urin einfach und schnell detektiert werden. Das Verfahren eignet sich zum Beispiel auch zur Detektion von *Clostridioides difficite* und/oder dessen Toxins bei schweren Durchfallerkrankungen. Damit bietet das Verfahren eine schnelle und zuverlässige Identifikation der C. difficile-assoziierten Diarrhoe. Des Weiteren eignet sich das Verfahren zum Beispiel auch zur Detektion von Diarrhoe-Toxin produzierenden Stämmen von *Bacillus cereus* nach vorheriger Bestimmung der Spezies *Bacillus cereus* mittels eines Mikroorganismen-Identifikations-Software-Moduls. insbesondere eignet sich das Verfahren auch zur Detektion von virulenten Erregern, deren Virulenz nur unter bestimmten Voraussetzungen gegeben ist und nicht alleine durch die Bestimmung der Anwesenheit der Erreger vorgegeben ist. Zum Beispiel ist das Verfahren geeignet, zwischen einem sehr akuten Verlauf einer Endokarditis aufgrund eines hochvirulenten *Staphylococcus aureus* Stamms und einem gering-akutem Verlauf einer Endokarditis aufgrund eines schwach virulenten *Staphylococcus aureus* Stamms zu unterscheiden.

Des Weiteren ist das Verfahren geeignet, die Anwesenheit von zytotoxischen Faktoren wie zum Beispiel Botulinum-Neurotoxinen in analytischen Proben mittels Nervenzellen als tierische Zellen auf einfache Weise zu detektieren.

Weiterhin ist das Verfahren geeignet, bei Verwendung von Tumorzellen als tierische Zellen und Chemotherapeutika als zytotoxische Faktoren, die Empfindlichkeit von Tumorzellen gegenüber Chemotherapeutika zur Abschätzung der therapeutischen Wirksamkeit einer Chemotherapie auf einfache Weise zu bestimmen.

### Definitionen

- Das ortsaufgelöste Massenspektrum umfasst die massenspektrometrischen Rohdaten bis hin zu einer bearbeiteten Peakliste, die nur noch die Intensitäten der jeweiligen Massenbereiche (m/z) und die zugehörigen Messpositionen aufweist. Dabei kann ein ortsaufgelöstes Massenspektrum aus einer Vielzahl von Intensitätswerten in einem zusammenhängenden Massenbereich als auch aus den Intensitätswerten mehrerer getrennter Massenbereiche bestehen. Das ortsaufgelöste Massenspektrum kann vor der Verwendung im Verfahren einer Signalverarbeitung unterzogen werden, die beispielsweise eine Korrektur (Subtraktion) der Basislinie, eine Glättung von Massensignalen, eine Eliminierung von Rauschsignalen, eine Auswahl von Massensignalen über einem festgelegten Rauschwert, und/oder die Peakfindung (peak finding) und/oder die Merkmalsdetektion (feature detection) zur Erstellung einer Peakliste umfasst. Das ortsaufgelöste Massenspektrum ist bevorzugt ein Einzelmassenspektrum. Jedes Einzelmassenspektrum wird bevorzugt durch einen einzelnen Laserbeschuss oder Strahlbeschuss erzeugt. Das ortsaufgelöste Massenspektrum kann ein Summenmassenspektrum einer Messposition sein, insoweit Einzelmassenspektren der gleichen Messposition zusammengefasst wurden. Jedoch ist ein ortsaufgelöstes Massenspektrum im Sinne der Erfindung kein Summenmassenspektrum der Probenstelle, d.h. die Einzelmassenspektren verschiedener Messpositionen der Probenstelle werden nicht zusammengefasst. Bevorzugte m/z-Bereiche liegen zwischen m/z 1000 und m/z 20000, weiterhin bevorzugt zwischen m/z 2000 und m/z 15000, insbesondere bevorzugt zwischen m/z 2000 und m/z 10000.
- Der Begriff Verfahren wird bevorzugt für die gesamte Erfindung verwendet, falls für den Fachmann aus dem Kontext eine nähere Spezifizierung des Verfahrens nicht erkennbar oder naheliegend ist. Somit bezieht sich der Begriff Verfahren, falls aus dem Kontext eine nähere Spezifizierung des Verfahrens nicht erkennbar oder naheliegend ist, in seiner nicht näher spezifizierbaren Form bevorzugt auf einen Teil oder die Gesamtheit der Ansprüche (inklusive Systemanspruch) - soweit gemäß dem Fachmann anwendbar.
- Ursprungsprobe ist eine entnommene Probe. Bevorzugt ist sie weitgehend unverändert ab der Probennahme.
- Zytotoxizität und die zytotoxische Wirkung ist das Maß für die Fähigkeit von bestimmten zytotoxischen Faktoren tierische Zellen, wie zum Beispiel Immunzelleri, zu schädigen. Sie kann konzentrationsabhängig sein. Die zytotoxische Wirkung auf tierische Zellen kann proliferationsverlangsamend und/oder proliferationshemmend sein. Des Weiteren kann die zytotoxische Wirkung auf tierische Zellen wachstumshemmend und/oder wachstumsverlangsamend sein. Des Weiteren kann die zytotoxische Wirkung auf tierische Zellen zellteilungshemmend und/oder zellteilungsverlangsamend sein. Des Weiteren kann die zytotoxische Wirkung auf tierische Zellen nekrotisch sein. Des Weiteren kann die zytotoxische Wirkung auf tierische Zellen zellmigrationshemmend und/oder zellmigrationsverlangsamend sein.
- Zytotoxische Faktoren sind Faktoren mit (potentieller) zytotoxischer Wirkung. Sie können die Proliferation, Zellmigration, Teilung und/oder Wachstum der tierischen Zellen verlangsamen oder unterbinden oder die tierischen Zellen zerstören. Zytotoxische Faktoren umfassen alle chemischen Substanzen oder biologische Materie, insbesondere Bakterien, Viren, Pilze, Mikroorganismen, Parasiten, tot oder lebend, aktiv oder inaktiv, die tatsächlich oder potenziell eine zytotoxische Wirkung auf mindestens eine Art von tierischen Zellen aufweisen. Sie können zum Beispiel gelöst, suspendiert, oder adsorbiert oder immobilisiert an einer Oberfläche (zum Beispiel auf der Oberfläche des massenspektrometrischen Probenträgers) vorliegen. Umgebungsbedingen wie zum Beispiel Temperatur oder die atmosphärische LuftZusammensetzung sind keine zytotoxischen Faktoren.
- Tierische Zellen sind Zellen von Organismen aus der taxonomischen Stufe: Reich: Tiere. Zu der Gruppe der Tiere im Rahmen der Begriffsdefinition "tierische Zellen" gehören unter anderem Zellen von Insekten, wie zum Beispiel Gliederfüßer und/oder Ringelwürmer; Wirbeltieren, Säugetieren und/oder Menschen. Protozoen und/oder Euglena sind von den tierischen Zellen bevorzugt ausgeschlossen. Weiterhin bevorzugt sind die tierischen Zellen Gewebe-bildende Zellen. Die tierischen Zellen sind bevorzugt nicht Teil der analytischen Probe. Die tierischen Zellen können zum Beispiel Proben von Gewebezellen oder Tumorzellen von Menschen oder Tieren, insbesondere Säugetieren, sein. Insbesondere können sie kontinuierliche Zelllinien sein. Die tierischen Zellen sind im Rahmen der Anmeldung tierische Zellen, dessen Proliferationsfähigkeit und/oder Migrationsfähigkeit herangezogen wird, um die zytotoxische Wirkung eines zytotoxischen Faktors oder analytischen Probe zu bestimmen.
- Mikroorganismen umfassen bevorzugt alle prokaryotischen Organismen, umfassend Archaeabakteria und Bacteria. Des Weiteren umfassen Mikroorganismen alle Protozoen. Des Weiteren umfassen Mikroorganismen bestimmte eukaryotischen Organismen, wie zum Beispiel bestimmte (insbesondere einzellige) Pilze, wie zum Beispiel Hefen, und (insbesondere einzellige) Algen. Gewebebildende eukaryotischen Organismen zählen nicht zu den Mikroorganismen. Bevorzugt sind eukaryotischen Mikroorganismen einzellig. Des Weiteren umfassen Mikroorganismen Viren. Mikroorganismen können bevorzugt tierische, einschließlich menschliche, pflanzliche oder mikrobielle Wirte bzw. Wirtszellen besiedeln und/oder infizieren und/oder auf und/oder in ihnen vorkommen. Zu den Mikroorganismen zählen weiterhin bevorzugt auch Mikroorganismen, die unabhängig von Wirten in der Umwelt (z.B. Wasser, Boden, Luft, Oberflächen) vorkommen. Der Begriff mikrobiell ist dem Begriff Mikroorganismus gleichzusetzen.
- Probenstelle ist bevorzugt eine Teilfläche des massenspektrometrischen Probenträgers, auf der eine massenspektrometrischen Probe oder Referenzprobe aufgetragen und vermessen wird. Des Weiteren ist die Probenstelle bevorzugt eine Teilfläche des massenspektrometrischen Probenträgers, die zur Aufnahme und/oder Kultivierung einer massenspektrometrischen Probe bestimmt ist.
- Restflüssigkeit: Die Flüssigkeiten sämtlicher Bestandteile der massenspektrometrischen Probe sind zusammen die Restflüssigkeit. Zellinhalte intakter tierischer Zellen sind nicht Teil der Restflüssigkeit.
- Unter Ausdehnung und flächiger Ausdehnung der tierischen Zellen ist bevorzugt die flächige Ausbreitung der tierischen Zellpopulation zu verstehen, die durch Proliferation, Wachstum und/oder Teilung der tierischen Zellen hervorgerufen wird. Alternativ oder zusätzlich zur Proliferation, Wachstum und/oder Teilung kann die flächige Ausbreitung im Rahmen der Ausdehnung und flächigen Ausdehnung der tierischen Zellen auch durch Zellmigration der tierischen Zellen hervorgerufen werden.
- Konfluenzgrad ist der Bedeckungsgrad der Oberfläche einer Probenstelle mit adhärent-wachsenden tierischen Zellen. Adhärent-wachsende tierische Zellen sind hier gleichbedeutend mit dem Ausdruck adhärent-proliferierende tierische Zellen.
- Analytische Proben umfassen zu untersuchende Proben mit unbekanntem Auswerteergebnis, nämlich des potentiellen Vorliegens einer zytotoxischen Wirkung auf die tierischen Zellen. Damit zählt auch eine Lösung mit bekannten zytotoxischen Stoffen als analytische Probe, solange das Auswerteergebnis unklar ist. So ist beispielsweise auch eine Lösung mit einer als zytotoxisch eingestuften Substanz in verschiedenen Konzentrationen und/oder in Kombination mit verschiedene Gewebezellen als tierische Zellen als analytische Probe einzustufen. Die analytische Probe ist eine Probe, die mit dem ortsauflösenden Massenspektrometer analysiert wird. Sie umfasst potenziell mindestens einen zytotoxischen Faktor. Die analytische Probe kann in unverändertem Zustand, d.h. zum Beispiel als Ursprungsprobe, auf der Probenstelle bereitgestellt werden, oder sie kann vorzugsweise vor der Bereitstellung auf der Probenstelle einer Anreicherungs- oder Kulturvorbereitung, einer Konzentration und/oder einem Extraktions- oder Reinigungsschritt gemäß den dem Fachmann bekannten Methoden unterzogen werden, um den potenziellen zytotoxischen Faktor aufzukonzentrieren, zu isolieren oder aufzureinigen.
- Referenzproben dienen der Generierung von Referenzwerten. Referenzproben sind bevorzugt Proben mit bekanntem und/oder erwartetem Auswertergebnis. Bevorzugt durchlaufen Sie das Verfahren parallel zur massenspektrometrischen Probe umfassend die analytische Probe mit unbekanntem Auswertergebnis. Alternativ werden die Referenzwerte im Vorfeld generiert und in einer Datenbank gespeichert und bei Durchführung des beanspruchten Verfahrens abgerufen. Zum besseren Verständnis werden folgend Beispiele für Referenzproben genannt. Die Referenzproben können bevorzugt aus folgender Gruppe ausgewählt werden:
   - Leerprobe; lediglich die verwendete Matrix, falls eine Matrix im Verfahren verwendet wird, wird vermessen
   - Massenspektrometrische Probe bestehend aus Nährlösung
   - Massenspektrometrische Probe bestehend aus tierischen Zellen
   - Massenspektrometrische Probe bestehend aus Nährlösung und tierischen Zellen
   - Massenspektrometrische Probe bestehend aus einem oder mehreren zytotoxischen Faktoren
   - Massenspektrometrische Probe bestehend aus einem oder mehreren . Zytotoxizitätsfaktor-neutralisierenden Faktoren
   - Massenspektrometrische Probe umfasst Nährlösung, tierischen Zellen und ein oder mehrere zytotoxische Faktoren mit bekannter zytotoxischer Wirkung auf die verwendeten tierischen Zellen und somit bekannten Auswerteergebnis.

Somit dienen die Referenzproben unter anderem der Zuordnung der Massenbereiche zu Bestandteilen der massenspektrometrischen Probe. Dadurch können zellspezifisch und nicht-zellspezifische massenspektrometrische Signale differenziert werden und zellspezifischen massenspektrometrischen Signaturen identifiziert werden. Des Weiteren dienen sie zur Pröliferationskontrolle oder als Prozesskontrolle.

Die Auswahl dieser Referenzproben, das Hinzufügen weiterer hier nicht aufgeführter Referenzproben, sowie deren Kombination erschließt sich dem Fachmann aus der jeweiligen Anwendung, dem Versuchsziels und der Zusammensetzung der analytischen Probe und/oder massenspektrometrischen Probe. Alternativ kann in einer anderen Ausgestaltung auf gespeicherte Referenzwerte für das Verfahren zurückgegriffen werden.
- Proliferation umfasst das Wachstum und die Teilung von tierischen Zellen. Die Proliferationsfähigkeit ist ein Maß für das erfolgte Wachstum und die Teilung der tierischen Zellen. Die Proliferationsfähigkeit wird im Verfahren mittels dem Bedeckungsgrad oder der Zell-Ausdehnungsweite bestimmt.
- Migrationsfähigkeit umfasst die Fähigkeit der Zellen, Ihren Standort eigenständig zu verändern. Diese aktive eigenständige (selbsttätige) Ortsveränderung der tierischen Zellen ist die Zellmigration. Die Migrationsfähigkeit schließt bevorzugt die ungerichtete Spontanbewegung, die gerichtete, chemotaktische Bewegung und/oder die Änderung der Bewegungsgeschwindigkeit (Chemokinetik) mit ein.
- Zellspezifisches massenspektrometrisches Signal bedeutet, dass das massenspektrometrische Signal durch einen beliebigen Bestandteil der tierischen Zelle erzeugt worden ist. Das zellspezifisches massenspektrometrisches Signal ist den verwendeten tierischen Zellen der massenspektrometrischen Probe zuordenbar, bevorzugt eindeutig zuordenbar. Die Bestandteile der tierischen Zelle können dabei Proteinen, wie zum Beispiel Ribosomen, Peptide, Nukleinsäuren, Lipide, und/oder sonstige Bestandteile der tierischen Zelle sein. Bevorzugt sind von der tierischen Zelle in die Umgebung sekretierte Stoffe nicht umfasst.
- Eine zellspezifische massenspektrometrische Signatur weist eine Auswahl von einem oder mehreren zellspezifischen massenspektrometrische Signalen auf, die zum Beispiel durch Ihre Intensität, Position/Massenbereich und/oder Entfernung zu anderen massenspektrometrischen Signalen zur eindeutigen Identifizierung von tierischen Zellen an der vermessenen Messposition auch in Anwesenheit von Nährmedium, zytotoxischen Faktoren, Matrix und sonstigen Bestandteilen der massenspektrometrischen Proben geeignet sind. Bevorzugt ist das mindestens eine ausgewählte zellspezifische massenspektrometrische Signal markant. Das heißt, es ist eindeutig von den nicht-zellspezifischen massenspektrometrischen Signalen und von dem Hintergrundrauschen durch seine Intensität und Massenbereich (m/z) und/oder m/z-Beabstandung von anderen massenspektrometrischen Signalen unterscheidbar. Zellspezifische massenspektrometrische Signale, deren Intensität weitgehend in etwa vom Streubereich des Hintergrundrauschens umfasst sind, zählen nicht zu den markanten zellspezifischen massenspektrometrischen Signalen. Bevorzugt liegt eine Spektrenbibliothek an massenspektrometrischen Signaturen in einer Datenbank gespeichert vor. Weiterhin bevorzugt ist in der Spektrenbibliothek jedem Typ einer tierischen Zelle mindestens eine zellspezifische massenspektrometrische Signatur zugeordnet. Die gespeicherten Referenzspektren der zellspezifischen massenspektrometrischen Signaturen können zum Beispiel Massen, Massentoleranzen, mittlere Intensitäten, Streuung der Intensitäten und Auftrittswahrscheinlichkeiten enthalten. Die zellspezifischen massenspektrometrischen Signaturen und/oder zellspezifischen massenspektrometrischen Signale, insbesondere der Spektrenbibliothek, werden zum Beispiel aus mehrfachen Messungen an verschiedenen Zellkultivierungen der tierischen Zellen mit und insbesondere ohne zytotoxischen Faktoren, erzeugt. Bevorzugt werden die zellspezifischen massenspektrometrischen Signale und/oder zellspezifischen massenspektrometrischen Signaturen dabei automatisch durch eine Computerauswertung isoliert.
- Bevorzugt werden Vergleiche, Abgleiche, Zuweisungen, Ableitungen, Berechnungen, Ermittlungen, Bestimmungen, und weitere automatisierbare Schritte des Verfahrens mittels einer Datenverarbeitungseinheit durchgeführt. Die Datenverarbeitungseinheit ist bevorzugt ein Computer mit entsprechender Software.

Die vorliegende Erfindung wird nachstehend anhand von in den Zeichnungen dargestellten Ausführungsbeispielen näher erläutert.
- Fig. 1: zeigt schematisch eine Seitenansicht eines ortsauflösenden Massenspektrometers und zwar in (a) eine schematische Ansicht eines einfachen MALDI-ToF-MS und in (b) eine schematische Ansicht eines massenspektrometrischen Probenträgers.
- Fig. 2: zeigt schematisch verschiedene Ausführungsformen des erfindungsgemäßen Verfahrens nach Anspruch 1 und zwar in (a) und (b) Verfahren für nichtmatrix-basierte ortsauflösenden Massenspektrometer; in (c) und (d) Verfahren für matrix-basierte ortsauflösenden Massenspektrometer.
- Fig. 3: zeigt schematisch die einzelnen Schritte einer besonders bevorzugten Ausführungsform des erfindungsgemäßen Verfahrens nach Anspruch 1 entsprechend des in Fig. 2(c) dargestellten Ablaufschemas im Detail, wobei linksseitig in der Regel die Aufsicht und rechtsseitig die zugehörige Seitenansicht dargestellt ist. Das Beispiel zeigt eine Ausführungsform für ein matrix-basierten ortsauflösende Massenspektrometer mit Probenpräparationsschritt.
- Fig. 4: zeigt schematisch den Messschritt A des Verfahrens nach Anspruch 1 im Detail.
- Fig. 5: zeigt schematisch den ersten Auswerteschritt A nach Anspruch 1 im Detail.
- Fig. 6: zeigt schematisch die Ergebnisse der Auswerteschritte des Verfahrens nach Anspruch 1 entsprechend Fig. 2(c) im Detail.
- Fig. 7: zeigt schematisch verschiedene weitere bevorzugte Ausführungsformen des erfindungsgemäßen Verfahrens nach Anspruch 1 in Kombination mit ausgewählten abhängigen Unteransprüchen und zwar in (a) Verfahren für nicht-matrix-basierte ortsauflösenden Massenspektrometer; in (b), (c) und (d) Verfahren für matrix-basierte ortsauflösenden Massenspektrometer. Dabei weisen (b) bis (d) zusätzlich einen Auswerteschzwischenschritt auf.
- Fig. 8: zeigt schematisch bevorzugte Ausführungsformen des erfindungsgemäßen Verfahrens nach Anspruch 3 und zwar in (a) und (c) Verfahren für nichtmatrix-basierte ortsauflösenden Massenspektrometer; in (b) und (d) Verfahren für matrix-basierte ortsauflösenden Massenspektrometer.
- Fig. 9: zeigt schematisch Ausführungsformen für die Probenstellen mit der ersten und der zweiten Teilfläche entsprechend Anspruch 3 und zwar in (a) eine kreisrunde Probenstelle; in (b) eine länglich ausgeformte Probenstelle; in (c) die Probenstelle aus (a) im oder nach dem Kultivierungsschritt bei fehlender oder schwacher zytotoxischer Wirkung der analytischen Probe.
- Fig. 10: zeigt schematisch die Ergebnisse der Auswerteschritte des Verfahrens nach Anspruch 3 entsprechend Fig. 8(a, b, d) im Detail.

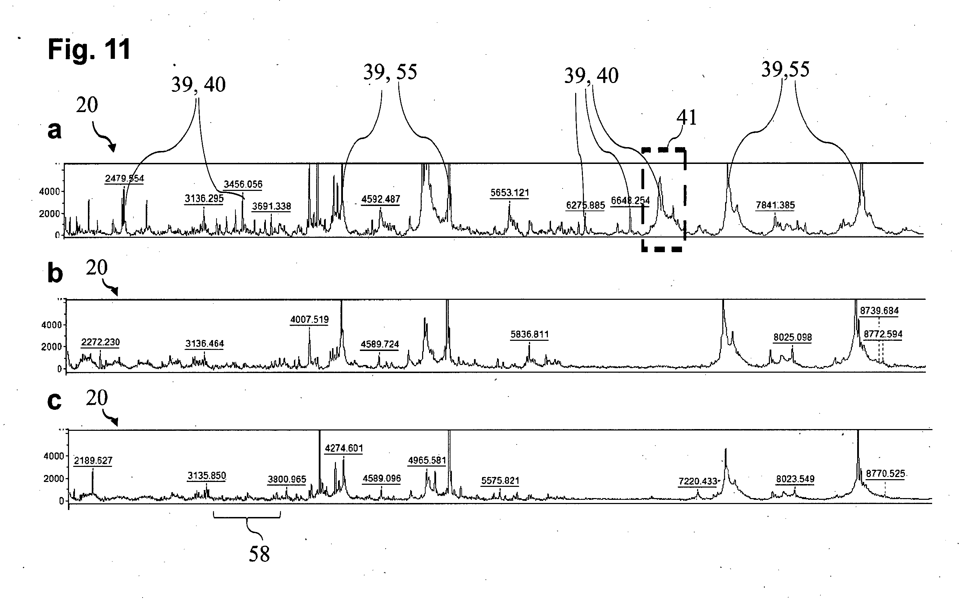

- Fig. 11: zeigt beispielhaft Massenspektren verschiedener Referenzproben, und zwar in (a) einer ersten Referenzprobe mit einem ausgesuchten Typ von tierischen Zellen und ohne zytotoxischen Faktor, in (b) einer Referenzprobe umfassend die gleichen tierischen Zellen wie in (a) in Anwesenheit eines zytotoxischen Faktors, und in (c) einer dritten Referenzprobe, wobei die dritte Referenzprobe keine tierischen Zellen und keinen zytotoxischen Faktor umfasst und wobei die dritte Referenzprobe Nährmedium und einen internen Standard umfasst. Die Referenzspektren dienen hier der Bestimmung der zellspezifischen massenspektrometrischen Signale und der zellspezifischen massenspektrometrischen Signaturen.
- Fig. 12: zeigt schematisch verschiedene Ausführungsformen des erfindungsgemäßen Verfahrens nach Anspruch 2 und zwar in (a) und (b) Verfahren für nichtmatrix-basierte Massenspektrometer; in (c) und (d) Verfahren für matrixbasierte Massenspektrometer.

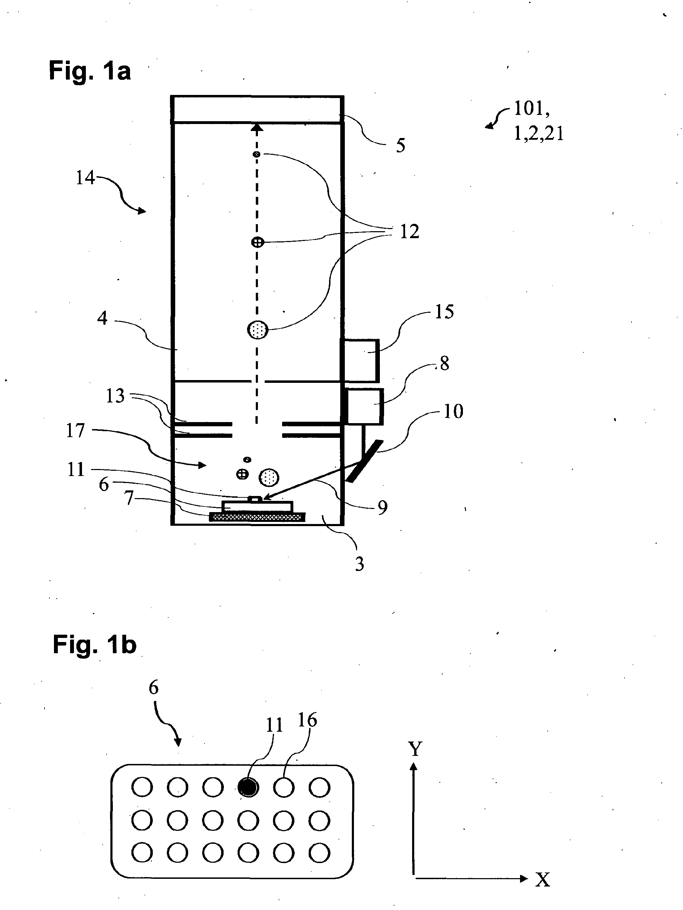

Fig. 1a zeigt eine vereinfachte schematische Darstellung eines einfachen Massenspektrometers (MS) 101, genauer eines ortsauflösenden Massenspektrometers 1, genauer eines MALDI-ToF-MS 2, genauer eines matrix-basierten ortsauflösenden Massenspektrometers 21. Dies ist nur eine beispielhafte Beschreibung eines Massenspektrometers. Andere Systemkomponenten, wie zum Beispiel ein lonenselektor, eine Fragmentierungszelle, oder ein lonenreflektor, usw. können ebenfalls in das System integriert sein.

Das MALDI-ToF-MS 2 umfasst eine lonenquelle 3 und einen Flugzeitmassenanalysator 14 (mit axialem loneneinschuss). Der Flugzeitmassenanalysator 14 umfasst ein Flugrohr 4, einen Detektor 5, Beschleunigungselektroden 13 und eine Beschleunigungsstrecke 17. Die lonenquelle 3 umfasst einen massenspektrometrischer Probenträger 6, der auf einem XY-Tisch 7 positioniert ist. Der massenspektrometrischer Probenträger 6 kann durch den XY-Tisch 7 in zwei Dimensionen X und Y bewegt werden. Des Weiteren umfasst die lonenquelle 3 einen Laser 8 und ein Spiegel- und/oder Linsensystem 10. Der Laser 8 erzeugt einen Laserstrahl 9. Der Laserstrahl 9 wird über das Spiegel- und/oder Linsensystem 10 auf eine vorbereite massenspektrometrische Probe 11 auf dem massenspektrometrischer Probenträger 6 gerichtet. Der Laserstrahl 9 erzeugt aus der vorbereiteten massenspektrometrischen Probe 11 Probenionen 12. Diese Probenionen 12 werden durch die Beschleunigungselektroden 13 beschleunigt und durchlaufen das Flugrohr 4 des Flugzeitmassenanalysators 14. Hier werden die Probenionen 12 aufgrund ihrer Masse und Ladung getrennt. Schließlich werden die Probenionen 12 mit dem Detektor 5 detektiert. Das Vakuum im Flugrohr 4 wird durch eine Vakuumpumpe 15 erzeugt.

Die Position des Laserstrahls 9 auf der Probenplatte 6 wird durch Verschieben des Spiegel- /Linsensystems 10 und/oder durch Verschieben des XY-Tisches 7 verändert. Bevorzugt weist der Laserstrahl beim Auftreffen auf der massenspektrometrischen Probe einen Strahldurchmessern von 1 nm bis 300 µm auf. Weiter bevorzugt weist der Laserstrahl beim Auftreffen auf der massenspektrometrischen Probe einen Strahldurchmessern von 100 nm bis 100 µm auf. Weiterhin bevorzugt weist der Laserstrahl beim Auftreffen auf der massenspektrometrischen Probe einen Strahldurchmessern von 1 µm bis 10 µm auf.

Die Vorbereitung der massenspektrometrischen Probe erfolgt im Falle eines MALDI-ToF-MS 2 durch Auftragung einer Matrix 29 auf die massenspektrometrischen Probe 11 und anschließender Trocknung.

Das Massenspektrometer 101 kann in einem positiven oder in einem negativen lonenmodus arbeiten. Die Massenspektrometer 101 sind unterteilbar in matrix-basierte Massenspektrometer, umfassend matrix-basierte ortsauflösende Massenspektrometer 21, und nicht-matrix-basierte Massenspektrometer, umfassend ortsauflösende nicht-matrix-basierte Massenspektrometer. Ein matrix-basiertes Massenspektrometer benötigt zur lonisierung der Moleküle in der lonenquelle, umfassend die Analyten der massenspektrometrischen Probe 11, und zur Erzeugung von Probenionen 12 eine zusätzliche chemische Substanz, die Matrix 29. In der Regel wird diese als Flüssigkeit aufgetragen. Diese führt bei Trocknung zur Cokristallisation mit dem Analyten. Dabei werden die Analytmoleküle in die Kristalle der Matrix 29 "eingebaut", während sich die Kristalle bilden. Häufig werden als Matrix-Hauptsubstanz kleine organische Moleküle gewählt, die bei der verwendeten Laserwellenlänge (z. B. Stickstofflaser bei einer Wellenlänge von 337,1 nm) Energie stark absorbieren. Als Matrix-Hauptsubstanz wird zum Beispiel Sinapinsäure, 2,5-Dihydroxybenzoesäure, α-Cyanohydroxyzimtsäure, 2,4,6-Trihydroxyacetophenon oder verwendet. Diese sind in der Regel mit Lösungsmitteln und weiteren Substanzen zur Matrix 29 zusammengemischt. Häufig werden als Lösungsmittel Wasser und organische Lösungsmittel wie Acetonitril oder Ethanol verwendet. Als weitere Substanz wird häufig Trifluoressigsäure (TFA) als Gegenionenquelle verwendet, um [M+H]-Ionen zu generieren. Als Beispiel für eine Matrix 29 sei hier Sinapinsäure in Acetonitril:Wasser:TFA (50:50:0.1) genannt. Kurze hochenergetische Laserpulse führen dann nach Relaxation im Kristallgitter zu explosionsartigen Teilchenablösungen an der Oberfläche des Kristalls. Gemeinsam mit der Matrix 29 werden dabei die eingeschlossenen Analytmoleküle mit in das Vakuum des Massenspektrometers 101 überführt und so der massenspektrometrischen Analyse zugänglich.

Es sei hier ausdrücklich vermerkt, dass im Rahmen der Erfindung auch alternative Massenspektrometer 101 und insbesondere alternative ortsauflösende Massenspektrometer 1 mit alternativen lonenquelle 3, die Ionen von massenspektrometrischen Proben auf einem massenspektrometrischer Probenträger 6 erzeugt, wie z.B. Desorption Elektrospray Ionisierung (DESI)-MS, eingesetzt werden können.

Die Massenspektrometer 101 können auch Flugzeit-Massenanalysatoren 14 mit orthogonalem loneneinschuss, elektrostatische lonenfallen (z.B. Orbitrap^{®}), Hochfrequenzlonenfallen, lonenzyklotronresonanz-lonenfallen und Quadrupole Massenfilter umfassen. Andere Systemkomponenten, wie zum Beispiel ein lonenleitsysteme, ein Massenfilter, lonenmobilitätsseparatoren und Fragmentierungszellen usw., können ebenfalls in das massenspektrometrische System integriert sein.

FIG. 1b zeigt eine vereinfachte schematische Darstellung einer Aufsicht eines einfachen massenspektrometrischer Probenträgers 6. Die Verschiebungsrichtungen der Position des Laserstrahls 9 auf der Probenplatte 6 in X-Richtung und Y-Richtung mittels des XY-Tisches 7 und/oder mittels des Spiegel- und oder Linsensystems 10 ist ebenfalls angegeben. Der massenspektrometrischer Probenträger 6 weist Probenstellen 16 auf. Auf die Probenstellen werden massenspektrometrische Proben 11 aufgetragen. Ein massenspektrometrischer Probenträger 6 weist mindestens eine, in der Regel aber mehrere Probenstellen .16 auf. Die Probenstellen 16 sind bevorzugt als kreisrunde Flächen ausgebildet. Bevorzugt weist die Probenstelle einen Durchmesser von 0.1 mm bis 10 cm auf. Weiterhin bevorzugt weist die Probenstelle einen Durchmesser von 0.2 mm bis 8 cm auf. Weiterhin bevorzugt weist die Probenstelle einen Durchmesser von 0.5 mm bis 4 cm auf. Besonders bevorzugt weist die Probenstelle einen Durchmesser von 1 mm bis 1 cm auf. Ganz besonders bevorzugt weist die Probenstelle einen Durchmesser von 2 mm bis 6 mm auf. Der Durchmesser der Probenstelle kann zum Beispiel in Abhängigkeit des Strahldurchmessers, der Konzentration der tierischen Zellen und/oder der Zellgröße der tierischen Zellen gewählt sein. Um die Ionisierung zumindest im Bereich der Probenstellen 16 zu fördern, ist die Oberfläche des massenspektrometrischer Probenträger 6 leitfähig. Ein solcher massenspektrometrischer Probenträger 6 besteht beispielsweise aus einem Polymer wie Polypropylen, aus Keramik oder Glas, wobei der massenspektrometrischer Probenträger 6 mit einer Schicht aus rostfreiem Stahl überzogen sein kann. Das Polymer, die Keramik oder das Glas kann zudem ein leitfähiges Material wie z. B. Ruß enthalten. Alternativ besteht der massenspektrometrische Probenträger 6 beispielsweise aus einem leitenden Metall. Massenspektrometrische Probenträger 6 weisen in der Regel 48 bis 96 Probenstellen 16 auf. Zusätzlich können sie mehrere Referenzbereiche aufweisen.

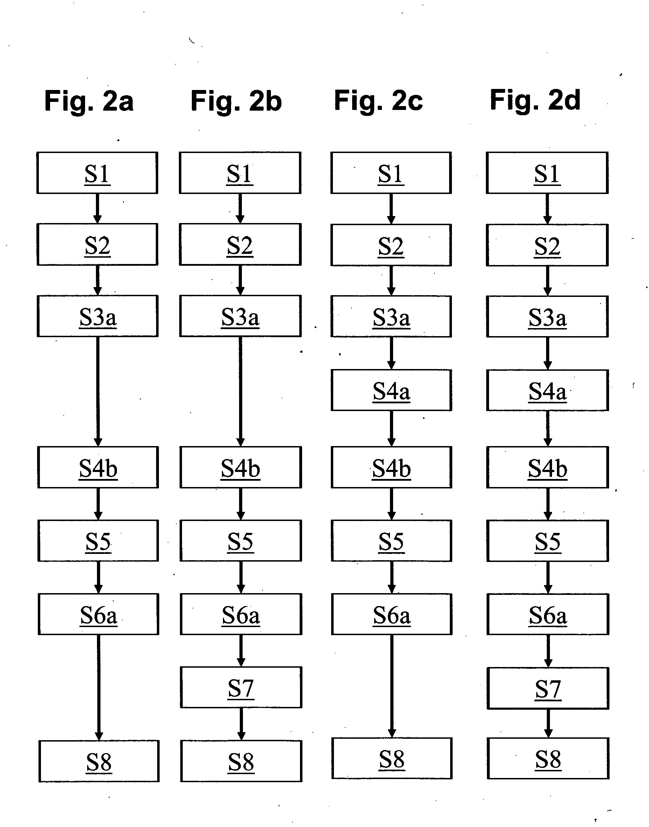

Fig.2 zeigt schematisch verschiedene bevorzugte Ausführungsformen mit den einzelnen Schritten des erfindungsgemäßen Verfahrens nach Anspruch 1. Die Verfahrensschritte werden bevorzugt in dargestellter sequenzieller Reihenfolge durchgeführt.

So zeigt Fig. 2a eine besonders bevorzugte Ausführungsform des Verfahrens, in dem folgenden Schritte durchgeführt werden: Probenbereitstellungsschritt A S1, Kultivierungsschritt S2, Flüssigkeitsentfernungsschritt S3a, Messschritt A S4b, erste Auswerteschritt A S5, zweiter Auswerteschritt A S6a, vierter Auswerteschritt A S8. Die zytotoxische Wirkung 46 wird im vierten Auswerteschritt A S8 unmittelbar aus dem Bedeckungsgrad 45 abgeleitet. Typische dem Fachmann bekannte Zwischenberechnungen sind im Rahmen der unmittelbaren Ableitung jedoch nicht ausgeschlossen. Diese bevorzugte Ausführungsform des Verfahrens wird für nicht-matrix-basierte ortsauflösenden Massenspektrometern verwendet. Eine ortspezifische Auftragung einer Matrix 29 findet nicht statt.

Das Verfahren weist somit folgende Merkmale auf:
Verfahren zur Bestimmung der zytotoxischen Wirkung 46 einer analytischen Probe auf tierische Zellen 23, umfassend folgende Schritte:
- einen Probenbereitstellungsschritt A S1, in dem eine massenspektrometrische Probe 11, umfassend tierische Zellen 23, Nährmedium und die analytische Probe, welche potenziell einen zytotoxischen Faktor aufweist, auf mindestens einer Probenstelle 16 eines massenspektrometrischen Probenträgers 6 bereitgestellt wird;
- einen Kultivierungsschritt S2, in dem die massenspektrometrische Probe 11 auf der Probenstelle 16 des massenspektrometrischen Probenträgers 6 in einer Kultivierungsvorrichtung 24 inkubiert wird;
- einen Flüssigkeitsentfernungsschritt S3a, in dem Restflüssigkeit der massenspektrometrischen Probe 11 von der Probenstelle 16 entfernt wird;
- einen Messschritt A S4b, in dem ortsaufgelöste Massenspektren 38 an einer Vielzahl von Messpositionen 33 in mindestens einer Teilfläche 34 der Probenstelle 16 mittels eines nicht-matrix-basierten ortsauflösenden Massenspektrometers 1 aufgenommen werden;
- einen ersten Auswerteschritt A S5, in dem jedes ortsaufgelöste Massenspektrum 38 hinsichtlich des Vorhandenseins einer zellspezifischen massenspektrometrischen Signatur 41 für die tierischen Zellen 23 analysiert wird und jeder Messposition 33 ein Zellpräsenzwert 42 zugewiesen wird;
- einen zweiten Auswerteschritt A S6a, in dem ein Bedeckungsgrad 45 durch tierische Zellen 23 für mindestens die Teilfläche 34 der Probenstelle 16 aus den Zellpräsenzwerten 42 der Messpositionen 33 bestimmt wird;
- einen vierten Auswerteschritt A S8, in dem die zytotoxische Wirkung 46 der analytischen Probe auf die tierischen Zellen 23 aus dem bestimmten Bedeckungsgrad 45 des zweiten Auswerteschritt A S6a abgeleitet wird.

Fig. 2b zeigt eine alternative bevorzugte Ausführungsform des Verfahrens, in dem die folgenden Schritte durchgeführt werden: Probenbereitstellungsschritt A S1, Kultivierungsschritt S2, Flüssigkeitsentfernungsschritt S3a, Messschritt A S4b, erster Auswerteschritt A S5, zweiter Auswerteschritt A S6a, dritter Auswerteschritt A S7,vierter Auswerteschritt A S8. Die zytotoxische Wirkung 19 wird im vierten Auswerteschritt A S8 aus der Proliferationsfähigkeit der tierischen Zellen abgeleitet und somit mittelbar aus dem Bedeckungsgrad 45. Diese bevorzugte Ausführungsform des Verfahrens wird für nicht-matrix-basierte ortsauflösenden Massenspektrometern verwendet. Eine ortspezifische Auftragung einer Matrix 29 findet nicht statt.

Das Verfahren weist somit folgende Merkmale auf:
Verfahren zur Bestimmung der zytotoxischen Wirkung 46 einer analytischen Probe auf tierische Zellen 23, umfassend folgende Schritte:
- einen Probenbereitstellungsschritt A S1, in dem eine massenspektrometrischen Probe 11, umfassend tierische Zellen 23, Nährmedium und die analytische Probe, welche potenziell einen zytotoxischen Faktor aufweist, auf mindestens einer Probenstelle 16 eines massenspektrometrischen Probenträgers 6 bereitgestellt wird;
- einen Kultivierungsschritt S2, in dem die massenspektrometrische Probe 11 auf der Probenstelle 16 des massenspektrometrischen Probenträgers 6 in einer Kultivierungsvorrichtung 24 inkubiert wird;
- einen Flüssigkeitsentfernungsschritt S3a, in dem Restflüssigkeit der massenspektrometrischen Probe 11 von der Probenstelle 16 entfernt wird;
- einen Messschritt A S4b, in dem ortsaufgelöste Massenspektren 38 an einer Vielzahl von Messpositionen 33 in mindestens einer Teilfläche 34 der Probenstelle 16 mittels eines nicht-matrix-basierten ortsauflösenden Massenspektrometers aufgenommen werden;
- einen ersten Auswerteschritt A S5, in dem jedes ortsaufgelöste Massenspektrum 38 hinsichtlich des Vorhandenseins einer zellspezifischen massenspektrometrischen Signatur 41 für die tierischen Zellen 23 analysiert wird und jeder Messposition 33 ein Zellpräsenzwert 42 zugewiesen wird;
- einen zweiten Auswerteschritt A S6a, in dem ein Bedeckungsgrad 45 durch tierische Zellen 23 für mindestens die Teilfläche 34 der Probenstelle 16 aus den Zellpräsenzwerten 42 der Messpositionen 33 bestimmt wird;
- einen dritten Auswerteschritt A S7, in dem eine Proliferationsfähigkeit aus dem bestimmten Bedeckungsgrad 45 abgeleitet wird;
- einen vierten Auswerteschritt A S8, in dem die zytotoxische Wirkung 46 der analytischen Probe auf die tierischen Zellen 23 mittelbar aus dem bestimmten Bedeckungsgrad 45 des zweiten Auswerteschritt A S6a abgeleitet wird, wobei hierzu die zytotoxische Wirkung 46 der analytischen Probe auf die tierischen Zellen 23 aus der abgeleiteten Proliferationsfähigkeit des dritter Auswerteschritt A S7 abgeleitet wird.

Fig. 2c zeigt eine besonders bevorzugte alternative Ausführungsform des Verfahrens, in dem die folgenden Schritte durchgeführt werden: Probenbereitstellungsschritt A S1, Kultivierungsschritt S2, Flüssigkeitsentfernungsschritt S3a, Probenpräparationsschritt A S4a, Messschritt A S4b, erster Auswerteschritt A S5, zweiter Auswerteschritt A S6a, vierter Auswerteschritt A S8. Die zytotoxische Wirkung 19 wird im vierten Auswerteschritt A S8 unmittelbar aus dem Bedeckungsgrad 45 abgeleitet. Diese bevorzugte Ausführungsform des Verfahrens wird für matrix-basierte ortsauflösenden Massenspektrometern 1,21 verwendet. Vor dem Messschritt A S4b findet im Probenpräparationsschritt A S4a eine ortspezifische Auftragung einer Matrix 29 statt.

Das Verfahren weist somit folgende Merkmale auf:
Verfahren zur Bestimmung der zytotoxischen Wirkung 46 einer analytischen Probe auf tierische Zellen 23, umfassend folgende Schritte:
- einen Probenbereitstellungsschritt A S1, in dem eine massenspektrometrischen Probe 11, umfassend tierische Zellen 23, Nährmedium und die analytische Probe, welche potenziell einen zytotoxischen Faktor aufweist, auf mindestens einer Probenstelle 16 eines massenspektrometrischen Probenträgers 6 bereitgestellt wird;
- einen Kultivierungsschritt S2, in dem die massenspektrometrische Probe 11 auf der Probenstelle 16 des massenspektrometrischen Probenträgers 6 in einer Kultivierungsvorrichtung 24 inkubiert wird;
- einen Flüssigkeitsentfernungsschritt S3a, in dem Restflüssigkeit der massenspektrometrischen Probe 11 von der Probenstelle 16 entfernt wird;
- einen Probenpräparationsschritt A S4a, in dem eine Präparation der Probenstelle 16 durch ortstreue Auftragung einer Matrix 29 auf mindestens die eine Teilfläche 34 der Probenstelle 16 erfolgt;
- einen Messschritt A S4b, in dem ortsaufgelöste Massenspektren 38 an einer Vielzahl von Messpositionen 33 in mindestens einer Teilfläche 34 der Probenstelle 16 mittels eines matrix-basierten ortsauflösenden Massenspektrometers 1 aufgenommen werden;
- einen ersten Auswerteschritt A S5, in dem jedes ortsaufgelöste Massenspektrum 38 hinsichtlich des Vorhandenseins einer zellspezifischen massenspektrometrischen Signatur 41 für die tierischen Zellen 23 analysiert wird und jeder Messposition 33 ein Zellpräsenzwert 42 zugewiesen wird;
- einen zweiten Auswerteschritt A S6a, in dem ein Bedeckungsgrad 45 durch tierische Zellen 23 für mindestens die Teilfläche 34 der Probenstelle 16 aus den Zellpräsenzwerten 42 der Messpositionen 33 bestimmt wird;
- einen vierten Auswerteschritt A S8, in dem die zytotoxische Wirkung 46 der analytischen Probe auf die tierischen Zellen 23 aus dem bestimmten Bedeckungsgrad 45 des zweiten Auswerteschrittes A S6a abgeleitet wird.

Fig. 2d zeigt eine bevorzugte alternative Ausführungsform des Verfahrens, in dem die folgenden Schritte durchgeführt werden: Probenbereitstellungsschritt A S1, Kultivierungsschritt S2, Flüssigkeitsentfernungsschritt S3a, Probenpräparationsschritt A S4a, Messschritt A S4b, erster Auswerteschritt A S5, zweiter Auswerteschritt A S6a, dritter Auswerteschritt A S7, vierter Auswerteschritt A S8. Die zytotoxische Wirkung wird im vierten Auswerteschritt A S8 aus der Proliferationsfähigkeit der tierischen Zellen abgeleitet und somit mittelbar aus dem Bedeckungsgrad 45. Diese bevorzugte Ausführungsform des Verfahrens wird für matrix-basierte ortsauflösenden Massenspektrometern 1,21 verwendet.

Vor dem Messschritt A S4b findet im Probenpräparationsschritt A S4a eine ortspezifische Auftragung einer Matrix 29 statt.

Das Verfahren weist somit folgende Merkmale auf:
Verfahren zur Bestimmung der zytotoxischen Wirkung 46 einer analytischen Probe auf tierische Zellen 23, umfassend folgende Schritte:
- einen Probenbereitstellungsschritt A S1, in dem eine massenspektrometrischen Probe 11, umfassend tierische Zellen 23, Nährmedium und die analytische Probe, welche potenziell einen zytotoxischen Faktor aufweist, auf mindestens einer Probenstelle 16 eines massenspektrometrischen Probenträgers 6 bereitgestellt wird;
- einen Kultivierungsschritt S2, in dem die massenspektrometrische Probe 11 auf der Probenstelle 16 des massenspektrometrischen Probenträgers 6 in einer Kultivierungsvorrichtung 24 inkubiert wird;
- einen Flüssigkeitsentfernungsschritt S3a, in dem Restflüssigkeit der massenspektrometrischen Probe 11 von der Probenstelle 16 entfernt wird;
- einen Probenpräparationsschritt A S4a, in dem eine Präparation der Probenstelle 16 durch ortstreue Auftragung einer Matrix 29 auf mindestens die eine Teilfläche 34 der Probenstelle 16 erfolgt;
- einen Messschritt A S4b, in dem ortsaufgelöste Massenspektren 38 an einer Vielzahl von Messpositionen 33 in mindestens einer Teilfläche 34 der Probenstelle 16 mittels eines matrix-basierten ortsauflösenden Massenspektrometers 1 aufgenommen werden;
- einen ersten Auswerteschritt A S5, in dem jedes ortsaufgelöste Massenspektrum 38 hinsichtlich des Vorhandenseins einer zellspezifischen massenspektrometrischen Signatur 41 für die tierischen Zellen 23 analysiert wird und jeder Messposition 33 ein Zellpräsenzwert 42 zugewiesen wird;
- einen zweiten Auswerteschritt A S6a, in dem ein Bedeckungsgrad 45 durch tierische Zellen 23 für mindestens die Teilfläche 34 der Probenstelle 16 aus den Zellpräsenzwerten 42 der Messpositionen 33 bestimmt wird;
- einen dritten Auswerteschritt A S7, in dem eine Proliferationsfähigkeit aus dem bestimmten Bedeckungsgrades 45 abgeleitet wird;
- einen vierten Auswerteschritt A S8, in dem die zytotoxische Wirkung 46 der analytischen Probe auf die tierischen Zellen 23 mittelbar aus dem bestimmten Bedeckungsgrades 45 des zweiten Auswerteschritt A S6a abgeleitet wird, wobei hierzu die zytotoxische Wirkung 46 der analytischen Probe auf die tierischen Zellen 23 aus der abgeleiteten Proliferationsfähigkeit des dritter Auswerteschritt A S7 abgeleitet wird.

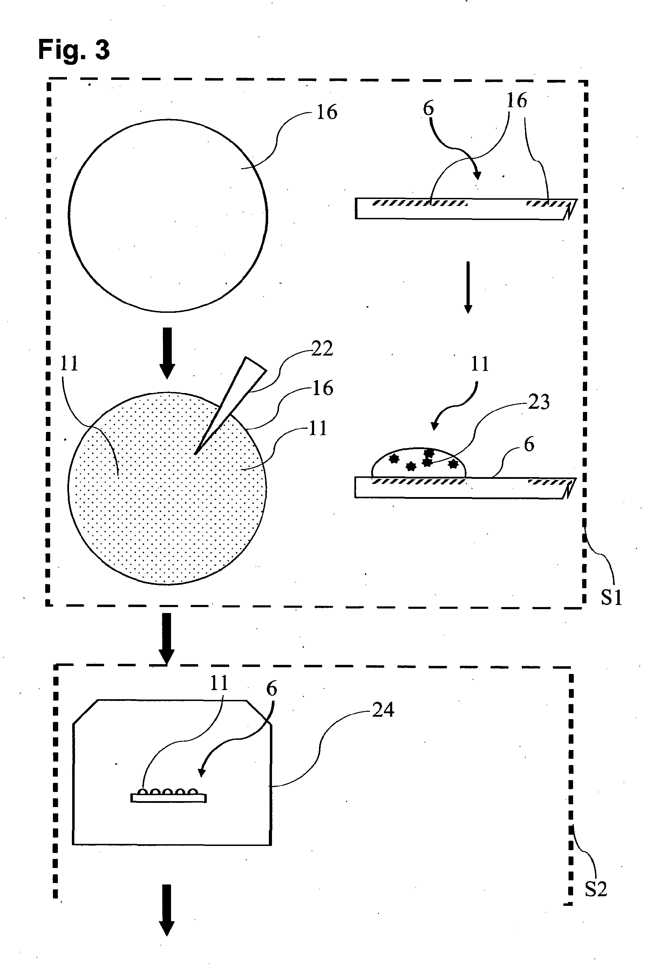

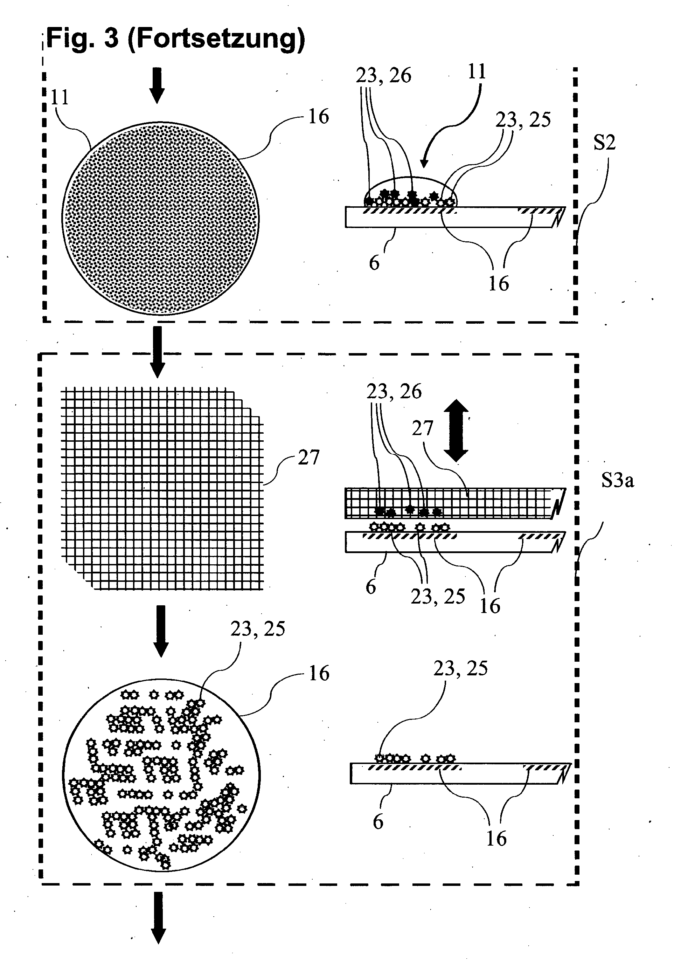

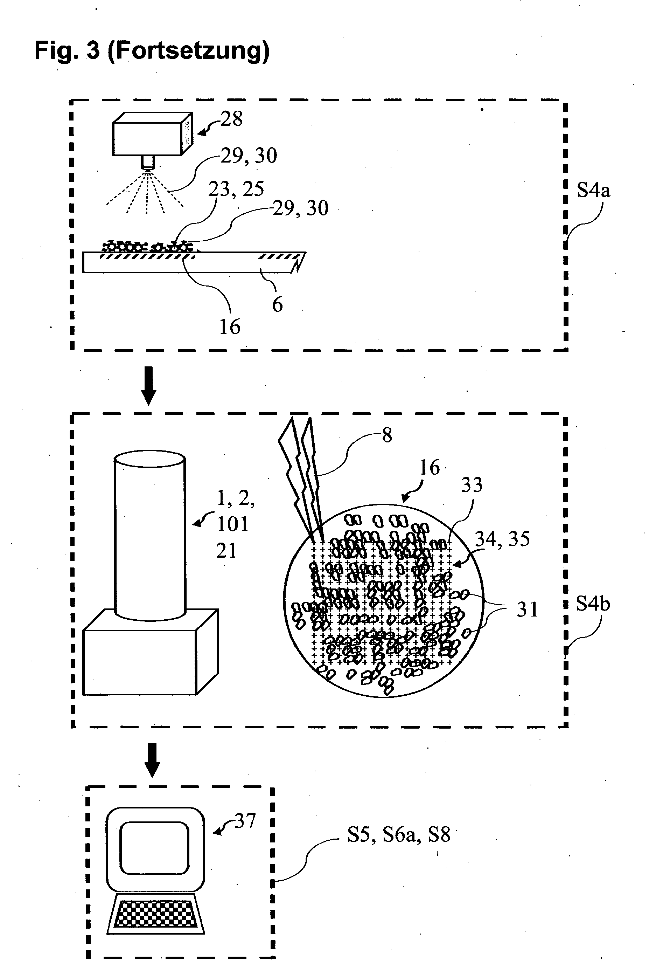

Fig. 3 zeigt schematisch die einzelnen Schritte der besonders bevorzugten Ausführungsformen des erfindungsgemäßen Verfahrens nach Anspruch 1 entsprechend des in Fig. 2(c) dargestellten Ablaufschemas im Detail. Dabei können bestimmte einzelne Schritte vollständig oder teilweise ebenfalls Schritte der Ansprüche nach Anspruch 2 und/oder 3 abbilden.

Im Probenbereitstellungsschritt A S1 wird auf einer Probenstelle 16 auf einem massenspektrometrischen Probenträger 6 eine massenspektrometrische Probe 11 mittels einer Applikationsvorrichtung 22 aufgebracht. In diesem Beispiel erfolgt die Auftragung mittels einer Pipette. Die massenspektrometrische Probe 11 umfasst tierische Zellen 23, welche proliferationsfähig sind.

Des Weiteren umfasst die massenspektrometrische Probe 11 eine analytische Probe und Nährmedium. Zur beispielhaften Verdeutlichung des Verfahrens weist die analytische Probe keine zytotoxische Wirkung auf bzw. sie beinhaltet keine zytotoxischen Faktoren. In diesem Beispiel werden die Bestandteile der massenspektrometrische Probe 11 bereits in einer Mischung aufgetragen. In anderen bevorzugten Ausführungsformen werden die Bestandteile der massenspektrometrischen Probe 11 teilweise sequentiell oder voneinander getrennt aufgetragen. Des Weiteren ist ebenfalls das Nutzen von vorpräparierten massenspektrometrischen Probenträgern 6 umfasst, welche beispielsweise bereits die tierischen Zellen 23 und/oder das Nährmedium in getrockneter Form auf den Probenstellen 16 aufweisen. Die Auftragung der Bestandteile der massenspektrometrische Probe 11 erfolgt in diesem Beispiel in Form einer vermischten Lösung/Suspension. Neben der massenspektrometrische Probe 11 wird ebenfalls eine Referenzprobe auf einer weiteren Probenstelle 16 aufgetragen, welche zwar die tierischen Zellen 23 und das Nährmedium, jedoch keinen zytotoxischen Faktor oder analytische Probe umfasst (nicht dargestellt). Die Referenzprobe wird analog zur massenspektrometrische Probe 11 behandelt und später im Rahmen der Auswertung zur Erzeugung eines Referenzwertes verwendet.

Die tierischen Zellen 23 sind beispielweise proliferationsfähige adhärent-wachsende Zellen kontinuierlicher Zelllinien, wie z.B. CHO- oder MDCK-Zellen.

Nach Auftragung der massenspektrometrische Probe 11 erfolgt im Kultivierungsschritt S2 die Kultivierung der tierischen Zellen 23. Dazu wird der massenspektrometrischen Probenträger 6 in einer Kultivierungsvorrichtung 24 verbracht. Die Kultivierungsvorrichtung 24 ist in diesem Beispiel ein Zellkulturschrank. Hier erfolgt die Proliferation der tierischen Zellen 23. Wenn die tierischen Zellen 23 noch nicht adhäriert bereitgestellt worden sind, erfolgt hier ebenfalls das Adhärieren der tierischen Zellen 23, wenn kein zytotoxischer Faktor anwesend ist. Ebenso erfolgt in diesem Schritt die Proliferation der tierischen Zellen 23, wenn kein zytotoxischer Faktor anwesend ist. Die Kultivierungsvorrichtung 24 stellt optimale Wachstumsbedingungen, das heißt Proliferationsbedingungen, für die tierischen Zellen 23 zur Verfügung. Sie kontrolliert Luftfeuchtigkeit, Temperatur, CO₂-Gehalt; O₂-Gehalt und/oder weitere Parameter in der Kultivierungsvorrichtung 24. Die Dauer der Kultivierung wird an der Referenzprobe oder anhand einer zuvor festgelegten Zeit eingestellt. So ist bei ausreichender Kultivierungsdauer in der Kultivierungsvorrichtung 24 eine Erhöhung des Bedeckungsgrades 45 in der Referenzprobe feststellbar. Nach der Kultivierung liegen auf der Probenstelle 16 adhärente tierische Zellen 23, 25 und nicht-adhärente tierische Zellen 23, 26 vor. Die nicht-adhärenten tierischen Zellen 23, 25 sind z.B. tierische Zellen 23, die sich noch in Suspension befinden und sich nicht ablagern und adhärieren konnten oder abgestorbene adhärente tierischen Zellen 23, die sich wieder abgelöst haben.

Im folgenden Flüssigkeitsentfernungsschritt S3a werden die flüssigen Bestandteile, wie z.B. das Nährmedium und Puffer, der massenspektrometrischen Probe 11 entfernt. Die adhärenten tierischen Zellen 23,25 verbleiben dabei an ihrer adhärierten Position auf der Probenstelle 16. Dies erfolgt beispielsweise mittels einer Flüssigkeitsaufsaugvorrichtung 27 wie z.B. mit einem saugfähigen Material, welches mit der massenspektrometrischen Probe 11 auf der Probenstelle 16 in Kontakt gebracht wird. Beim Absaugen der Flüssigkeit der massenspektrometrischen Probe 11 werden zudem nicht-adhärierte tierische Zellen 26 teilweise oder weitgehend vollständig mitentfernt. Das Ergebnis am Ende des Flüssigkeitsentfernungsschritts S3a sind (weitgehend) trockene Probenstellen 16.

Da in diesem Beispiel ein matrix-basiertes ortsauflösendes Massenspektrometer 1 im Messschritt A S4b eingesetzt wird, wird vor der Messung der vorgelagerte Probenpräparationsschritt A S4a durchgeführt, welcher bei nicht matrix-basierten ortsauflösenden Massenspektrometern unterbleibt. Der massenspektrometrische Probenträger 6 wird im Probenpräparationsschritt A S4a mittels einer Sprühvorrichtung 28 mit einer Matrix 29 besprüht. Die Sprühvorrichtung 28 erzeugt Matrix-Mikrotröpfchen 30 und/oder Matrix-Nanotröpfchen. Des Weiteren ist die Sprühvorrichtung 28 derart eingestellt und ausgestaltet, dass die bereits aufgetragenen Matrixtröpfchen während der Auftragung der Matrix 29 trocknen können. Dies wird erreicht, indem die Sprührate der Sprühvorrichtung angepasst ist, die Matrixtröpfchen möglichst klein sind und/oder eine Trocknung der Oberfläche der massenspektrometrische Probenträger 6 erfolgt. Zudem kann eine hochflüchtige Matrix 29 verwendet werden. Dadurch wir die Matrix 29 ortstreu aufgetragen. Damit wird sichergestellt, dass sich kein oder lediglich ein kurz vorherrschender und dünner kontinuierlicher Matrixfilm auf der Probenstelle 16 bildet. Die aufgetragene Matrix 29 schließt unter anderem die tierischen Zellen 23 auf, wodurch aufgeschlossene tierische Zellen 31 erzeugt werden. Entscheidend bei der ortstreuen Auftragung ist, dass die Zellbestandteile und Zellinhalte der tierischen Zellen 23 weitgehend an der Position der jeweiligen tierischen Zelle 23 verbleiben.

Nach Trocknung der Matrix 29 wird die massenspektrometrische Probe 11 durch ein matrix-basiertes ortsauflösendes Massenspektrometer 21 im Messschritt A S4b vermessen. Das matrix-basiertes ortsauflösendes Massenspektrometer 21 ist in diesem Beispiel ein MALDI-ToF-MS 2. Dazu werden an einer Vielzahl von Messpositionen 33 ortsaufgelöste Massenspektren 38 in einer Teilfläche 34 der Probenstelle 16 aufgenommen. Die Messpositionen 33 sind in Form eines Rasters 35 über der Teilfläche 34 der Probenstelle 16 verteilt. Die Teilfläche 34 wird in diesem Beispiel vom Raster 35 aufgespannt. Die ortsaufgelöste Massenspektren 38 werden an eine als Computer mit Software ausgebildete Datenverarbeitungseinheit 37 weitergeleitet, von dieser verarbeitet und ausgewertet. Die Datenverarbeitungseinheit 37 führt die Auswerteschritte aus. In diesem Beispiel führt sie den ersten Auswerteschritt A S5, den zweiten Auswerteschritt A S6a und den vierten Auswerteschritt A S8 aus. Danach gibt Sie ein Ergebnis aus, dass anzeigt, ob die analytische Probe eine zytotoxische Wirkung 46 aufwies. Zudem wird der Grad der zytotoxischen Wirkung 46 angegeben. Die Datenverarbeitungseinheit 37 dient ebenfalls der Steuerung des matrixbasierten ortsaufgelösten MS 21 im Messschritt A S4b und der Speicherung der erzeugten Daten.

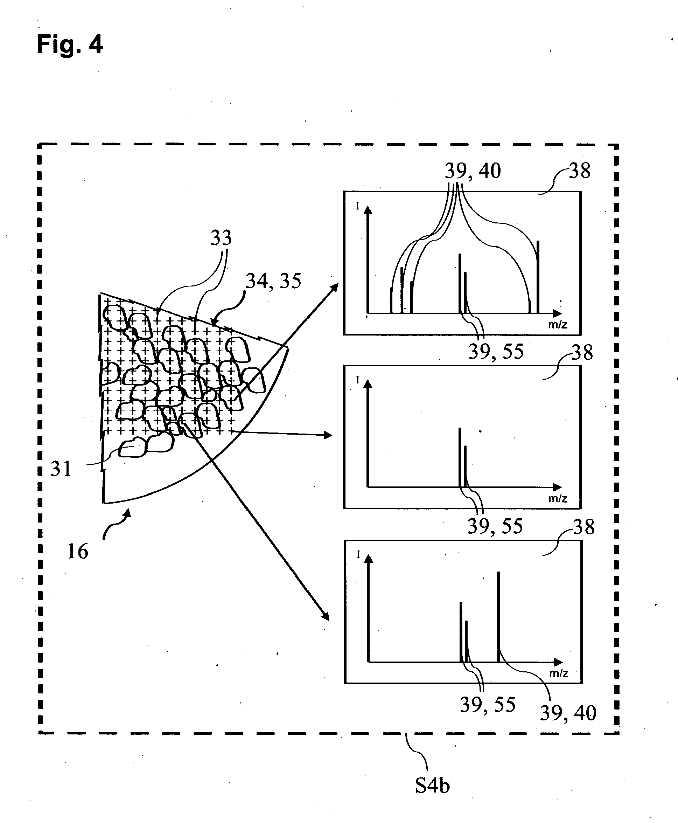

Fig. 4 zeigt schematisch den Messschritt A S4b des Verfahrens nach Anspruch 1 im Detail. Von jeder Messposition 33 auf der Teilfläche 34 der Probenstelle 16 wird mindestens ein ortsaufgelöstes Massenspektrum 38 aufgenommen. Das ortsaufgelöste Massenspektrum 38 weist massenspektrometrische Signale 39 auf. Diese werden unter anderem von Resten des Nährmediums, den tierischen Zellen 23, und der analytischen Probe erzeugt. Der Inhalt und die Bestandteile der tierischen Zellen 23 erzeugen zellspezifische massenspektrometrische Signale 40. Diese werden von nicht-zellspezifische massenspektrometrische Signalen 55 mit Hilfe einer Datenbank unterschieden. Die Datenbank wird bevorzugt zuvor mit Hilfe von Referenzproben der einzelnen Bestandteile der massenspektrometrischen Probe 11 erzeugt oder aus anderen Datenbänken erstellt. Die Datenbank umfasst Referenzdaten (Referenzwerte) und/oder Referenzdatensätze. Referenzdaten und Referenzdatensätze können spektrometrische Messdaten oder davon abgeleitete Daten umfassen. Sie können von tierischen Zellen 23, Nährmedien, Matrizes 29, Standards, zytotoxischen Faktoren (also Bakterien, Viren und/oder Pilzen oder Bestandteilen dieser Gruppen; chemischen Stoffen, und sonstigen potenziell zytotoxisch wirkenden Faktoren), Zytotoxizitätsfaktor-neutralisierenden Faktoren, und/oder von sonstigen Bestandteilen einer analytischen Probe stammen.

Referenzdaten oder ein Referenzdatensatz kann ein Massenspektrum und/oder ein aus einem Massenspektrum abgeleitetes Daten-n-Tupel umfassen. Ein Beispiel für ein Daten-n-Tupel ist eine Liste aus Abundanzen im massenspektrometrischen Signal 39 und diesen zugeordnete enge Massenkanäle, gegebenenfalls zuzüglich etwaiger Metainformation zum Massenspektrum. Abgeleitete Daten können beispielsweise aus den ursprünglichen Massenspektren erzeugte Peaklisten der prominentesten massenspektrometrischen Signale 39 oder anderweitig reduzierte Daten, z.B. mittels Grundliniensubtraktion, Ableitung, Rauschentfernung und dergleichen, umfassen.

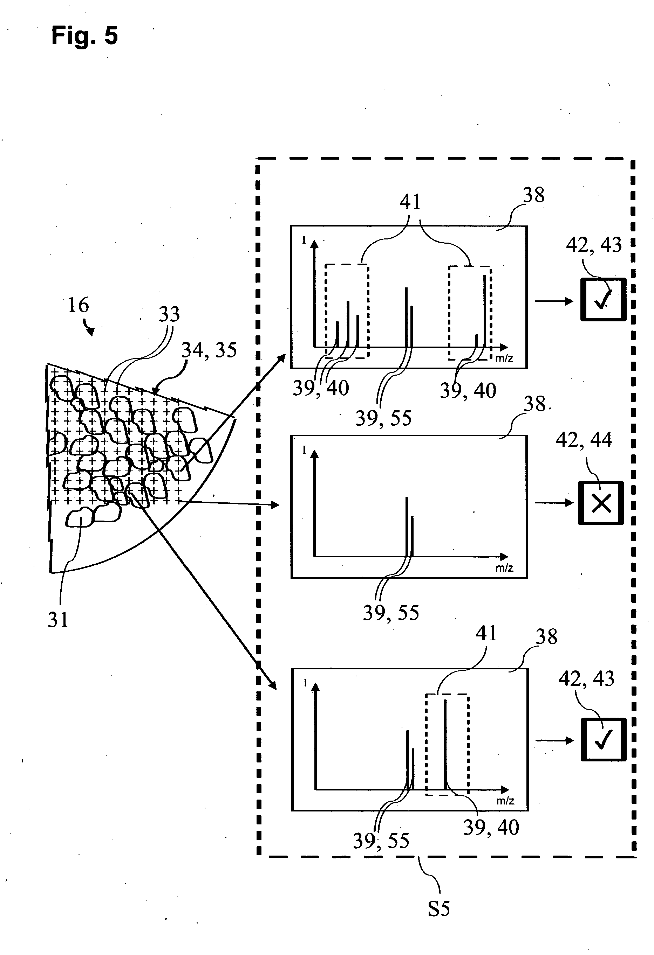

Bei matrix-basierten ortsauflösenden Massenspektrometern 21 befinden sich nach dem Probenpräparationsschritt A,B S4a,S104a anstelle der weitgehend intakten tierischen Zellen 23 nun weitgehend aufgeschlossene tierische Zellen 31 an den Messpositionen 33. Ist auf einer Messposition 33 eine aufgeschlossene tierische Zelle 31 vorhanden, weist das ortsaufgelöste Massenspektrum 38 ebenfalls zellspezifische massenspektrometrische Signale 40 auf. Bei nicht-matrix basierten ortsauflösenden Massenspektrometern werden die massenspektrometrischen Signalen 40 weitgehend von intakten tierischen Zellen 23 erzeugt.
Auf einem Teil der Messpositionen 33 in Fig. 5 befinden sich somit aufgeschlossene tierische Zellen 31 und auf einem anderen Teil der Messpositionen 33 befinden sich keine aufgeschlossene tierische Zellen 31 oder tierische Zellen 23. Ist auf einer Messposition 33 keine aufgeschlossene tierische Zelle 31 vorhanden, weist das ortsaufgelöste Massenspektrum 38 keine zellspezifische massenspektrometrische Signale 40 auf.

Fig. 5 zeigt schematisch den ersten Auswerteschritt A S5 des Verfahrens nach Anspruch 1 im Detail. Zuerst wird jedes ortsaufgelöste Massenspektrum 38 hinsichtlich des Vorhandenseins zellspezifischer massenspektrometrische Signaturen 41 analysiert. Sie werden von der Datenverarbeitungseinheit 37 mittels eines Abgleichs der ortsaufgelösten Massenspektren 38 der einzelnen Messpositionen 33 der massenspektrometrischen Probe 11 umfassend die analytische Probe oder davon abgeleitete Daten mit erstellten oder gespeicherten Massenspektren von Referenzproben oder davon abgeleitete Daten identifiziert. Beim Abgleich wird der Übereinstimmungsgrad zwischen den Messdaten oder davon abgeleiteten Daten der massenspektrometrischen Probe umfassend die analytische Probe und den erstellten oder gespeicherten Messdaten oder davon abgeleiteten Daten der Referenzproben ermittelt. Zum Beispiel kann der Übereinstimmungsgrad mit Ähnlichkeitsmaßzahlen ("scores") oder dessen Logarithmus ("log(score)"), wie sie zum Beispiel von kommerziellen Systemen wie dem MALDI Biotyper^{®} (Bruker) verwendet werden, ermittelt werden. Des Weiteren kann auch ein Klassifikator, z.B. in Form einer Rechenvorschrift, zur Klassifikation verwendet werden, welcher zuvor mittels Klassifikator-Training ermittelt worden ist. Dem kundigen Fachmann sind weitere Verfahren bekannt. Die Datenverarbeitungseinheit 37 sucht nach zellspezifischen massenspektrometrischen Signaturen 41 in jedem ortsaufgelösten Massenspektrum 38, um festzulegen, ob an der Messposition 33 eine tierische Zelle vorlag. Dabei wird für jede Messposition 33 einer Probenstelle 16 bevorzugt die gleiche massenspektrometrischen Signatur 41 verwendet. In Abhängigkeit der Anwesenheit einer zellspezifischen massenspektrometrischen Signatur 41 in dem jeweiligen ortsaufgelösten Massenspektrum 38 wird jeder Messposition 33 ein Zellpräsenzwert 42 zugewiesen. Wenn eine zellspezifische Signatur 41 in einem ortsaufgelösten Massenspektrum 38 gefunden wurde, wird der Messposition 33 ein positiver Zellpräsenzwert 43 zugewiesen, der die Anwesenheit einer tierischen Zelle 23 an der Messposition 33 anzeigt. Wenn keine zellspezifische Signatur 41 in einem ortsaufgelösten Massenspektrum 38 gefunden wurde, wird der Messposition 33 ein negativer Zellpräsenzwert 44 zugewiesen, der die Abwesenheit einer tierischen Zelle 23 anzeigt.

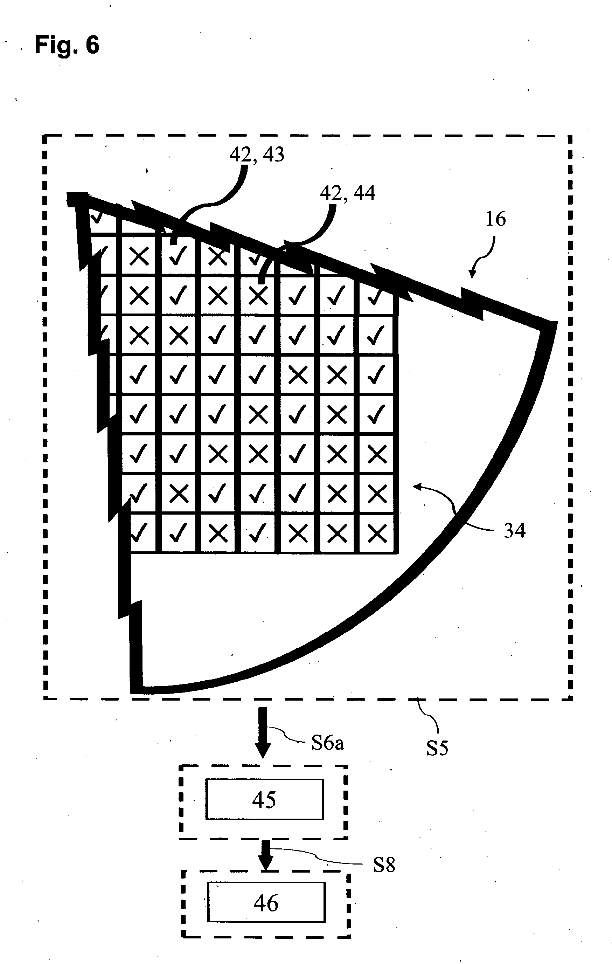

Fig. 6 zeigt schematisch die Ergebnisse der Auswerteschritte: zweiter Auswerteschritt A S6a und vierter Auswerteschritt A S8 des Verfahrens nach Anspruch 1 entsprechend Fig. 2(c) im Detail. Die schematische Darstellung dient dabei in erster Linie zu Verdeutlichungszwecken im Rahmen dieser Anmeldung. Auch die Auswerteschritte aus Fig. 6 werden von der Datenverarbeitungseinheit 37 ausgeführt. Nachdem jeder Messposition 33 im ersten Auswerteschritt A S5 ein Zellpräsenzwert 42 zugewiesen worden ist, wird im zweiten Auswerteschritt A S6a ein Bedeckungsgrad 45 der Teilfläche 34 der Probenstelle 16 mit tierischen Zellen 23 daraus bestimmt. Dazu wird die Teilfläche 34 der Probenstelle 16 entsprechend dem Raster 35 eingeteilt. Anschließend wird der Anteil der Teilfläche mit positiven Zellpräsenzwerten 43 an der gesamten Teilfläche 34 der Probenstelle 16 berechnet. Alternativ wird die Anzahl der positiven Zellpräsenzwerte 43 ins Verhältnis zur Anzahl der negativen Zellpräsenzwerte 44 gesetzt.

Aus dem Bedeckungsgrad 45 wird anschließend im vierten (letzten) Auswerteschritt S8 die zytotoxische Wirkung 46 abgeleitet. Hierzu wird der Bedeckungsgrad 45 mit dem Bedeckungsgrad 45 der Referenzprobe und/oder mit gespeicherten Bedeckungsgraden 45 verglichen. Anschließend erfolgt die Ableitung der zytotoxischen Wirkung 46. Hierdurch wird auch bestimmt, ob ein zytotoxischer Faktor in der massenspektrometrischen Probe 11 anwesend ist.

Die zytotoxische Wirkung 46 des zytotoxischen Faktors wird beispielsweise wie folgt abgestuft:
i) Keine zytotoxische Wirkung 46, wobei der Bedeckungsgrad 45 der massenspektrometrischen Probe 11 umfassend die analytische Probe dem Bedeckungsgrad 45 einer Referenzprobe ohne zytotoxischen Faktor entspricht. Die tierischen Zellen 23 konnten ungehindert proliferieren;
ii) starke zytotoxische Wirkung 46, wobei keine tierischen Zellen 23 oder keine Proliferation der tierischen Zellen 23 auf der Probenstelle 16 festgestellt werden konnten. Es gab keine Adhärenz, kein Wachstum und/oder keine Zellteilung der tierischen Zellen 23. Dabei entspricht der Bedeckungsgrad 45 der massenspektrometrischen Probe 11 umfassend die analytische Probe bevorzugt dem Bedeckungsgrad 45 einer Referenzprobe mit zytostatisch-wirkendem zytotoxischen Faktor, oder ist geringer, oder es konnten keine tierischen Zellen 23 auf der Probenstelle 16 detektiert werden;
iii) mittelstarke zytotoxische Wirkung 46, wobei ein Bedeckungsgrad 45 kleiner 50% der Referenzprobe festgestellt werden konnte;
iv) schwache zytotoxische Wirkung46, wobei ein Bedeckungsgrad 45 größer 50% der Referenzprobe festgestellt werden konnte.

In einer alternativen bevorzugten Ausgestaltung wird aus dem Bedeckungsgrad 45 die Proliferationsfähigkeit abgeleitet (nicht in Fig. 6 dargestellt). Dabei wird vom Bedeckungsgrad 45 abgeleitet, wie gut die tierischen Zellen 23 unter den Bedingungen der massenspektrometrischen Probe 11 proliferieren können. Die Ableitung der zytotoxischen Wirkung 46 erfolgt dann alternativ oder zusätzlich aus der Proliferationsfähigkeit.

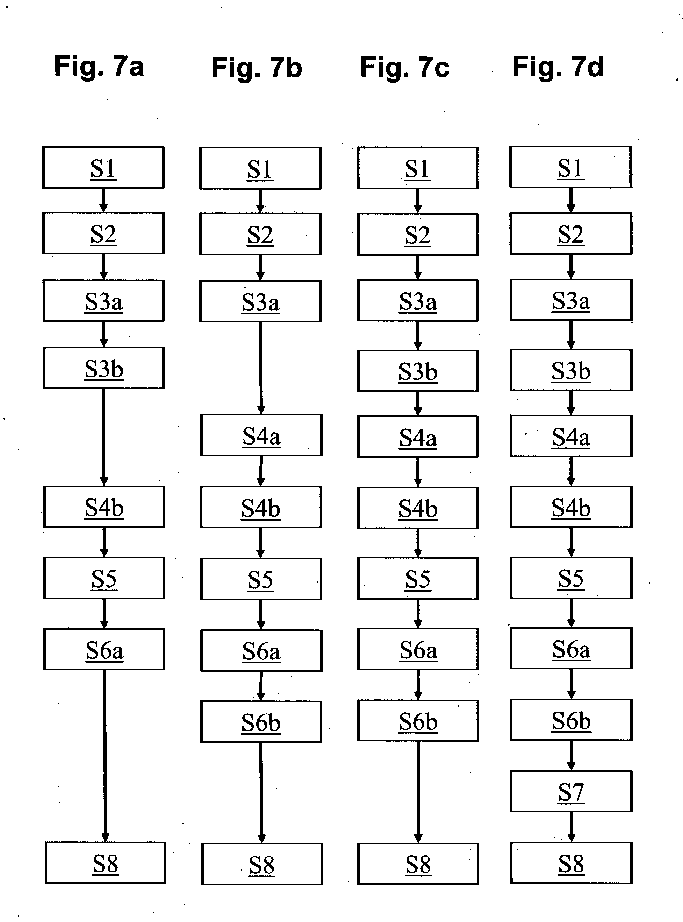

Fig. 7 (a-d) zeigt schematisch verschiedene bevorzugte Ausführungsformen des erfindungsgemäßen Verfahrens nach Anspruch 1 in Kombination mit ausgewählten abhängigen Unteransprüchen. Die Verfahrensschritte werden bevorzugt in dargestellter sequenzieller Reihenfolge durchgeführt. Es sind nicht sämtliche mögliche Kombination dargestellt. Die restlichen Kombinationen erschließen sich jedoch dem Fachmann ohne Schwierigkeiten.

Fig. 7a zeigt eine bevorzugte Ausführungsform des Verfahrens, in dem folgende Schritte durchgeführt werden: Probenbereitstellungsschritt A S1, Kultivierungsschritt S2, Flüssigkeitsentfernungsschritt S3a, Waschschritt S3b, Messschritt A S4b, erster Auswerteschritt A S5, zweiter Auswerteschritt A S6a, vierter Auswerteschritt A S8. Das Überspringen des dritten Auswerteschrittes A S7 vereinfacht das Verfahren. Beim Waschschritt S3b wird der massenspektrometrische Probenträger 6 mit einer Waschlösung, zum Beispiel Puffer, gespült. Der zusätzliche Waschschritt S3b nach der Kultivierung verringert nicht-zellspezifische massenspektrometrische Signale 55, Hintergrundsignale und Hintergrundrauschen 58, welche zum Beispiel durch das Nährmedium, ausgetretenen Zellinhalt beschädigter tierischer Zellen 23 oder sonstige Bestandteile der massenspektrometrischen Probe 11 generiert werden. Des Weiteren werden nicht adhärierte tierische Zellen 23, welche falsch positive Zellpräsenzwerte 43 erzeugen und das Ergebnis verfälschen könnten, durch das Waschen noch zuverlässiger entfernt. Insbesondere ist der Waschschritt S3b somit zu empfehlen, wenn das Verfahren im Probenbereitstellungsschritt S1 bei Bereitstellung der tierischen Zellen 23 die tierischen Zellen 23 in suspendierter Form aufgetragen werden und nicht bereits auf der Probenstelle 16 vorliegen. Die zytotoxische Wirkung 46 wird im vierten Auswerteschritt A S8 aus dem Bedeckungsgrad 45 abgeleitet. Diese bevorzugte Ausführungsform des Verfahrens wird für nicht-matrix-basierte ortsauflösenden Massenspektrometern 1 verwendet. Eine ortspezifische Auftragung einer Matrix 29 findet nicht statt.

Fig. 7b zeigt eine bevorzugte Ausführungsform des Verfahrens, in dem folgende Schritte durchgeführt werden: Probenbereitstellungsschritt A S1, Kultivierungsschritt S2, Flüssigkeitsentfernungsschritt S3a, Probenpräparationsschritt A S4a, Messschritt A S4b, erster Auswerteschritt A S5, zweiter Auswerteschritt A S6a, Auswertezwischenschritt A S6b, vierter Auswerteschritt A S8. Ebenso kann der Auswertezwischenschritt A S6b bei Verfahren mit nicht-matrix-basierten ortsauflösenden Massenspektrometern 21 verwendet werden. Dieses Verfahren wird bevorzugt bei matrix-basierten ortsauflösenden Massenspektrometern 21 mit einer Referenzprobe durchgeführt. Der Auswertezwischenschritt A S6b wird durchgeführt, wenn eine Referenzprobe mitgeführt wird. Dadurch kann auf einfache und zuverlässige Weise eine Beurteilung der zytotoxischen Wirkung 46 durchgeführt werden. Die Referenzprobe kann auch an anderer Stelle des Verfahrens in die Berechnungen miteinfließen. Im Auswertezwischenschritt A S6b wird ein Vergleich des Bedeckungsgrades 45 der Probenstelle 16 der Referenzprobe mit dem Bedeckungsgrad 45 der Probenstelle 16 der massenspektrometrischen Probe 11 umfassend die analytische Probe durchgeführt. Dazu wird der Bedeckungsgrad 45 der Referenzprobe als 100%-Wert festgesetzt und Bedeckungsgrad 45 der massenspektrometrischen Probe 11 umfassend die analytische Probe dazu ins Verhältnis gesetzt. Die zytotoxische Wirkung 46 wird im vierten Auswerteschritt A S8 aus der dem Vergleich der ermittelten Bedeckungsgrade 45 der massenspektrometrischen Probe und der Referenzprobe abgeleitet. Dazu wird die zytotoxische Wirkung 46 zum Beispiel anhand von Stufen, bei denen die Abstufungen der zytotoxischen Wirkung 46 Wertebereichen der Bedeckungsgrade 45 zugeordnet sind, eingestuft. Typische dem Fachmann bekannte Zwischenberechnungen sind hier impliziert.

Fig. 7c zeigt eine bevorzugte Ausführungsform des Verfahrens, in dem folgende Schritte durchgeführt werden: Probenbereitstellungsschritt A S1, Kultivierungsschritt S2, Flüssigkeitsentfernungsschritt S3a, Waschschritt S3b, Messschritt A S4b, erster Auswerteschritt A S5, zweiter Auswerteschritt A S6a, Auswertezwischenschritt A S6b, vierter Auswerteschritt A S8. Dieses Verfahren wird bevorzugt bei matrix-basierten ortsauflösenden Massenspektrometern 21 mit einer Referenzprobe und bei Auftragung der tierischen Zellen 23 in suspendierter Form im Probenbereitstellungsschritt A S1 durchgeführt. Der Einsatz und die Einbeziehung der Referenzprobe in die Auswertung führen auf einfache Weise zu einer präzisen und zuverlässigen Beurteilung der zytotoxischen Wirkung 46. Der Waschschritt S3b erhöht ebenfalls die Zuverlässigkeit und Präzision des Verfahrens.
Fig. 7d zeigt eine bevorzugte Ausführungsform des Verfahrens, in dem folgende Schritte durchgeführt werden: Probenbereitstellungsschritt A S1, Kultivierungsschritt S2, Flüssigkeitsentfernungsschritt S3a, Waschschritt S3b, Messschritt A S4b, erster Auswerteschritt A S5, zweiter Auswerteschritt A S6a, Auswertezwischenschritt A S6b, dritter Auswerteschritt A S7, vierter Auswerteschritt A S8. Dieses Verfahren wird bevorzugt bei matrix-basierten ortsauflösenden Massenspektrometern 21 mit einer Referenzprobe und bei Auftragung der tierischen Zellen 23 in suspendierter Form im Probenbereitstellungsschritt A S1 durchgeführt, wenn zudem die Proliferationsfähigkeit der tierischen Zellen 23 ermittelt werden soll.

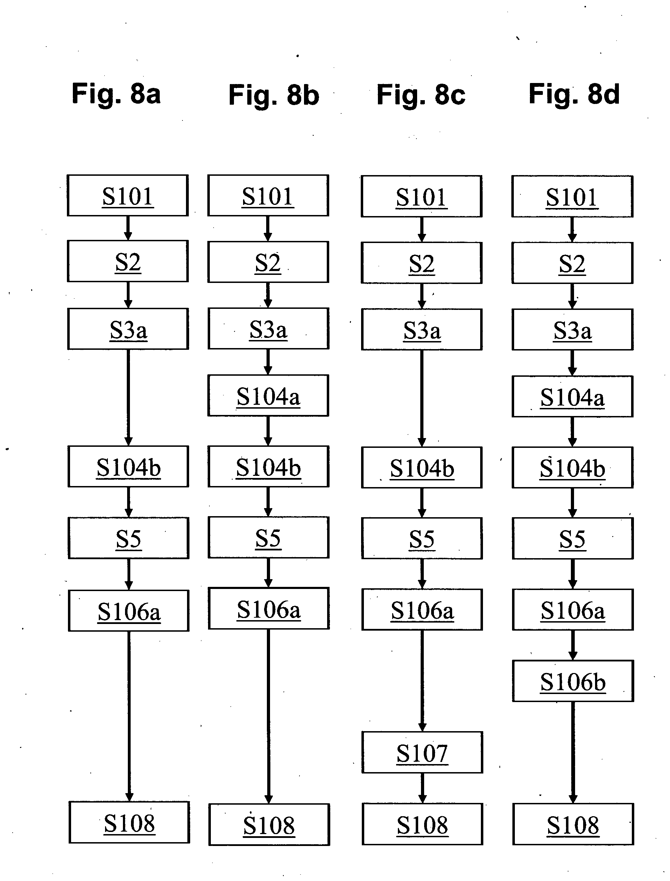

Fig. 8 (a-d) zeigt schematisch bevorzugte Ausführungsformen des erfindungsgemäßen Verfahrens nach Anspruch 3. Vom Verfahren nach Anspruch 1 abweichende Schritte sind mit abweichenden Bezugszeichen und dem Buchstaben B gekennzeichnet. Die Verfahrensschritte sind jedoch in wesentlichen Merkmalen übereinstimmend mit den bereits gezeigten Verfahrensschritten nach Fig. 3. Die Verfahrensschritte werden bevorzugt in dargestellter sequenzieller Reihenfolge ausgeführt.

So zeigt Fig. 8a eine besonders bevorzugte Ausführungsform des Verfahrens für nicht-matrix-basierte ortsauflösendem Massenspektrometer. In diesem Verfahren werden folgende Schritte durchgeführt: Probenbereitstellungsschritt B S101, Kultivierungsschritt S2, Flüssigkeitsentfernungsschritt S3a, Messschritt B S104b, erster Auswerteschritt A S5, zweiter Auswerteschritt B S106a, vierter Auswerteschritt B S108. Eine ortspezifische Auftragung einer Matrix 29 findet nicht statt. Die zytotoxische Wirkung 46 wird im vierten Auswerteschritt B S108 aus der Zell-Ausdehnungsweite 53 abgeleitet. Typische dem Fachmann bekannte Zwischenberechnungen sind hier impliziert. Detailbeschreibungen der Verfahrensschritte sind der Figurenbeschreibung zu Fig. 8b zu entnehmen.

Das Verfahren weist somit folgende Merkmale auf:
Verfahren zur Bestimmung der zytotoxischen Wirkung 56 einer analytischen Probe auf tierische Zellen 23 umfassend folgende Schritte:
- einen Probenbereitstellungsschritt B S101, in dem eine massenspektrometrischen Probe 11 umfassend tierische Zellen 23, Nährmedium und die analytischen Probe, welche potentiell einen zytotoxischen Faktor aufweist, auf mindestens einer Probenstelle 16 eines massenspektrometrischen Probenträgers 6 bereitgestellt wird, wobei die Probenstelle 16 eine erste und eine zweite Teilfläche 47, 48 aufweist, wobei die tierischen Zellen 23 auf der ersten Teilfläche 47 der Probenstelle 16 bereitgestellt werden und auf der zweiten Teilfläche 48 der Probenstelle 16 keine tierischen Zellen 23 bereitgestellt werden und wobei die restliche massenspektrometrische Probe 11 auf beiden Teilflächen 47, 48 bereitgestellt werden;
- einen Kultivierungsschritt S2, in dem die massenspektrometrische Probe 11 auf der Probenstelle 16 des massenspektrometrischen Probenträgers 6 in einer Kultivierungsvorrichtung 24 inkubiert wird;
- einen Flüssigkeitsentfernungsschritt S3a, in dem Restflüssigkeiten der massenspektrometrischen Probe 11 auf der Probenstelle 16 entfernt werden;
- einem Messschritt B S104b, in dem ortsaufgelöste Massenspektren 38 an mindestens zwei Messpositionen 56,57 der Probenstelle 16 mittels eines nicht-matrix-basierten ortsauflösendem Massenspektrometers 1 aufgenommen werden, wobei die erste Messposition 33, 56 noch tierische Zellen 23 aufweist und die zweite Messposition 33, 57 keine tierischen Zellen 23 aufweist,
   wobei sich beide Messpositionen 56, 57 auf einer Erstreckungsrichtung 50 ausgehend von einem Referenzpunkt 51 befinden; .
- einen ersten Auswerteschritt A S5, in dem jedes ortsaufgelösten Massenspektrums 38 hinsichtlich des Vorhandenseins einer zellspezifischen massenspektrometrischen Signatur 41 für die tierische Zellen 23 analysiert wird und jeder Messposition 33, 56, 57 ein Zellpräsenzwert 42 zugewiesen wird;
- einen zweiten Auswerteschritt B S106a, in dem eine Zell-Ausdehnungsweite 53 aus mindestens einer Entfernung von einem Referenzpunkt 51 zu einem Umschlagpunkt 52 ermittelt wird, wobei jeder Umschlagpunkt 52 mindestens aus der ersten und der zweiten Messposition 56, 57 berechnet wird;
- einem vierten Auswerteschritt B S108, in dem die zytotoxische Wirkung 46 der analytischen Probe aus der Zell-Ausdehnungsweite 53 des zweiten Auswerteschrittes B S106a abgeleitet wird.

Fig. 8b zeigt eine weitere besonders bevorzugte Ausführungsform des Verfahrens für matrix-basierte ortsauflösendem Massenspektrometer 21. In diesem Verfahren werden folgende Schritte durchgeführt: Probenbereitstellungsschritt B S101, Kultivierungsschritt S2, Flüssigkeitsentfernungsschritt S3a, Probenpräparationsschritt B S104a, Messschritt B S104b, erster Auswerteschritt A S5, zweiter Auswerteschritt B S106a, vierter Auswerteschritt B S108. Die zytotoxische Wirkung 46 wird im vierten Auswerteschritt B S108 aus der Zell-Ausdehnungsweite 53 abgeleitet. Typische dem Fachmann bekannte Zwischenberechnungen sind hier impliziert.

Das Verfahren weist somit folgende Merkmale auf:
Verfahren zur Bestimmung der zytotoxischen Wirkung 56 einer analytischen Probe auf tierische Zellen 23 umfassend folgende Schritte:
- einen Probenbereitstellungsschritt B S101, in dem eine massenspektrometrischen Probe 11 umfassend tierische Zellen 23, Nährmedium und die analytischen Probe, welche potentiell einen zytotoxischen Faktor aufweist, auf mindestens einer Probenstelle 16 eines massenspektrometrischen Probenträgers 6 bereitgestellt wird, wobei die Probenstelle 16 eine erste und eine zweite Teilfläche 47, 48 aufweist, wobei die tierischen Zellen 23 auf der ersten Teilfläche 47 der Probenstelle 16 bereitgestellt werden und auf der zweiten Teilfläche 48 der Probenstelle 16 keine tierischen Zellen 23 bereitgestellt werden und wobei die restliche massenspektrometrische Probe 11 auf beiden Teilflächen 47, 48 bereitgestellt werden;
- einen Kultivierungsschritt S2, in dem die massenspektrometrische Probe 11 auf der Probenstelle 16 des massenspektrometrischen Probenträgers 6 in einer Kultivierungsvorrichtung 24 inkubiert wird;
- einen Flüssigkeitsentfernungsschritt S3a, in dem Restflüssigkeiten der massenspektrometrischen Probe 11 auf der Probenstelle 16 entfernt werden;
- Probenpräparationsschritt B S104a, in dem eine Präparation der Probenstelle 16 durch ortstreue Auftragung einer Matrix 29 auf mindestens einen Teil beider Teilflächen 47,48 der Probenstelle 48 erfolgt;
- einem Messschritt B S104b, in dem ortsaufgelöste Massenspektren 38 an mindestens zwei Messpositionen 56,57 der Probenstelle 16 mittels eines matrix-basierten ortsauflösendem Massenspektrometers 1 aufgenommen werden, wobei die erste Messposition 33, 56 noch tierische Zellen 23 aufweist und die zweite Messposition 33, 57 keine tierischen Zellen 23 aufweist, wobei sich beide Messpositionen 56, 57 auf einer Erstreckungsrichtung 50 ausgehend von einem Referenzpunkt 51 befinden;
- einen ersten Auswerteschritt A S5, in dem jedes ortsaufgelösten Massenspektrums 38 hinsichtlich des Vorhandenseins einer zellspezifischen massenspektrometrischen Signatur 41 für die tierische Zellen 23 analysiert wird und jeder Messposition 33, 56, 57 ein Zellpräsenzwert 42 zugewiesen wird;
- einen zweiten Auswerteschritt B S106a, in dem eine Zell-Ausdehnungsweite 53 aus mindestens einer Entfernung von einem Referenzpunkt 51 zu einem Umschlagpunkt 52 ermittelt wird, wobei jeder Umschlagpunkt 52 mindestens aus der ersten und der zweiten Messposition 56, 57 berechnet wird;
- einem vierten Auswerteschritt B S108, in dem die zytotoxische Wirkung 46 der analytischen Probe aus der Zell-Ausdehnungsweite 53 des zweiten Auswerteschrittes B S106a abgeleitet wird.

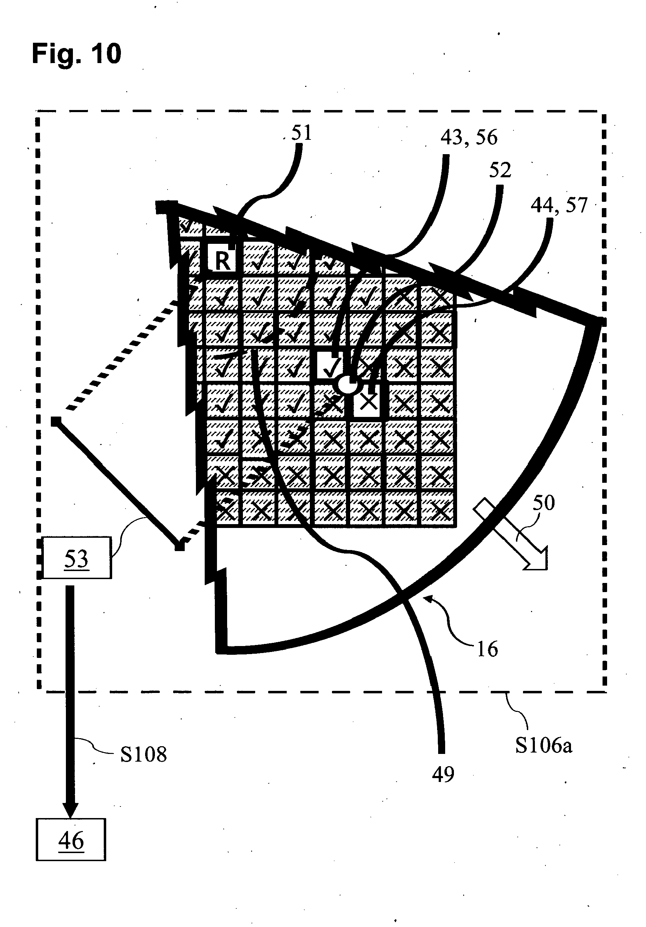

Exemplarisch für die anderen bevorzugten Ausführungsformen nach Anspruch 3 oder Anspruch 20 (z.B. in Fig. 8 a-d) wird diese Ausführungsform hier im Detail beschrieben:
- Probenbereitstellungsschritt B S101: Die tierischen Zellen 23 werden auf der ersten Teilfläche 47 bereitgestellt. Dazu werden die tierische Zellen 23 mit Nährmedium auf der ersten Teilfläche 47 platziert und dort inkubiert. Nachdem die tierischen Zellen 23 auf der ersten Teilfläche 47 angewachsen und adhäriert sind, wird die Restflüssigkeit entfernt. Dabei werden ebenfalls nicht adhärierte tierische Zellen 23 entfernt. Dann erfolgt die Zugabe der restlichen massenspektrometrische Probe 11 ohne tierische Zellen 23. Die restliche massenspektrometrische Probe 11 umfasst unter anderem die analytische Probe, zum Beispiel eine Urinprobe, welche potenziell einen zytotoxischen Faktor enthält, und Nährmedium. Die restliche massenspektrometrische Probe 11 wird auf die gesamte Probenstelle 16, d.h. die erste Teilfläche 47 und die zweite Teilfläche 48, aufgetragen.
- Kultivierungsschritt S2: Die tierischen Zellen 23 werden analog zum Kultivierungsschritt S2 in der Figurenbeschreibung von Fig. 3 kultiviert.
- Flüssigkeitsentfernungsschritt S3a: Die Restflüssigkeiten der massenspektrometrischen Probe 11 auf der Probenstelle 16 werden entfernt. Dieser Schritt erfolgt analog zum Flüssigkeitsentfernungsschritt S3a in der Figurenbeschreibung von Fig. 3. Probenpräparationsschritt B S104a: Die Auftragung erfolgt analog zum Probenpräparationsschritt A 4a in der Figurenbeschreibung von Fig. 3. Dabei wird die Matrix 29 in diesem Beispiel ortstreu auf die gesamte Probenstelle 16 aufgetragen.
- Messschritt B S104b: Es werden ortsaufgelöste Massenspektren 38 an einer Vielzahl von Messpositionen 33 aufgenommen. In einer alternativen bevorzugten Ausgestaltung werden ortsaufgelöste Massenspektren 38 nur an einer Vielzahl von Messpositionen 33 entlang von Erstreckungsrichtungen 50 aufgenommen, an denen die spätere Bestimmung der Zell-Ausdehnungsweite 53 stattfindet. Dies spart unnötige Messungen.
- Erster Auswerteschritt S5: Jedes ortsaugelöste Massenspektrum 38 wird hinsichtlich des Vorhandenseins einer zellspezifischen massenspektrometrischen Signatur 41 für die tierische Zellen 23 analysiert wird und jeder Messposition 33 wird ein Zellpräsenzwert 42 zugewiesen. Dieser erste Auswerteschritt S5 wird analog zu den Figurenbeschreibungen von Fig. 4 und 5 ausgeführt.
- Zweiter Auswerteschritt B S106a: Der Umschlagpunkt 52 entlang einer Erstreckungsrichtung wird ermittelt. Die Entfernung von einem Referenzwert zum Umschlagpunkt ergibt die Zell-Ausdehnungsweite. Weitere Details zum zweiten Auswerteschritt B S106a sind den Ausführungen zu Fig. 10 zu entnehmen.
- Vierter Auswerteschritt B S108: Die zytotoxische Wirkung wird aus der Zell-Ausdehnungsweite 53 abgeleitet, in dem die Zell-Ausdehnungsweite 53 mit Referenzwerten abgeglichen wird. Diese liegen bereits als gespeicherte Daten vor oder werden mithilfe mitlaufendender Referenzproben bestimmt. Anhand der Referenzwerte lässt sich die Zell-Ausdehnungsweite 53 einer zytotoxischen Wirkung 46 zuordnen. Anschließend wird ausgegeben, ob die analytische Probe eine zytotoxische Wirkung hatte. Zudem-wird ausgegeben, wie stark die zytotoxische Wirkung war. Bevorzugt wird im vierten Auswerteschritt B S108 aus der Zell-Ausdehnungsweite 53 die Proliferationsfähigkeit und/oder die Migrationsfähigkeit der tierischen Zellen und/oder die Virulenz der analytischen Probe direkt abgeleitet.

Fig. 8c zeigt eine alternative bevorzugte Ausführungsform des Verfahrens für nicht-matrix-basierte ortsauflösende Massenspektrometer. In diesem Verfahren werden folgende Schritte durchgeführt: Pröbenbereitstellungsschritt B S101, Kultivierungsschritt S2, Flüssigkeitsentfernungsschritt S3a, Messschritt B S104b, erster Auswerteschritt A S5, zweiter Auswerteschritt B S106a, dritter Auswerteschritt B S107, vierter Auswerteschritt B S108. Eine ortspezifische Auftragung einer Matrix 29 findet nicht statt. Die zytotoxische Wirkung 46 wird im vierten Auswerteschritt B S108 aus der Proliferationsfähigkeit und/oder Migrationsfähigkeit abgeleitet. Typische dem Fachmann bekannte Zwischenberechnungen sind hier impliziert.

Das Verfahren weist somit folgende Merkmale auf:
Verfahren zur Bestimmung der zytotoxischen Wirkung 56 einer analytischen Probe auf tierische Zellen 23 umfassend folgende Schritte:
- einen Probenbereitstellungsschritt B S101, in dem eine massenspektrometrischen Probe 11 umfassend tierische Zellen 23, Nährmedium und die analytischen Probe, welche potentiell einen zytotoxischen Faktor aufweist, auf mindestens einer Probenstelle 16 eines massenspektrometrischen Probenträgers 6 bereitgestellt wird, wobei die Probenstelle 16 eine erste und eine zweite Teilfläche 47, 48 aufweist, wobei die tierischen Zellen 23 auf der ersten Teilfläche 47 der Probenstelle 16 bereitgestellt werden und auf der zweiten Teilfläche 48 der Probenstelle 16 keine tierischen Zellen 23 bereitgestellt werden und wobei die restliche massenspektrometrische Probe 11 auf beiden Teilflächen 47, 48 bereitgestellt werden;
- einen Kultivierungsschritt S2, in dem die massenspektrometrische Probe 11 auf der Probenstelle 16 des massenspektrometrischen Probenträgers 6 in einer Kultivierungsvorrichtung 24 inkubiert wird;
- einen Flüssigkeitsentfernungsschritt S3a, in dem Restflüssigkeiten der massenspektrometrischen Probe 11 auf der Probenstelle 16 entfernt werden;
- einem Messschritt B S104b, in dem ortsaufgelöste Massenspektren 38 an mindestens zwei Messpositionen 56,57 der Probenstelle 16 mittels eines nicht-matrix-basierten ortsauflösendem Massenspektrometers 1 aufgenommen werden, wobei die erste Messposition 33, 56 noch tierische Zellen 23 aufweist und die zweite Messposition 33, 57 keine tierischen Zellen 23 aufweist,
   wobei sich beide Messpositionen 56, 57 auf einer Erstreckungsrichtung 50 ausgehend von einem Referenzpunkt 51 befinden;
- einen ersten Auswerteschritt A S5, in dem jedes ortsaufgelösten Massenspektrums 38 hinsichtlich des Vorhandenseins einer zellspezifischen massenspektrometrischen Signatur 41 für die tierische Zellen 23 analysiert wird und jeder Messposition 33, 56, 57 ein Zellpräsenzwert 42 zugewiesen wird;
- einen zweiten Auswerteschritt B S106a, in dem eine Zell-Ausdehnungsweite 53 aus mindestens einer Entfernung von einem Referenzpunkt 51 zu einem Umschlagpunkt 52 ermittelt wird, wobei jeder Umschlagpunkt 52 mindestens aus der ersten und der zweiten Messposition 56, 57 berechnet wird;
- einen dritten Auswerteschritt B S107, in dem eine Proliferationsfähigkeit und/oder eine Migrationsfähigkeit der tierischen Zellen 23 aus der bestimmten Zell-Ausdehnungsweite 53 abgeleitet wird;
- einem viertem Auswerteschritt B S108, in dem die zytotoxische Wirkung 46 der analytischen Probe aus der Proliferationsfähigkeit und/oder der Migrationsfähigkeit des dritten Auswerteschrittes B S107 abgeleitet wird.

Fig. 8d zeigt eine alternative bevorzugte Ausführungsform des Verfahrens für matrix-basierte ortsauflösendem Massenspektrometer 21. In diesem Verfahren werden folgende Schritte durchgeführt: Probenbereitstellungsschritt B S101, Kultivierungsschritt S2, Flüssigkeitsentfernungsschritt S3a, Probenpräparationsschritt B S104a, Messschritt B S104b, erster Auswerteschritt A S5, zweiter Auswerteschritt B S106a, Auswertezwischenschritt B S106b, vierter Auswerteschritt B S108. Im Auswertezwischenschritt B S106b wird ein Vergleich der Zell-Ausdehnungsweite 53 mindestens einer Referenzprobe mit der Zell-Ausdehnungsweite 53 der massenspektrometrischen Probe 11 durchgeführt. Dazu wird die Zell-Ausdehnungsweite 53 der Referenzprobe als 100%-Wert festgesetzt und die Zell-Ausdehnungsweite 53 der massenspektrometrischen Probe 11 dazu ins Verhältnis gesetzt. Die zytotoxische Wirkung 46 wird im vierten Auswerteschritt B S108 aus der dem Vergleich der ermittelten Zell-Ausdehnungsweiten 53 der massenspektrometrischen Probe 11 und der Referenzprobe abgeleitet. Dazu wird die zytotoxische Wirkung anhand von Stufen, bei denen die Abstufungen der zytotoxischen Wirkung Wertebereichen der Zell-Ausdehnungsweite 53 zugeordnet sind, eingestuft. Typische dem Fachmann bekannte Zwischenberechnungen sind hier impliziert.

Das Verfahren weist somit folgende Merkmale auf:
Verfahren zur Bestimmung der zytotoxischen Wirkung 56 einer analytischen Probe auf tierische Zellen 23 umfassend folgende Schritte:
- einen Probenbereitstellungsschritt B S101, in dem eine massenspektrometrischen Probe 11 umfassend tierische Zellen 23, Nährmedium und die analytischen Probe, welche potentiell einen zytotoxischen Faktor aufweist, auf mindestens einer Probenstelle 16 eines massenspektrometrischen Probenträgers 6 bereitgestellt wird, wobei die Probenstelle 16 eine erste und eine zweite Teilfläche 47, 48 aufweist, wobei die tierischen Zellen 23 auf der ersten Teilfläche 47 der Probenstelle 16 bereitgestellt werden und auf der zweiten Teilfläche 48 der Probenstelle 16 keine tierischen Zellen 23 bereitgestellt werden und wobei die restliche massenspektrometrische Probe 11 auf beiden Teilflächen 47, 48 bereitgestellt werden;
- einen Kultivierungsschritt S2, in dem die massenspektrometrische Probe 11 auf der Probenstelle 16 des massenspektrometrischen Probenträgers 6 in einer Kultivierungsvorrichtung 24 inkubiert wird;
- einen Flüssigkeitsentfernungsschritt S3a, in dem Restflüssigkeiten der massenspektrometrischen Probe 11 auf der Probenstelle 16 entfernt werden;
- Probenpräparationsschritt B S104a, in dem eine Präparation der Probenstelle 16 durch ortstreue Auftragung einer Matrix 29 auf mindestens einen Teil beider Teilflächen 47,48 der Probenstelle 48 erfolgt;
- einem Messschritt B S104b, in dem ortsaufgelöste Massenspektren 38 an mindestens zwei Messpositionen 56,57 der Probenstelle 16 mittels eines matrix-basierten ortsauflösendem Massenspektrometers 1 aufgenommen werden, wobei die erste Messposition 33, 56 noch tierische Zellen 23 aufweist und die zweite Messposition 33, 57 keine tierischen Zellen 23 aufweist,
   wobei sich beide Messpositionen 56, 57 auf einer Erstreckungsrichtung 50 ausgehend von einem Referenzpunkt 51 befinden;
- einen ersten Auswerteschritt A S5, in dem jedes ortsaufgelösten Massenspektrums 38 hinsichtlich des Vorhandenseins einer zellspezifischen massenspektrometrischen Signatur 41 für die tierische Zellen 23 analysiert wird und jeder Messposition 33, 56, 57 ein Zellpräsenzwert 42 zugewiesen wird;
- einen zweiten Auswerteschritt B S106a, in dem eine Zell-Ausdehnungsweite 53 aus mindestens einer Entfernung von einem Referenzpunkt 51 zu einem Umschlagpunkt 52 ermittelt wird, wobei jeder Umschlagpunkt 52 mindestens aus der ersten und der zweiten Messposition 56, 57 berechnet wird;
- einen Auswertezwischenschritt B S106b, in dem ein Vergleich der Zell-Ausdehnungsweite 53 mindestens einer Referenzprobe mit der Zell-Ausdehnungsweite 53 der massenspektrometrischen Probe 11 umfassend die analytische Probe durchgeführt wird und in die Ableitung der zytotoxischen Wirkung 46 im vierten Auswerteschritt B S108 einbezogen wird;
- einem vierten Auswerteschritt B S108, in dem die zytotoxische Wirkung 46 der analytischen Probe aus der Zell-Ausdehnungsweite 53 der analytischen Probe des zweiten Auswerteschrittes B S106a unter Einbeziehung des Vergleichs aus dem Auswertezwischenschritt B S106b abgeleitet wird.

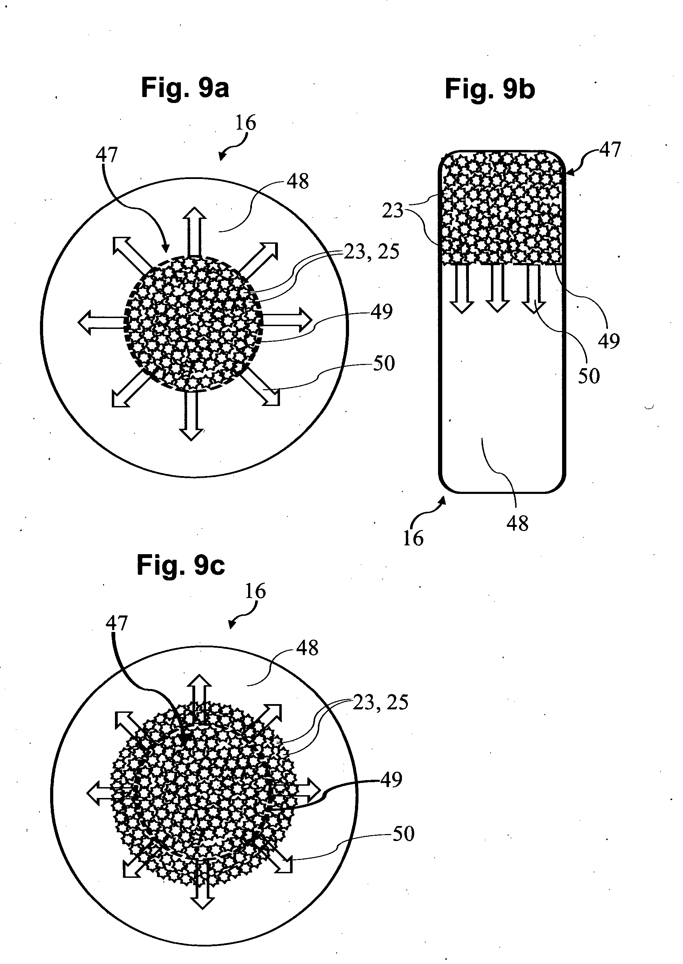

Fig. 9 zeigt schematisch bevorzugte Ausführungsformen der Probenstellen 16 nach Anspruch 3. So zeigt Fig. 9a eine kreisrunde Probenstelle 16 mit einer ersten Teilfläche 47 und einer zweiten Teilfläche 48 im Probenbereitstellungsschritt B S101. Auf der ersten Teilfläche 47 sind bereits die tierischen Zellen 23 bereitgestellt. Zu Verdeutlichungszwecken ist die erste Teilfläche 47 mit einer imaginären unterbrochenen Grenzlinie 49 von der zweiten Teilfläche 48 abgegrenzt. Die zweite Teilfläche 48 ist frei von tierischen Zellen 23. Die Ausdehnung der tierischen Zellen 23 auf die zweite Teilfläche 48 im folgenden Kultivierungsschritt S2 erfolgt bevorzugt in den Erstreckungsrichtungen 50. Die Erstreckungsrichtungen 50 erstrecken sich bevorzugt orthogonal zur Grenzlinie 49. Fig. 9b zeigt eine alternative Ausführungsform der Probenstellen 16 nach Anspruch 3 im Probenbereitstellungsschritt B S101. Die Probenstelle 16 ist länglich ausgebildet. Sie weist ebenfalls eine erste Teilfläche 47 und eine zweite Teilfläche 48 auf. Zu Verdeutlichungszwecken ist die erste Teilfläche 47 mit einer imaginären unterbrochenen Grenzlinie 49 von der zweiten Teilfläche 48 abgegrenzt. Auf der ersten Teilfläche 47 sind bereits die tierischen Zellen 23 bereitgestellt. Hier wird die Zell-Ausdehnungsweite 53 im späteren zweiten Auswerteschritt B S106a in lediglich einer Erstreckungsrichtung 50 an verschiedenen Positionen der Grenzlinie 49 orthogonal zur Grenzlinie 49 bestimmt.

Weitere Ausführungsformen der Probenstellen 16 sind im Rahmen der Erfindung ebenfalls umfasst. So sind weitere Formen der Probenstelle 16 eingeschlossen. Ebenfalls ist die Ausführungsform umfasst, in der die Probenstelle 16 kreisrund ausgebildet ist und die ersten Teilfläche 47 und die zweite Teilfläche 48 derart ausgebildet sind, dass die angrenzenden Seiten der beiden Teilflächen 47,48 eine imaginäre Grenzlinie 49 bilden, welche gerade ist.

Fig. 9c zeigt schematisch die Probenstelle 16 aus Fig. 9a nach dem Kultivierungsschritt S2, in dem eine Proliferation stattfand. Die tierischen Zellen 23 haben sich von der ersten Teilfläche 47 aus über den Grenzlinie 49 hinaus auf die zweite Teilfläche 48 ausgebreitet.

Die Zell-Ausdehnungsweite 53 im späteren zweiten Auswerteschritt B S106a wird in den divergierenden Erstreckungsrichtung 50 bestimmt.

Fig. 10 zeigt schematisch den zweiten Auswerteschritt B S106a und den vierten Auswerteschritt B S108 des Verfahrens nach Anspruch 3 wie es zum Beispiel entsprechend der Fig. 8 (a-d) und entsprechend Fig. 9c im Detail abgebildet worden ist. Insbesondere wird die Berechnung eines Umschlagpunktes 52 und die Ermittlung einer Zell-Ausdehnungsweite 53 gezeigt.

In diesem Beispiel beginnen die Erstreckungsrichtungen 50 am gleichen Referenzpunkt 51. Der Referenzpunkt 51 liegt in diesem Beispiel im Zentrum der ersten Teilfläche 47. Zur Ermittlung eines Umschlagpunkt 52 auf einer Erstreckungsrichtung 50 werden ausgehend von einem Referenzpunkt 51 aus die Zellpräsenzwerte 42 überprüft. Dazu wird entlang der Erstreckungsrichtung 50 eine Änderung von einem positiven Zellpräsenzwert 43 zu einem negativen Zellpräsenzwert 44 an zwei in Erstreckungsrichtung 50 benachbarten Messpositionen 33, 56, 57 ermittelt. Somit befinden sich in Erstreckungsrichtung 50 vom Referenzpunkt 51 ausgehend vor dem Umschlagpunkt 52 auf weitgehend jeder Messposition 33 tierische Zellen 23 und nach dem Umschlagpunkt 52 weitgehend keine tierischen Zellen 23. Der Umschlagpunkt 52 wird somit in Erstreckungsrichtung 50 aus einer ersten und einer zweiten Messposition 56, 57 mit jeweils verschiedenen Zellpräsenzwerten 42 berechnet, einem positiven Zellpräsenzwert 43 und einen negativen Zellpräsenzwert 44. Der Umschlagpunkt 52 liegt bevorzugt mittig zwischen beiden Messpositionen 56, 57. Der Abstand vom Referenzpunkt 51 zum Umschlagspunk 52 ergibt die Zell-Ausdehnungsweite 53. Diese wird bevorzugt für mehrere Erstreckungsrichtungen 50 ermittelt. Anschließend folgt eine Mittelung der berechneten Zell-Ausdehnungsweiten 53 einer Probenstelle 16. Aus der gemittelten Zell-Ausdehnungsweite 53 wird die zytotoxische Wirkung 46 der analytischen Probe 16 abgeleitet.

Die zytotoxische Wirkung 46 der analytischen Probe wird beispielsweise wie folgt abgestuft:
(i) Keine zytotoxische Wirkung 46, wobei die Zell-Ausdehnungsweite 53 der massenspektrometrischen Probe 11 umfassend die analytische Probe der Zell-Ausdehnungsweite 53 einer Referenzprobe ohne zytotoxischen Faktor entspricht. Die tierischen Zellen 23 konnten ungehindert wachsen und sich teilen;
(ii) starke zytotoxische Wirkung 56, wobei keine tierischen Zellen 23 auf der Probenstelle 16 oder keine tierischen Zellen 23 auf der zweiten Teilfläche 48 der Probenstelle 16 festgestellt werden konnten. Es gab kein Wachstum oder Zellteilung der tierischen Zellen 23. Die Zell-Ausdehnungsweite 53 ist weitgehend 0. Alternativ haben sich die tierischen Zellen 23 von der ersten Teilfläche 47 abgelöst, weshalb keine erste Messposition 56 am ursprünglichen Rand der ersten Teilfläche 47 feststellbar ist;
(iii) Mittelstarke zytotoxische Wirkung 46, wobei eine Zell-Ausdehnungsweite 53 kleiner 50% der Referenzprobe festgestellt werden konnte;
(iv) schwache zytotoxische Wirkung 46, wobei eine Zell-Ausdehnungsweite 53 größer 50% der Referenzprobe festgestellt werden konnte.

Fig. 11 zeigt beispielhaft Massenspektren 20 verschiedener massenspektrometrischer Proben 11 mit zellspezifischen massenspektrometrischen Signalen 40, nicht-zellspezifischen massenspektrometrischen Signalen 55 und zellspezifischen massenspektrometrischen Signaturen 41. Das verwendete Massenspektrometer 101 war ein nicht-ortsauflösendes MALDI-ToF. Auch erfolgte der Auftrag der Matrix 29 nicht ortstreu. Somit stellen die Massenspektren 20 Summenspektren einer ganzen Probenstelle 16 dar und sind nicht erfindungsgemäß erzeugt worden. Zudem sind die Massenspektren 20 nicht ortsaugelöst. Es soll hier unter anderem zur Verdeutlichung der zellspezifischen massenspektrometrischen Signale 40, der zellspezifischen massenspektrometrischen Signaturen 41, der nicht-zellspezifischen massenspektrometrischen Signalen 55 und einer möglichen Ermittlung von Referenzwerten dienen. Dabei ist insbesondere das Massenspektrum 20 in Fig. 11 (b) hier nur näherungsweise ein Beispiel, da hier eine kleine Restanzahl an tierischen Zellen 23, welche bereits vor dem Kultivierungsschritt S2 vorlagen (cell seed), weitgehend schwache zellspezifische massenspektrometrische Signale 40 erzeugen können.

In Fig. 11 (a) ist ein Massenspektrum 20 einer ersten Referenzprobe umfassend tierische Zellen 23 und Nährmedium dargestellt. Die tierischen Zellen 23 sind Zellen der humanen kontinuierlichen Zelllinie A549. Als Nährmedium für die Kultivierung wurde das komplexe Nährmedium DMEN mit 10% fötalem Kälberserum (FCS) verwendet. Die Kultivierung der tierischen Zellen 23 erfolgte für 24 h. Ein zytotoxischer Faktor war nicht vorhanden. Das Massenspektrum 20 umfasst massenspektrometrische Signale 39, die durch die tierischen Zellen 23, das Nährmedium, den zytotoxischen Faktor und die Matrix 29 erzeugt worden sind. Es umfasst zellspezifische massenspektrometrische Signale 40, nicht-zellspezifische massenspektrometrische Signale 55 und eine ausgewählte zellspezifische massenspektrometrische Signatur 41.

In Fig. 11 (b) ist ein Massenspektrum 20 einer zweiten Referenzprobe (respektive einer massenspektrometrischen Probe umfassend eine analytische Probe mit einem zytotoxischen Faktor) umfassend tierische Zellen 23, Nährmedium und einem zytotoxischen Faktor dargestellt. Die tierischen Zellen 23 sind ebenfalls Zellen der humanen kontinuierlichen Zelllinie A549. Als Nährmedium für die Kultivierung wurde ebenso das komplexe Nährmedium DMEN mit 10% fötalem Kälberserum (FCS) verwendet. Der zytotoxische Faktor ist Tetradecyltrimethylammoniumchlorid. Die Kultivierung der tierischen Zellen 23 erfolgte für 24 h. Das Massenspektrum 20 umfasst nicht-zellspezifische massenspektrometrische Signale 55, die im Wesentlichen durch das Nährmedium, den zytotoxischen Faktor und die Matrix 29 erzeugt worden sind. Aufgrund der Anwesenheit eines zytotoxischen Faktors während des Kultivierungsschrittes S2 erfolgte keine Proliferation der tierischen Zellen 23. Markante zellspezifische massenspektrometrische Signale 40 sind nicht vorhanden. Da es sich in diesem Beispiel um ein nicht erfindungsgemäßes Summen-Massenspektrum der gesamten Probenstelle 16 handelt, sind ggfs. vereinzelt schwache zellspezifische massenspektrometrische Signale 40 in diesem Beispiel zu erkennen, welche bei Nutzung eines ortsauflösenden Massenspektrometers 1 weitgehend fehlen. Diese schwachen zellspezifischen massenspektrometrische Signale 40 sind der geringen Anzahl an zu Beginn bereitgestellten tierischen Zellen 23 zuzuordnen (cell seed).

Des Weiteren können schwache und in der Anzahl geringe massenspektrometrische Signale 39 durch von tierischen Zellen 23 ausgeschiedene Proteine, durch Zellbestandteile zerstörter tierischer Zellen 23 oder durch Stoffwechselprodukte erzeugt werden, welche sich während des Kultivierungsschrittes S2 teilweise über einzelne Messpositionen 33 einer Probenstelle 16 verteilen. Diese massenspektrometrische Signale 39 sind in der Regel jedoch ebenfalls von geringer Intensität und können gegebenenfalls durch einen optionalen Waschschritt S3B vor ortstreuer Auftragung der Matrix 29 auf einfache Weise weitgehend entfernt werden. Bevorzugt wird jedoch eine massenspektrometrische Signatur 41 gewählt, welche von solchen Störeinflüssen weitgehend unbeeinflusst ist. Diese massenspektrometrische Signatur 41 kann zum Beispiel mit entsprechenden Referenzproben ermittelt werden. Sie kann zum Beispiel auch mit Hilfe eines Summen-Massenspektrums der gesamten Probenstelle 16 eines nicht-ortsauflösendes MALDI-ToFs wie in Fig. 11 (b) erfolgen. Diese Daten können in einer Datenbank der Datenverarbeitungseinheit 37 zuvor gespeichert und dem Verfahren zur Verfügung gestellt sein.

In Fig. 11 (c) ist ein Massenspektrum 20 einer dritten Referenzprobe umfassend Nährmedium und einen internen Standard, wobei die dritte Referenzprobe keine tierischen Zellen 23 und keinen zytotoxischen Faktor umfasst, dargestellt. Das Nährmedium und die Matrix 29 entsprechen denen aus Fig. 11 (a) und (b). Zellspezifische massenspektrometrische Signale 40 sind nicht vorhanden.

Aus dem Vergleich der Massenspektren 20 der ersten Referenzprobe (a) mit der zweiten und dritten Referenzprobe in (b) und (c) sind die massenspektrometrischen Signale 39 den tierischen Zellen 23, dem Nährmedium und der Matrix 29 zuordenbar. Die ausgewählte zellspezifische massenspektrometrische Signatur 41 in Fig.11 (a) weist zellspezifischen massenspektrometrischen Signale 40 auf. Diese ausgewiesenen zellspezifischen massenspektrometrischen Signale 40 sind markant. So weisen die zellspezifischen massenspektrometrischen Signale 40 der zellspezifischen massenspektrometrischen Signatur 41 insbesondere einen hohen Intensitätsunterschied gegenüber einer Vielzahl nicht-zellspezifischen massenspektrometrischen Signalen 55, dem Hintergrundrauschen 58 und zu den Intensitäten in diesem Massenbereich der zweiten Referenzprobe (b) und der dritten Referenzprobe (c) auf.

Insbesondere stellt der hier beschriebene Vergleich von Massenspektren 20 von Referenzproben in Gänze oder in Teilen eine bevorzugte Option dar, die zellspezifischen massenspektrometrischen Signale 40 und die zellspezifischen massenspektrometrischen Signaturen 41 zu bestimmen. Weitere Vergleiche mit anderen Referenzproben sind hiermit explizit mitumfasst. Insbesondere kann die Bestimmung von zellspezifischen massenspektrometrischen Signalen 40 und von zellspezifischen massenspektrometrischen Signaturen 41 mittels Abgleichs von in einer Datenbank gespeicherten Massenspektren 20, insbesondere von Referenzproben, und/oder davon abgeleiteten Daten durchgeführt werden. Weitere Verfahren zur Bestimmung der zellspezifischen massenspektrometrischen Signale 40, insbesondere von markanten zellspezifischen massenspektrometrischen Signalen 40, sind dem Fachmann hinlänglich bekannt. So kann das Verfahren zum Beispiel eine Merkmalsfindung (feature detection), eine Merkmalsauswahl (feature selection) und /oder ein Klassifikator-Training umfassen. Zum Klassifikator-Training können zum Beispiel univariate Datenanalyseverfahren und/oder multivariate Datenanalyseverfahren verwendet werden. Univariate Datenanalyseverfahren umfassen zum Beispiel Receiver-Operator-Charakteristik, t-test, Kruskal-Wallis-test und/oder ANOVA. Multivariate Datenanalyseverfahren umfassen zum Beispiel Lineare-Diskriminanzanalyse (LDA), Random-Forest-Verfahren, Entscheidungsbäume, Hauptkomponentenanalyse (PCA), hierarchische und andere Clusterverfahren, Mustererkennung, neuronale Netze und/oder SVM.

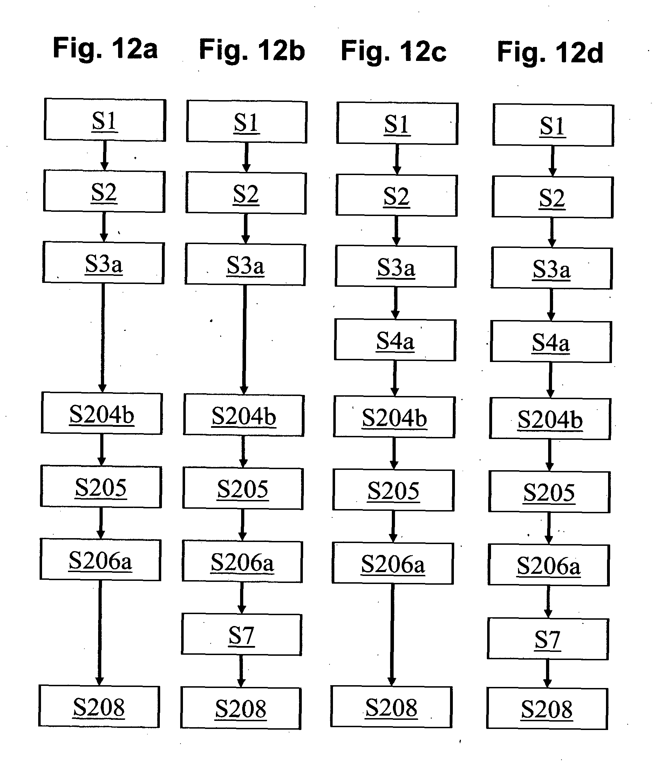

Fig.12 zeigt schematisch verschiedene bevorzugte Ausführungsformen mit den einzelnen Schritten des erfindungsgemäßen Verfahrens nach Anspruch 2. Vom Verfahren nach Anspruch 1 und Anspruch 3 abweichende Schritte sind mit abweichenden Bezugszeichen und dem Buchstaben B gekennzeichnet. Die Verfahrensschritte sind jedoch in wesentlichen Merkmalen übereinstimmend mit den bereits gezeigten Verfahrensschritten nach Fig. 3. Somit sind verschiedene Details zu den einzelnen Schritten trotz abweichender Kennung (A,B,C) den Beispielen zum Verfahren nach Anspruch 1 entnehmbar. Die Verfahrensschritte werden bevorzugt in dargestellter sequenzieller Reihenfolge durchgeführt.

So zeigt Fig. 12a eine besonders bevorzugte Ausführungsform des Verfahrens, in dem folgenden Schritte durchgeführt werden: Probenbereitstellungsschritt A S1, Kultivierungsschritt S2, Flüssigkeitsentfernungsschritt S3a, Messschritt C S204b, erster Auswerteschritt C S205, zweiter Auswerteschritt C S206a, vierter Auswerteschritt C S208. Die zytotoxische Wirkung 46 wird im vierten Auswerteschritt C S208 unmittelbar aus dem Bedeckungsgrad 45 abgeleitet. Typische dem Fachmann bekannte Zwischenberechnungen sind im Rahmen der unmittelbaren Ableitung jedoch nicht ausgeschlossen. Diese bevorzugte Ausführungsform des Verfahrens wird für nicht-matrix-basierte Massenspektrometer verwendet. Eine ortspezifische Auftragung einer Matrix 29 findet nicht statt. Das Verfahren weist somit folgende Merkmale auf:
Verfahren zur Bestimmung der zytotoxischen Wirkung 46 einer analytischen Probe auf tierische Zellen 23, umfassend folgende Schritte:
- einen Probenbereitstellungsschritt A S1, in dem eine massenspektrometrischen Probe 11, umfassend tierische Zellen 23, Nährmedium und die analytische Probe, welche potenziell einen zytotoxischen Faktor aufweist, auf mindestens einer Probenstelle 16 eines massenspektrometrischen Probenträgers 6 bereitgestellt wird;
- einen Kultivierungsschritt S2, in dem die massenspektrometrische Probe 11 auf der Probenstelle 16 des massenspektrometrischen Probenträgers 6 in einer Kultivierungsvorrichtung 24 inkubiert wird;
- einen Flüssigkeitsentfernungsschritt S3a, in dem Restflüssigkeit der massenspektrometrischen Probe 11 von der Probenstelle 16 entfernt wird;
- einen Messschritt C S204b, in dem Massenspektren 38 an einer Vielzahl von Messpositionen 33 verteilt über mindestens eine Teilfläche 34 der Probenstelle 16 mittels eines nicht-matrix-basierten Massenspektrometers aufgenommen werden;
- einen ersten Auswerteschritt C S205, in dem jedes Massenspektrum 20 hinsichtlich des Vorhandenseins einer zellspezifischen massenspektrometrischen Signatur 41 für die tierischen Zellen 23 analysiert wird;
- einen zweiten Auswerteschritt C S206a, in dem ein Bedeckungsgrad 45 durch tierische Zellen 23 für mindestens die Teilfläche 34 der Probenstelle 16 aus dem Ergebnis des ersten Auswerteschrittes C S205c bestimmt wird;
- einen vierten Auswerteschritt C S208, in dem die zytotoxische Wirkung 46 der analytischen Probe auf die tierischen Zellen 23 unmittelbar aus dem bestimmten Bedeckungsgrad 45 des zweiten Auswerteschrittes C S206a abgeleitet wird.

Fig. 12b zeigt eine alternative bevorzugte Ausführungsform des Verfahrens, in dem die folgenden Schritte durchgeführt werden: Probenbereitstellungsschritt A S1, Kultivierungsschritt S2, Flüssigkeitsentfernungsschritt S3a, Messschritt C S204b, erster Auswerteschritt C S205, zweiter Auswerteschritt C S206a, dritter Auswerteschritt A S7, in dem eine Proliferationsfähigkeit aus dem bestimmten Bedeckungsgrades 45 abgeleitet wird, vierter Auswerteschritt CS208. Die zytotoxische Wirkung 19 wird im vierten Auswerteschritt C S208 aus der Proliferationsfähigkeit der tierischen Zellen abgeleitet und somit mittelbar aus dem Bedeckungsgrad 45. Diese bevorzugte, Ausführungsform des Verfahrens wird für nicht-matrix-basierte Massenspektrometer verwendet. Eine ortspezifische Auftragung einer Matrix 29 findet nicht statt.

Das Verfahren weist somit folgende Merkmale auf:
Verfahren zur Bestimmung der zytotoxischen Wirkung 46 einer analytischen Probe auf tierische Zellen 23, umfassend folgende Schritte:
- einen Probenbereitstellungsschritt A S1, in dem eine massenspektrometrischen Probe 11, umfassend tierische Zellen 23, Nährmedium und die analytische Probe, welche potenziell einen zytotoxischen Faktor aufweist, auf mindestens einer Probenstelle 16 eines massenspektrometrischen Probenträgers 6 bereitgestellt wird;
- einen Kultivierungsschritt S2, in dem die massenspektrometrische Probe 11 auf der Probenstelle 16 des massenspektrometrischen Probenträgers 6 in einer Kultivierungsvorrichtung 24 inkubiert wird;
- einen Flüssigkeitsentfernungsschritt S3a, in dem Restflüssigkeit der massenspektrometrischen Probe 11 von der Probenstelle 16 entfernt wird;
- einen Messschritt C S204b, in dem Massenspektren 20 an einer Vielzahl von Messpositionen 33 verteilt über mindestens eine Teilfläche 34 der Probenstelle 16 mittels eines nicht-matrix-basierten Massenspektrometers aufgenommen werden;
- einen ersten Auswerteschritt C S205, in dem jedes Massenspektrum 20 hinsichtlich des Vorhandenseins einer zellspezifischen massenspektrometrischen Signatur 41 für die tierischen Zeiten 23 analysiert wird;
- einen zweiten Auswerteschritt C S206a, in dem ein Bedeckungsgrad 45 durch tierische Zellen 23 für mindestens die Teilfläche 34 der Probenstelle 16 aus dem Ergebnis des ersten Auswerteschrittes C S205c bestimmt wird;
- ein dritten Auswerteschritt A S7, in dem eine Proliferationsfähigkeit aus dem bestimmten Bedeckungsgrades 45 abgeleitet wird;
- einen vierten Auswerteschritt C S208, in dem die zytotoxische Wirkung 46 der analytischen Probe auf die tierischen Zellen 23 aus der bestimmten Proliferationsfähigkeit des dritten Auswerteschrittes A S7 und somit mittelbar aus dem Bedeckungsgrad 45, abgeleitet wird.

Fig. 12c zeigt eine besonders bevorzugte alternative Ausführungsform des Verfahrens, in dem die folgenden Schritte durchgeführt werden: Probenbereitstellungsschritt A S1, Kultivierungsschritt S2, Flüssigkeitsentfernungsschritt S3a, Probenpräparationsschritt S4a, Messschritt C S204b, erster Auswerteschritt C S205, zweiter Auswerteschritt C S206a, vierter Auswerteschritt C S208. Die zytotoxische Wirkung 19 wird im vierten Auswerteschritt C S208 unmittelbar aus dem Bedeckungsgrad 45 abgeleitet. Diese bevorzugte Ausführungsform des Verfahrens wird für matrix-basierte Massenspektrometer verwendet. Vor dem Messschritt C S204b findet im Probenpräparationsschritt A S4a eine ortspezifische Auftragung einer Matrix 29 statt.

Das Verfahren weist somit folgende Merkmale auf:
Verfahren zur Bestimmung der zytotoxischen Wirkung 46 einer analytischen Probe auf tierische Zellen 23, umfassend folgende Schritte:
- einen Probenbereitstellungsschritt A S1, in dem eine massenspektrometrischen Probe 11, umfassend tierische Zellen 23, Nährmedium und die analytische Probe, welche potenziell einen zytotoxischen Faktor aufweist, auf mindestens einer Probenstelle 16 eines massenspektrometrischen Probenträgers 6 bereitgestellt wird;
- einen Kultivierungsschritt S2, in dem die massenspektrometrische Probe 11 auf der Probenstelle 16 des massenspektrometrischen Probenträgers 6 in einer Kultivierungsvorrichtung 24 inkubiert wird;
- einen Flüssigkeitsentfernungsschritt S3a, in dem Restflüssigkeit der massenspektrometrischen Probe 11 von der Probenstelle 16 entfernt wird;
- Probenpräparationsschritt A S4a, in dem eine Präparation der Probenstelle 16 durch ortstreue Auftragung einer Matrix 29 auf mindestens die eine Teilfläche 34 der Probenstelle 16 erfolgt;
- einen Messschritt C S204b, in dem Massenspektren 20 an einer Vielzahl von Messpositionen 33 verteilt über mindestens eine Teilfläche 34 der Probenstelle 16 mittels eines matrix-basierten Massenspektrometers aufgenommen werden;
- einen ersten Auswerteschritt C S205, in dem jedes Massenspektrum 20 hinsichtlich des Vorhandenseins einer zellspezifischen massenspektrometrischen Signatur 41 für die tierischen Zellen 23 analysiert wird;
- einen zweiten Auswerteschritt C S206a, in dem ein Bedeckungsgrad 45 durch tierische Zellen 23 für mindestens die Teilfläche 34 der Probenstelle 16 aus dem Ergebnis des ersten Auswerteschrittes C S205c bestimmt wird;
- ein dritten Auswerteschritt A S7, in dem eine Proliferationsfähigkeit aus dem bestimmten Bedeckungsgrades 45 abgeleitet wird;
- einen vierten Auswerteschritt C S208, in dem die zytotoxische Wirkung 46 der analytischen Probe auf die tierischen Zellen 23 unmittelbar aus dem bestimmten Bedeckungsgrad 45 des zweiten Auswerteschrittes C S206a abgeleitet wird.

Fig. 12d zeigt eine bevorzugte alternative Ausführungsform des Verfahrens, in dem die folgenden Schritte durchgeführt werden: Probenbereitstellungsschritt A S1, Kultivierungsschritt S2, Flüssigkeitsentfernungsschritt S3a, Probenpräparationsschritt A S4a, Messschritt C S204b, erster Auswerteschritt C S205, zweiter Auswerteschritt C S206a, dritter Auswerteschritt A S7, vierter Auswerteschritt C S208. Die zytotoxische Wirkung 19 wird im vierten Auswerteschritt C S208 aus der Proliferationsfähigkeit der tierischen Zellen abgeleitet und somit mittelbar aus dem Bedeckungsgrad 45. Diese bevorzugte Ausführungsform des Verfahrens wird für matrix-basierte Massenspektrometer verwendet. Vor dem Messschritt C S204b findet im Probenpräparationsschritt A S4a eine ortspezifische Auftragung einer Matrix 29 statt.

Das Verfahren weist somit folgende Merkmale auf:
Verfahren zur Bestimmung der zytotoxischen Wirkung 46 einer analytischen Probe auf tierische Zellen 23, umfassend folgende Schritte:
- einen Probenbereitstellungsschritt A S1, in dem eine massenspektrometrischen Probe 11, umfassend tierische Zellen 23, Nährmedium und die analytische Probe, welche potenziell einen zytotoxischen Faktor aufweist, auf mindestens einer Probenstelle 16 eines massenspektrometrischen Probenträgers 6 bereitgestellt wird;
- einen Kultivierungsschritt S2, in dem die massenspektrometrische Probe 11 auf der Probenstelle 16 des massenspektrometrischen Probenträgers 6 in einer Kultivierungsvorrichtung 24 inkubiert wird;
- einen Flüssigkeitsentfernungsschritt S3a, in dem Restflüssigkeit der massenspektrometrischen Probe 11 von der Probenstelle 16 entfernt wird;
- Probenpräparationsschritt A S4a, in dem eine Präparation der Probenstelle 16 durch ortstreue Auftragung einer Matrix 29 auf mindestens die eine Teilfläche 34 der Probenstelle 16 erfolgt;
- einen Messschritt C S204b, in dem Massenspektren 20 an einer Vielzahl von Messpositionen 33 verteilt über mindestens eine Teilfläche 34 der Probenstelle 16 mittels eines matrix-basierten Massenspektrometers aufgenommen werden;
- einen ersten Auswerteschritt C S205, in dem jedes Massenspektrum 20 hinsichtlich des Vorhandenseins einer zellspezifischen massenspektrometrischen Signatur 41 für die tierischen Zellen 23 analysiert wird;
- einen zweiten Auswerteschritt C S206a, in dem ein Bedeckungsgrad 45 durch tierische Zellen 23 für mindestens die Teilfläche 34 der Probenstelle 16 aus dem Ergebnis des ersten Auswerteschrittes C S205c bestimmt wird;
- ein dritten Auswerteschritt A S7, in dem eine Proliferationsfähigkeit aus dem bestimmten Bedeckungsgrades 45 abgeleitet wird;
- einen vierten Auswerteschritt C S208, in dem die zytotoxische Wirkung 46 der analytischen Probe auf die tierischen Zellen 23 aus der bestimmten Proliferationsfähigkeit, des dritten Auswerteschrittes A S7, somit mittelbar aus dem Bedeckungsgrad 45, abgeleitet wird.

### Bezugszeichenliste

- 1: Ortsauflösendes Massenspektrometer
- 2: MALDI-ToF-Massenspektrometer
- 3: MALDI-Ionenquelle
- 4: Flugrohr
- 5: Detektor
- 6: Massenspektrometrischer Probenträger
- 7: XY-Tisch
- 8: Laser
- 9: Laserstrahl
- 10: Spiegel-/Linsensystem
- 11: Massenspektrometrische Probe
- 12: Probenionen
- 13: Beschleunigungselektrode
- 14: Flugzeit-Massenanalysator
- 15: Vakuumpumpe
- 16: Probenstelle
- 17: Beschleunigungsstrecke
- 20: Massenspektrum - nicht ortsaufgelöst
- 21: Matrix-basiertes ortsauflösendes Massenspektrometer
- 22: Applikationsvorrichtung
- 23: Tierische Zellen
- 24: Kultivierungsvorrichtung
- 25: Adhärente tierische Zelle
- 26: Nicht-adhärente tierische Zelle
- 27: Flüssigkeitsaufsaugvorrichtung
- 28: Sprühvorrichtung
- 29: Matrix
- 30: Matrix-Mikrotröpfchen
- 31: Aufgeschlossene tierische Zelle
- 33: Messposition
- 34: Teilfläche der Probenstelle
- 35: Raster
- 37: Datenverarbeitungseinheit
- 38: Ortsaufgelöstes Massenspektrum
- 39: Massenspektrometrisches Signal
- 40: Zell spezifisches massenspektrometrisches Signal
- 41: Zellspezifische massenspektrometrische Signatur
- 42: Zellpräsenzwert
- 43: Positiver Zellpräsenzwert
- 44: Negativer Zellpräsenzwert
- 45: Bedeckungsgrad
- 46: Zytotoxische Wirkung
- 47: Erste Teilfläche
- 48: Zweite Teilfläche
- 49: Grenzlinie, (illustrativ)
- 50: Erstreckungsrichtung
- 51: Referenzpunkt
- 52: Umschlagpunkt
- 53: Zell-Ausdehnungsweite
- 55: Nicht-zellspezifisches massenspektrometrisches Signal
- 56: Erste Messposition
- 57: Zweite Messposition
- 58: Hintergrundrauschen
- 101: Massenspektrometer

- S1: Probenbereitstellungsschritt A
- S2: Kultivierungsschritt
- S3a: Flüssigkeitsentfernungsschritt
- S3b: Wachschritt
- S4a: Probenpräparationsschritt A
- S4b: Messschritt A
- S5: Erster Auswerteschritt A
- S6a: Zweiter Auswerteschritt A
- S6b: Auswertezwischenschritt A
- S7: Dritter Auswerteschritt A
- S8: Vierter Auswerteschritt A
- S101: Probenbereitstellungsschritt B
- S104a: Probenpräparationsschritt B
- S104b: Messschritt B
- S106a: Zweiter Auswerteschritt B
- S106b: Auswertezwischenschritt B
- S107: Dritter Auswerteschritt B
- S108: Vierter Auswerteschritt B
- S204b: Messschritt C
- S205: Erster Auswerteschritt A
- S206a: Zweiter Auswerteschritt C
- S208: Vierter Auswerteschritt C

## Patentansprüche

1. Verfahren zur Bestimmung der zytotoxischen Wirkung (46) einer analytischen Probe auf tierische Zellen (23), umfassend folgende Schritte:
- einen Probenbereitstellungsschritt A (S1), in dem eine massenspektrometrischen Probe (11), umfassend tierische Zellen (23), Nährmedium und die analytische Probe, welche potenziell einen zytotoxischen Faktor aufweist, auf mindestens einer Probenstelle (16) eines massenspektrometrischen Probenträgers (6) bereitgestellt wird;
- einen Kultivierungsschritt (S2), in dem die massenspektrometrische Probe (11) auf der Probenstelle (16) des massenspektrometrischen Probenträgers (6) in einer Kultivierungsvorrichtung (24) inkubiert wird;
- einen Flüssigkeitsentfernungsschritt (S3a), in dem Restflüssigkeit der massenspektrometrischen Probe (11) von der Probenstelle (16) entfernt wird;
- einen Messschritt A (S4b), in dem ortsaufgelöste Massenspektren (38) an einer Vielzahl von Messpositionen (33) in mindestens einer Teilfläche (34) der Probenstelle (16) mittels eines ortsauflösenden Massenspektrometers (1) aufgenommen werden, wobei bei Verwendung von einem matrix-basierten ortsauflösende Massenspektrometer (21) vor der Aufnahme der ortsaufgelösten Massenspektren (21) im Messschritt A (S4b) in einem vorgelagerten Probenpräparationsschritt A (S4a) eine Präparation der Probenstelle (16) durch ortstreue Auftragung einer Matrix (29) auf mindestens die eine Teilfläche (34) der Probenstelle (16) erfolgt;
- einen ersten Auswerteschritt A (S5), in dem jedes ortsaufgelöste Massenspektrum (38) hinsichtlich des Vorhandenseins einer zellspezifischen massenspektrometrischen Signatur (41) für die tierischen Zellen (23) analysiert wird und jeder Messposition (33) ein Zellpräsenzwert (42) zugewiesen wird;
- einen zweiten Auswerteschritt A (S6a), in dem ein Bedeckungsgrad (45) durch tierische Zellen (23) für mindestens die Teilfläche (34) der Probenstelle (16) aus den Zellpräsenzwerten (42) der Messpositionen (33) bestimmt wird;
- und/oder einen dritten Auswerteschritt A (S7), in dem eine Proliferationsfähigkeit aus dem bestimmten Bedeckungsgrades (45) abgeleitet wird;
- einen vierten Auswerteschritt A (S8), in dem die zytotoxische Wirkung (46) der analytischen Probe auf die tierischen Zellen (23) mittelbar oder unmittelbar aus dem bestimmten Bedeckungsgrad (45) des zweiten Auswerteschrittes A (S6a) abgeleitet wird, wobei hierzu die zytotoxische Wirkung (46) der analytischen Probe auf die tierischen Zellen (23) aus dem Ergebnis wenigstens einer der beiden Schritte abgeleitet wird: zweiter Auswerteschritt A (S6a), dritter Auswerteschritt A (S7).

2. Verfahren zur Bestimmung der zytotoxischen Wirkung (46) einer analytischen Probe auf tierische Zellen (23), umfassend folgende Schritte:
- einen Probenbereitstellungsschritt A (S1), in dem eine massenspektrometrischen Probe (11), umfassend tierische Zellen (23), Nährmedium und die analytische Probe, welche potenziell einen zytotoxischen Faktor aufweist, auf mindestens einer Probenstelle (16) eines massenspektrometrischen Probenträgers (6) bereitgestellt wird;
- einen Kultivierungsschritt (S2), in dem die massenspektrometrische Probe (11) auf der Probenstelle (16) des massenspektrometrischen Probenträgers (6) in einer Kultivierungsvorrichtung (24) inkubiert wird;
- einen Flüssigkeitsentfernungsschritt (S3a), in dem Restflüssigkeit der massenspektrometrischen Probe (11) von der Probenstelle (16) entfernt wird;
- einen Messschritt C (S204b), in dem Massenspektren (20) an einer Vielzahl von Messpositionen (33) verteilt über mindestens eine Teilfläche (34) der Probenstelle (16) mittels eines Massenspektrometers aufgenommen werden, wobei bei Verwendung von einem matrix-basierten Massenspektrometer vor der Aufnahme der Massenspektren (20) im Messschritt C (S204b) in einem vorgelagerten Probenpräparationsschritt A (S4a) eine Präparation der Probenstelle (16) durch ortstreue Auftragung einer Matrix (29) auf mindestens die eine Teilfläche (34) der Probenstelle (16) erfolgt;
- einen ersten Auswerteschritt C (S205), in dem jedes Massenspektrum (20) hinsichtlich des Vorhandenseins einer zellspezifischen massenspektrometrischen Signatur (41) für die tierischen Zellen (23) analysiert wird;
- einen zweiten Auswerteschritt C (S206a), in dem ein Bedeckungsgrad (45) durch tierische Zellen (23) für mindestens die Teilfläche (34) der Probenstelle (16) aus dem Ergebnis des ersten Auswerteschrittes C (S205) bestimmt wird;
- einen vierten Auswerteschritt C (S208), in dem die zytotoxische Wirkung (46) der analytischen Probe auf die tierischen Zellen (23) aus dem bestimmten Bedeckungsgrad (45) des zweiten Auswerteschrittes C (S206a) abgeleitet wird.

3. Verfahren zur Bestimmung der zytotoxischen Wirkung (56) einer analytischen Probe auf tierische Zellen (23) umfassend folgende Schritte:
- einen Probenbereitstellungsschritt B (S101), in dem eine massenspektrometrischen Probe (11) umfassend tierische Zellen (23), Nährmedium und die analytischen Probe, welche potentiell einen zytotoxischen Faktor aufweist, auf mindestens einer Probenstelle (16) eines massenspektrometrischen Probenträgers (6) bereitgestellt wird, wobei die Probenstelle (16) eine erste und eine zweite Teilfläche (47, 48) aufweist, wobei die tierischen Zellen 23 auf der ersten Teilfläche (47) der Probenstelle (16) bereitgestellt werden und auf der zweiten Teilfläche (48) der Probenstelle (16) keine tierischen Zellen (23) bereitgestellt werden und wobei die restliche massenspektrometrische Probe (11) auf beiden Teilflächen (47, 48) bereitgestellt wird;
- einen Kultivierungsschritt (S2), in dem die massenspektrometrische Probe (11) auf der Probenstelle 16 des massenspektrometrischen Probenträgers (6) in einer Kultivierungsvorrichtung (24) inkubiert wird;
- einen Flüssigkeitsentfernungsschritt (S3a), in dem Restflüssigkeiten der massenspektrometrischen Probe (11) auf der Probenstelle (16) entfernt werden;
- einem Messschritt B (S104b), in dem ortsaufgelöste Massenspektren (38) an mindestens zwei Messpositionen (56,57) der Probenstelle (16) mittels eines ortsauflösendem Massenspektrometers (1) aufgenommen werden, wobei die erste Messposition (33, 56) noch tierische Zellen (23) aufweist und die zweite Messposition (33, 57) keine tierischen Zellen (23) aufweist,
wobei sich beide Messpositionen (56, 57) auf einer Erstreckungsrichtung (50) ausgehend von einem Referenzpunkt (51) befinden, wobei bei Verwendung von einem matrix-basierten ortsauflösende Massenspektrometer (1) vor der Aufnahme der ortsaufgelösten Massenspektren (38) im Messschritt B (S104b) in einem vorgelagerten Probenpräparationsschritt B (S104a) eine Präparation der Probenstelle (16) durch ortstreue Auftragung einer Matrix (29) auf mindestens einen Teil beider Teilflächen (47,48) der Probenstelle (48) erfolgt;
- einen ersten Auswerteschritt A (S5), in dem jedes ortsaufgelösten Massenspektrums (38) hinsichtlich des Vorhandenseins einer zellspezifischen massenspektrometrischen Signatur (41) für die tierische Zellen (23) analysiert wird und jeder Messposition (33, 56, 57) ein Zellpräsenzwert (42) zugewiesen wird;
- einen zweiten Auswerteschritt B (S106a), in dem eine Zell-Ausdehnungsweite (53) aus mindestens einer Entfernung von einem Referenzpunkt (51) zu einem Umschlagpunkt (52) ermittelt wird, wobei jeder Umschlagpunkt (52) mindestens aus der ersten und der zweiten Messposition (56, 57) berechnet wird;
- einem vierten Auswerteschritt B (S108), in dem die zytotoxische Wirkung (46) der analytischen Probe aus dem Ergebnis des vorangegangenen Auswerteschrittes abgeleitet wird.

4. Verfahren nach Anspruch 1 oder einem der weiteren Ansprüche, **dadurch gekennzeichnet, dass** die tierischen Zellen (23) adhärent wachsende tierische Zellen sind.

5. Verfahren nach Anspruch 1,2 oder 4, **dadurch gekennzeichnet, dass** die Vielzahl von Messpositionen (33) in der mindestens einen Teilfläche (34) der Probenstelle (16) derart örtlich verteilt sind, sodass der bestimmte Bedeckungsgrad (45) repräsentativ für die Teilfläche (34) und/oder die Probenstelle (16) ist.

6. Verfahren nach Anspruch 1 oder einem der weiteren Ansprüche, **dadurch gekennzeichnet, dass** die massenspektrometrische Probe (11) im Probenbereitstellungsschritt A,B (S1, S101) des Weiteren einen potenziell Zytotoxizitätsfaktor-neutralisierenden Faktor umfasst.

7. Verfahren nach Anspruch 1 oder einem der weiteren Ansprüche, **dadurch gekennzeichnet, dass** zusätzlich zur massenspektrometrischen Probe (11) umfassend die analytische Probe mindestens eine Referenzprobe das Verfahren durchläuft.

8. Verfahren nach Anspruch 7, **dadurch gekennzeichnet, dass** die Referenzprobe den potenziell zytotoxischen Faktor und/oder den potenziell Zytotoxizitätsfaktor-neutralisierenden Faktor nicht umfasst und nach der Bestimmung des Bedeckungsgrades (45) im zweiten Auswerteschritt A,C (S6a, S206a) in einem Auswertezwischenschritt A (S6b) ein Vergleich des Bedeckungsgrades (45) der Referenzprobe mit dem Bedeckungsgrad (45) der massenspektrometrischen Probe (11) umfassend die analytische Probe durchgeführt wird und der Vergleich in die Ableitung der Proliferationsfähigkeit im dritten Auswerteschritt A (S7) und/oder in die Ableitung der zytotoxischen Wirkung (46) im vierten Auswerteschritt A,C (S8, S108) einbezogen wird.

9. Verfahren nach Anspruch 1 oder einem der weiteren Ansprüche, **dadurch gekennzeichnet, dass** die massenspektrometrische Probe (11) im Probenbereitstellungsschritt A,B (S1, S101) bereitgestellt wird, indem die tierischen Zellen (23) vor Auftragung des Nährmediums und der analytischen Probe bereits auf den Probenstellen (16) vorliegen;
oder indem die tierischen Zellen (23) in suspendierter Form auf die Probenstellen (16) aufgetragen werden.

10. Verfahren nach Anspruch 1 oder einem der weiteren Ansprüche, **dadurch gekennzeichnet, dass** die tierischen Zellen (23) durch Auftragung der tierischen Zellen (23) in suspendierter Form auf die Probenstellen (16) im Probenbereitstellungsschritt A,B (S1, S101) bereitgestellt werden und, in einem Waschschritt (S3B) die tierischen Zellen (23) auf dem Probenträger (16) gewaschen und restliche Waschflüssigkeiten entfernt werden, wobei der Waschschritt (S3B) dem Flüssigkeitsentfernungsschritt (S3a) folgt oder ihn substituiert.

11. Verfahren nach Anspruch 1 oder einem der weiteren Ansprüche, **dadurch gekennzeichnet, dass** die analytische Probe bereitgestellt wird, indem eine Ursprungsprobe, Bestandteile der Ursprungsprobe oder ein isolierter zytotoxischer Faktor, bereitgestellt wird.

12. Verfahren nach Anspruch 1 oder einem der weiteren Ansprüche, **dadurch gekennzeichnet, dass** die Ursprungsprobe aus folgender Gruppe ausgewählt ist:
- Probe eines Menschen
- Probe eines Tieres
- Umweltprobe

13. Verfahren nach Anspruch 1 oder einem der weiteren Ansprüche, **dadurch gekennzeichnet, dass** die tierischen Zellen (23) Wirbeltierzellen, Säugetierzellen und/oder humane Zellen sind.

14. Verfahren nach Anspruch 1 oder einem der weiteren Ansprüche, **dadurch gekennzeichnet, dass** die tierischen Zellen (23) kontinuierlichen Zelllinien sind oder aus entnommenen Gewebeproben oder entnommenen Tumorproben eines Menschen oder Tieres stammen.

15. Verfahren nach Anspruch 1 oder einem der weiteren Ansprüche, **dadurch gekennzeichnet, dass** der mindestens eine zytotoxische Faktor aus folgender Gruppe stammt:
- chemisches Element
- niedermolekulare chemische Verbindung
- hochmolekulare chemische Verbindung
- pharmazeutischer Wirkstoff
- Toxin
- Archaea
- Bakterium
- Virus
- Pilz
- Protozoe
- Alge
- Parasit
- Bestandteil von Mikroorganismen
- Sekretierte Substanz von Mikroorganismen
- zytotoxische Substanz biologischen Ursprungs
- zytotoxische Substanz mikrobiellen Ursprungs aus einer humanen Probe
- Chemotherapeutikum
- Antimikrobielles Mittel
- Antivirales Mittel
- Antifungales Mittel
- Antibakterielles Mittel
- Antiparasitäres Mittel
- Protein
- Peptid
- Antikörper
- Antitumoraler Wirkstoff
- Biozid

16. Verfahren nach Anspruch 1 oder einem der weiteren Ansprüche, **dadurch gekennzeichnet, dass** die analytische Probe als zytotoxischen Faktor bakterielle Zellen oder deren Bestandteile umfasst und der Zytotoxizitätsfaktor-neutralisierende Faktor ein antibakterielles Mittel ist, dass gegen die bakteriellen Zellen oder deren Bestandteile gerichtet ist; oder
dass die analytische Probe als zytotoxischen Faktor Viruspartikel oder deren Bestandteile umfasst und der Zytotoxizitätsfaktor-neutralisierende Faktor ein antivirales Mittel ist, dass gegen die Viruspartikel oder deren Bestandteile gerichtet ist; oder dass die analytische Probe als zytotoxischen Faktor fungale Zellen oder deren Bestandteile umfasst und der Zytotoxizitätsfaktor-neutralisierende Faktor ein antifungales Mittel ist, dass gegen die fungalen Zellen oder deren Bestandteile gerichtet ist; oder dass die analytische Probe als zytotoxischen Faktor einen Parasiten umfasst und der Zytotoxizitätsfaktor-neutralisierende Faktor ein antiparasitäres Mittel ist, dass gegen den Parasiten gerichtet ist.

17. Verfahren nach Anspruch 1 oder einem der weiteren Ansprüche, **dadurch gekennzeichnet, dass** eine konzentrationsabhängige zytotoxische Wirkung (46) der analytischen Probe bestimmt wird, indem jeweils unterschiedliche Konzentrationen der analytischen Probe auf verschiedene Probenstellen (16) bereitgestellt werden und die konzentrationsabhängige zytotoxische Wirkung (46) eine Funktion der bestimmten Bedeckungsgrade (45) oder der bestimmten Zell-Ausdehnungsweiten (53) der verschiedenen Probenstellen (16) und der jeweils unterschiedlichen Konzentrationen der analytischen Probe ist.

18. Verfahren nach Anspruch 1 oder einem der weiteren Ansprüche 3 bis 17, **dadurch gekennzeichnet, dass** das ortsauflösende Massenspektrometer (1) aus folgender Gruppe ausgewählt ist:
- LDI - Massenspektrometer
- MALDI - Massenspektrometer
- DESI - Massenspektrometer
- MALDESI - Massenspektrometer
- SIMS
- SIMS Imaging - Massenspektrometer
- MALDI-ToF - Massenspektrometer (2)
- MALDI-ToF-ToF - Massenspektrometer
- MALDI-MSI - Massenspektrometer
- Ortsauflösendes Massenspektrometer (1) mit einer Mikro-Fluid-Probenionisierungsvorrichtung.

19. Verfahren nach Anspruch 1 oder einem der weiteren Ansprüche, **dadurch gekennzeichnet, dass** die ortstreue Auftragung der Matrix (29) mittels eines Sprühverfahrens, Sublimationsverfahren oder sequenzieller gerichteter Positionierung einer Vielzahl von Matrix-Mikrotröpfchen (30) oder Matrix-Nanotröpfchen auf der Probenstelle (16) erfolgt.

20. Verfahren nach Anspruch 3, **dadurch gekennzeichnet, dass** das Verfahren einen dritten Auswerteschritt B (S107) und/oder einen Auswertezwischenschritt B umfasst, wobei im dritten Auswerteschritt B (S107) eine Proliferationsfähigkeit und/oder eine Migrationsfähigkeit der tierischen Zellen (23) aus der bestimmten Zell-Ausdehnungsweite (53) des zweiten Auswerteschrittes B (S106a) abgeleitet wird und die zytotoxische Wirkung (46) der analytischen Probe im viertem Auswerteschritt B (S108) aus der bestimmten Proliferationsfähigkeit und/oder der Migrationsfähigkeit der tierischen Zellen (23) und/oder der Zell-Ausdehnungsweite (53) abgeleitet wird, und/oder wobei in einem Auswertezwischenschritt B (S106b) ein Vergleich der Zell-Ausdehnungsweite (53) mindestens einer Referenzprobe mit der Zell-Ausdehnungsweite (53) der massenspektrometrischen Probe (11) umfassend die analytische Probe durchgeführt wird, wobei der Vergleich in die Ableitung der Proliferationsfähigkeit und/oder der Migrationsfähigkeit in dritten Auswerteschritt B (S107) und/oder in die Ableitung der zytotoxische Wirkung (46) im vierten Auswerteschritt B (S108) einbezogen wird.

21. System zur Bestimmung der zytotoxischen Wirkung (46) einer analytischen Probe auf tierische Zellen (23) mittels eines ortsauflösenden Massenspektrometers (1) umfassend mindestens ein ortsauflösendes Massenspektrometer (1) zur Erzeugung ortsaufgelösten Massenspektren (38), einen massenspektrometrischen Probenträger (6) mit Probenstellen (16) und eine Datenverarbeitungseinheit (37) zur Steuerung des ortsauflösenden Massenspektrometers (1) und zur Auswertung der erzeugten ortsaufgelösten Massenspektren (38), **dadurch gekennzeichnet, dass** die Datenverarbeitungseinheit (37) dazu eingerichtet ist, jedes ortsaufgelösten Massenspektrums (38) hinsichtlich des Vorhandenseins mindestens einer zellspezifischen massenspektrometrischen Signatur (41) für die tierische Zellen (23) zu analysieren; einen Bedeckungsgrad (45) durch die tierischen Zellen (23) für mindestens eine Teilfläche (34) der Probenstellen (16) und/oder eine Zell-Ausdehnungsweite (53) der tierischen Zellen (23) in mindestens einer Erstreckungsrichtung (50) der Probenstelle (16) zu bestimmen; und aus dem Bedeckungsgrad (45) und/oder aus der Zell-Ausdehnungsweite (53) die zytotoxische Wirkung (46) der analytischen Probe abzuleiten.
